# EUROPEAN PATENT SPECIFICATION

(11) **EP 2 659 275 B1**
(45) Date of publication and mention of the grant of the patent: **22.11.2017**
(21) Application number: 11810589.9
(22) Date of filing: 21.12.2011
(51) Int. Cl.: C07K 16/18, C07K 16/28, C07K 16/32, C07K 16/44, G01N 33/68

(54) **DETECTION OF A POLYPEPTIDE DIMER BY A BIVALENT BINDING AGENT**
ERKENNUNG EINES POLYPEPTIDDIMERS DURCH EIN BIVALENTES BINDEMITTEL
DÉTECTION D'UN DIMÈRE POLYPEPTIDIQUE PAR UN AGENT DE LIAISON BIVALENT

(30) Priority: 23.12.2010 EP 10196687; 13.07.2011 EP 11173844
(43) Date of publication of application: 06.11.2013
(73) Proprietor: Roche Diagnostics GmbH, 68305 Mannheim (DE); F. Hoffmann-La Roche AG, 4070 Basel (CH)
(72) Inventor: GERG, Michael, 81377 Muenchen (DE); HEINDL, Dieter, 81377 Muenchen (DE); MERTENS, Alfred, 69198 Schriesheim (DE); RUTZ, Christoph, 81475 Muenchen (DE); SCHRAEML, Michael, 82377 Penzberg (DE); SOUKUPOVA, Monika, 82405 Wessobrunn (DE); SUSTMANN, Claudio, 81373 Muenchen (DE); TACKE, Michael, 80689 Muenchen (DE); VAN DIECK, Jan, 80335 München (DE)
(74) Representative: Burger, Alexander
(86) International application number: PCT/EP2011/073567
(87) International publication number: WO 2012/085069

(56) References cited:
- US-A- 5 635 602
- US-A1- 2006 199 207
- US-A1- 2008 075 712
- US-A1- 2008 131 883
- FRANCOIS C ET AL: "Consturction of a bispecific antibody reacting with the alpha-and beta-chains ofthe human IL-2 receptor", JOURNAL OF IMMUNOLOGY, AMERICAN ASSOCIATION OF IMMUNOLOGISTS, US, vol. 150, no. 10, 1 January 1993 (1993-01-01), pages 4610-4619, XP002974197, ISSN: 0022-1767
- LEDBETTER J A ET AL: "CD28 LIGATION IN T-CELL ACTIVATION: EVIDENCE FOR TWO SIGNAL TRANSDUCTION PATHWAYS", BLOOD, AMERICAN SOCIETY OF HEMATOLOGY, US, vol. 75, no. 7, 1 April 1990 (1990-04-01), pages 1531-1539, XP000764199, ISSN: 0006-4971
- J. DONG ET AL: "Stable IgG-like Bispecific Antibodies Directed toward the Type I Insulin-like Growth Factor Receptor Demonstrate Enhanced Ligand Blockade and Anti-tumor Activity", JOURNAL OF BIOLOGICAL CHEMISTRY, vol. 286, no. 6, 11 February 2011 (2011-02-11), pages 4703-4717, XP055012243, ISSN: 0021-9258, DOI: 10.1074/jbc.M110.184317

### SEQUENCE LISTING

<110> Roche Diagnostics GmbH F. Hoffmann-La Roche AG
<120> Detection of a polypeptide dimer by a bi-valent binding agent
<130> 27514 WO-WN
<150> EP 10196687.7
   <151> 2010-12-23
<150> EP 11173844.9
   <151> 2011-07-13
<160> 28
<170> PatentIn version 3.5
<210> 1
   <211> 122
   <212> PRT
   <213> Mus musculus
<400> 1
   Gln Cys Asp Val Lys Leu Val Glu Ser Gly Gly Gly Leu Val Lys Pro
   1               5                   10                  15
   Gly Gly Ser Leu Lys Leu Ser Cys Ala Ala Ser Gly Phe Thr Phe Ser
               20                  25                  30
   Asp Tyr Pro Met Ser Trp Val Arg Gln Thr Pro Glu Lys Arg Leu Glu
           35                  40                  45
   Trp Val Ala Thr Ile Thr Thr Gly Gly Thr Tyr Thr Tyr Tyr Pro Asp
       50                  55                  60
   Ser Ile Lys Gly Arg Phe Thr Ile Ser Arg Asp Asn Ala Lys Asn Thr
   65                  70                  75                  80
   Leu Tyr Leu Gln Met Gly Ser Leu Gln Ser Glu Asp Ala Ala Met Tyr
                   85                  90                  95
   Tyr Cys Thr Arg Val Lys Thr Asp Leu Trp Trp Gly Leu Ala Tyr Trp
               100                 105                 110
   Gly Gln Gly Thr Leu Val Thr Val Ser Ala
           115                 120
<210> 2
   <211> 116
   <212> PRT
   <213> Mus musculus
<400> 2
   Gln Leu Val Leu Thr Gln Ser Ser Ser Ala Ser Phe Ser Leu Gly Ala
   1 5 10 15
   Ser Ala Lys Leu Thr Cys Thr Leu Ser Ser Gln His Ser Thr Tyr Thr
   20 25 30
   Ile Glu Trp Tyr Gln Gln Gln Pro Leu Lys Pro Pro Lys Tyr Val Met
   35 40 45
   Glu Leu Lys Lys Asp Gly Ser His Thr Thr Gly Asp Gly Ile Pro Asp
   50 55 60
   Arg Phe Ser Gly Ser Ser Ser Gly Ala Asp Arg Tyr Leu Ser Ile Ser
   65 70 75 80
   Asn Ile Gln Pro Glu Asp Glu Ser Ile Tyr Ile Cys Gly Val Gly Asp
   85 90 95
   Thr Ile Lys Glu Gln Phe Val Tyr Val Phe Gly Gly Gly Thr Lys Val
   100 105 110
   Thr Val Leu Gly
   115
<210> 3
   <211> 121
   <212> PRT
   <213> Mus musculus
<400> 3
   Glu Val Gln Leu Gln Gln Ser Gly Pro Ala Leu Val Lys Pro Gly Ala
   1 5 10 15
   Ser Val Lys Met Ser Cys Lys Ala Ser Gly Phe Thr Phe Thr Ser Tyr
   20 25 30
   Val Ile His Trp Val Lys Gln Lys Pro Gly Gln Gly Leu Glu Trp Ile
   35 40 45
   Gly Tyr Leu Asn Pro Tyr Asn Asp Asn Thr Lys Tyr Asn Glu Lys Phe
   50 55 60
   Lys Gly Lys Ala Thr Leu Thr Ser Asp Arg Ser Ser Ser Thr Val Tyr
   65 70 75 80
   Met Glu Phe Ser Ser Leu Thr Ser Glu Asp Ser Ala Val Tyr Phe Cys
   85 90 95
   Ala Arg Arg Gly Ile Tyr Ala Tyr Asp His Tyr Phe Asp Tyr Trp Gly
   100 105 110
   Gln Gly Thr Ser Leu Thr Val Ser Ser
   115 120
<210> 4
   <211> 106
   <212> PRT
   <213> Mus musculus
<400> 4
   Gln Ile Val Leu Thr Gln Ser Pro Ala Ile Met Ser Ala Ser Pro Gly
   1 5 10 15
   Glu Lys Val Thr Leu Thr Cys Ser Ala Ser Ser Ser Val Asn Tyr Met
   20 25 30
   Tyr Trp Tyr Gln Gln Lys Pro Gly Ser Ser Pro Arg Leu Leu Ile Tyr
   35 40 45
   Asp Thr Ser Asn Leu Ala Ser Gly Val Pro Val Arg Phe Ser Gly Ser
   50 55 60
   Gly Ser Val Thr Ser Tyr Ser Leu Thr Ile Ser Arg Met Glu Ala Glu
   65 70 75 80
   Asp Ala Ala Thr Tyr Tyr Cys Gln Gln Trp Ser Thr Tyr Pro Leu Thr
   85 90 95
   Phe Gly Ala Gly Thr Lys Leu Glu Leu Lys
   100 105
<210> 5
   <211> 19
   <212> DNA
   <213> Artificial Sequence
<220>
   <223> 19mer ssDNA
<400> 5
   agtctattaa tgcttctgc 19
<210> 6
   <211> 17
   <212> DNA
   <213> Artificial Sequence
<220>
   <223> 17mer ssDNA
<400> 6
   agttctatcg tcgtcca 17
<210> 7
   <211> 19
   <212> DNA
   <213> Artificial Sequence
<220>
   <223> complementary 19mer ssDNA
<400> 7
   gcagaagcat taatagact 19
<210> 8
   <211> 17
   <212> DNA
   <213> Artificial Sequence
<220>
   <223> complementary 17mer ssDNA
<400> 8
   tggacgacga tagaact 17
<210> 9
   <211> 33
   <212> PRT
   <213> Artificial Sequence
<220>
   <223> Synthetic peptide
<220>
   <221> MOD_RES
   <222> (16)..(17)
   <223> bAla
<400> 9
   Glu Arg Ala Glu Gln Gln Arg Ile Arg Ala Glu Arg Glu Lys Glu Xaa
   1               5                   10                  15
   Xaa Ser Leu Lys Asp Arg Ile Glu Lys Arg Arg Arg Ala Glu Arg Ala
               20                  25                  30
   Glu
<210> 10
   <211> 32
   <212> PRT
   <213> Artificial Sequence
<220>
   <223> Synthetic peptide
<220>
   <221> MOD_RES
   <222> (16)..(17)
   <223> Amino-trioxa-octanoic-acid (X represents Amino-trioxa-octanoic-acid)
<400> 10
   Ser Leu Lys Asp Arg Ile Glu Arg Arg Arg Ala Glu Arg Ala Glu Xaa
   1               5                   10                  15
   Xaa Glu Arg Ala Glu Gln Gln Arg Ile Arg Ala Glu Arg Glu Lys Glu
               20                  25                  30
<210> 11
   <211> 27
   <212> PRT
   <213> Artificial Sequence
<220>
   <223> Synthetic peptide
<400> 11
   Phe Asp Glu Arg Gln Pro Tyr Ala His Met Asn Gly Gly Arg Lys Asn
   1               5                   10                  15
   Glu Arg Ala Leu Pro Leu Pro Gln Ser Ser Thr
               20                  25
<210> 12
   <211> 28
   <212> PRT
   <213> Artificial Sequence
<220>
   <223> Synthetic peptide
<400> 12
   Tyr Glu Glu His Ile Pro Tyr Thr His Met Asn Gly Gly Lys Lys Asn
   1               5                   10                  15
   Gly Arg Ile Leu Thr Leu Pro Arg Ser Asn Pro Ser
               20                  25
<210> 13
   <211> 5
   <212> PRT
   <213> Artificial Sequence
<220>
   <223> Synthetic peptide (Spacer)
<400> 13
   Gly Gly Gly Gly Ser
   1 5
<210> 14
   <211> 9
   <212> PRT
   <213> Artificial Sequence
<220>
   <223> HA-Tag
<400> 14
   Tyr Pro Tyr Asp Val Pro Asp Tyr Ala
   1 5
<210> 15
   <211> 15
   <212> PRT
   <213> Artificial Sequence
<220>
   <223> Avi-Tag
<400> 15
   Gly Leu Asn Asp Ile Phe Glu Ala Gln Lys Ile Glu Trp His Glu
   1 5 10 15
<210> 16
   <211> 17
   <212> DNA
   <213> Artificial Sequence
<220>
   <223> oligonucleotide
<400> 16
   acctgctgct atcttga 17
<210> 17
   <211> 36
   <212> DNA
   <213> Artificial Sequence
<220>
   <223> linker oligonucleotide
<220>
   <221> misc_feature
   <223> Y represents biotinylated thymidine
<400> 17
   gcagaagcat taatagacyt ggacgacgat agaact 36
<210> 18
   <211> 36
   <212> DNA
   <213> Artificial Sequence
<220>
   <223> adaptor oligonucleotide
<220>
   <221> misc_feature
   <223> wherein C3 represents
<220>
   <221> misc_feature
   <223> wherein C3 represents a propane diol spacer
<400> 18
   gtctattaat gcttctgcct ggacgacgat agaact 36
<210> 19
   <211> 120
   <212> PRT
   <213> Mus musculus
<220>
   <221> misc_feature
   <223> 7.2.32 variable region heavy chain
<400> 19
   Glu Phe Glu Val Gln Leu Gln Glu Ser Gly Gly Gly Leu Val Gln Pro
   1               5                   10                  15
   Lys Gly Ser Leu Gln Leu Ser Cys Ala Ala Ser Gly Phe Thr Phe Asn
               20                  25                  30
   Thr Tyr Ala Met His Trp Val Arg Gln Ala Pro Gly Lys Gly Leu Glu
           35                  40                  45
   Trp Val Ala Arg Ile Arg Thr Glu Ser Ser Asp Tyr Ala Thr Asp Tyr
       50                  55                  60
   Ala Asp Ser Val Lys Asp Arg Phe Ile Ile Ser Arg Asp Asp Ser Gln
   65                  70                  75                  80
   Asn Met Leu Tyr Leu Gln Met Asn Asn Leu Lys Ser Glu Asp Thr Ala
                   85                  90                  95
   Ile Tyr Tyr Cys Val Arg Ser Ser Gly Phe Asp Tyr Trp Gly Gln Gly
               100                 105                 110
   Thr Thr Leu Thr Val Ser Ser Ser
                       115                 120
<210> 20
   <211> 110
   <212> PRT
   <213> Mus musculus
<220>
   <221> misc_feature
   <223> 7.2.32 variable region light chain
<400> 20
   Asp Ile Gln Met Thr Gln Ser Pro Ser Leu Pro Val Ser Leu Gly Asp
   1               5                   10                  15
   Gln Ala Ser Ile Ser Cys Arg Ser Ser Gln Ser Leu Val His Asp Asn
               20                  25                  30
   Gly Asn Thr Tyr Leu His Trp Phe Leu Gln Lys Pro Gly Gln Ser Pro
           35                  40                  45
   Lys Leu Leu Ile Tyr Lys Val Ser Asn Arg Phe Ser Gly Val Pro Asp
       50                  55                  60
   Arg Phe Gly Gly Ser Gly Ser Gly Thr Asp Phe Thr Leu Lys Ile Ser
   65                  70                  75                  80
   Gly Val Glu Ala Glu Asp Leu Gly Val Tyr Phe Cys Ser Gln Gly Thr
                   85                  90                  95
   His Val Pro Thr Phe Gly Gly Gly Thr Lys Leu Glu Ile Lys
               100                 105                 110
<210> 21
   <211> 111
   <212> PRT
   <213> Mus musculus
<220>
   <221> misc_feature
   <223> 4.1.43 variable region heavy chain
<400> 21
   Glu Val Gln Leu Gln Gln Ser Gly Pro Val Leu Val Lys Pro Gly Ala
   1               5                   10                  15
   Ser Val Lys Met Ser Cys Arg Pro Ser Gly Tyr Thr Phe Thr Asp Tyr
               20                  25                  30
   Asn Met His Trp Val Lys Gln Ser His Glu Lys Ser Leu Glu Trp Ile
           35                  40                  45
   Gly Tyr Ile Asn Pro Lys Asn Gly Ile Ile Ser Tyr Asn Gln Lys Phe
       50                  55                  60
   Lys Asp Lys Ala Thr Leu Thr Val Asn Lys Ser Ser Arg Thr Ala Tyr
   65                  70                  75                  80
   Met Glu Leu Arg Ser Leu Thr Ser Glu Asp Ser Ala Val Tyr Phe Cys
                   85                  90                  95
   Ala Gly Asp Tyr Trp Gly Gln Gly Thr Ser Val Thr Val Ser Ser
               100                 105                 110
<210> 22
   <211> 112
   <212> PRT
   <213> Mus musculus
<220>
   <221> misc_feature
   <223> 4.1.43 variable region light chain
<400> 22
   Asp Val Val Met Thr Gln Thr Pro Leu Ser Leu Pro Val Ser Leu Gly
   1               5                   10                  15
   Asp Gln Ala Ser Ile Ser Cys Arg Ser Ser Gln Ser Leu Val His Ser
               20                  25                  30
   Asn Gly Asn Thr Tyr Leu His Trp Tyr Leu Gln Lys Pro Gly Gln Ser
           35                  40                  45
   Pro Lys Leu Leu Ile Ser Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Phe Cys Ser Gln Ser
                   85                  90                  95
   Ile His Leu Pro Trp Thr Phe Gly Gly Gly Thr Lys Leu Glu Ile Lys
               100                 105                 110
<210> 23
   <211> 12
   <212> PRT
   <213> Artificial Sequence
<220>
   <223> Synthetic Peptide
<220>
   <221> misc_feature
   <223> Sortase cleavage-Tag
<400> 23
   Lys Leu Leu Pro Glu Thr Gly Gly Gly Ser Gly Ser
   1               5                   10
<210> 24
   <211> 481
   <212> PRT
   <213> Artificial Sequence
<220>
   <223> Construct HC dual binder
<220>
   <221> Fab HC 7.2.32
   <222> (1)..(252)
<220>
   <221> GGGGS
   <222> (253)..(257)
   <223> Amino acid sequence motive Gly-Gly-Gly-Gly-Ser (= GGGGS or G4S) is present = 1, 2, 3 or 4 times.
<220>
   <221> hemagglutinin-Tag
   <222> (258)..(266)
<220>
   <221> GGGGS
   <222> (267)..(271)
   <223> Amino acid sequence motive Gly-Gly-Gly-Gly-Ser (= GGGGS or G4S) is present = 1, 2, 3 or 4 times.
<220>
   <221> Fab HC 4.1.43
   <222> (272)..(481)
<400> 24
   Met Gly Leu Phe Cys Ser His Pro Gly Asp Pro Leu Ala Thr Thr Met
   1               5                   10                  15
   Gly Trp Ser Cys Ile Ile Leu Phe Leu Val Ala Thr Ala Thr Gly Val
               20                  25                  30
   His Ser Glu Phe Glu Val Gln Leu Gln Glu Ser Gly Gly Gly Leu Val
           35                  40                  45
   Gln Pro Lys Gly Ser Leu Gln Leu Ser Cys Ala Ala Ser Gly Phe Thr
       50                  55                  60
   Phe Asn Thr Tyr Ala Met His Trp Val Arg Gln Ala Pro Gly Lys Gly
   65                  70                  75                  80
   Leu Glu Trp Val Ala Arg Ile Arg Thr Glu Ser Ser Asp Tyr Ala Thr
                   85                  90                  95
   Asp Tyr Ala Asp Ser Val Lys Asp Arg Phe Ile Ile Ser Arg Asp Asp
               100                 105                 110
   Ser Gln Asn Met Leu Tyr Leu Gln Met Asn Asn Leu Lys Ser Glu Asp
           115                 120                 125
   Thr Ala Ile Tyr Tyr Cys Val Arg Ser Ser Gly Phe Asp Tyr Trp Gly
       130                 135                 140
   Gln Gly Thr Thr Leu Thr Val Ser Ser Ser Lys Thr Thr Pro Pro Ser
   145                 150                 155                 160
   Val Tyr Pro Leu Ala Pro Gly Cys Gly Asp Thr Thr Gly Ser Ser Val
                   165                 170                 175
   Thr Leu Gly Cys Leu Val Lys Gly Tyr Phe Pro Glu Ser Val Thr Val
               180                 185                 190
   Thr Trp Asn Ser Gly Ser Leu Ser Ser Ser Val His Thr Phe Pro Ala
           195                 200                 205
   Leu Leu Gln Ser Gly Leu Tyr Thr Met Ser Ser Ser Val Thr Val Pro
       210                 215                 220
   Ser Ser Thr Trp Pro Ser Gln Thr Val Thr Cys Ser Val Ala His Pro
   225                 230                 235                 240
   Ala Ser Ser Thr Thr Val Asp Lys Lys Leu Lys Leu Gly Gly Gly Gly
                   245                 250                 255
   Ser Tyr Pro Tyr Asp Val Pro Asp Tyr Ala Gly Gly Gly Gly Ser Gly
               260                 265                 270
   Thr Glu Val Gln Leu Gln Gln Ser Gly Pro Val Leu Val Lys Pro Gly
           275                 280                 285
   Ala Ser Val Lys Met Ser Cys Arg Pro Ser Gly Tyr Thr Phe Thr Asp
       290                 295                 300
   Tyr Asn Met His Trp Val Lys Gln Ser His Glu Lys Ser Leu Glu Trp
   305 310 315 320
   Ile Gly Tyr Ile Asn Pro Lys Asn Gly Ile Ile Ser Tyr Asn Gln Lys
   325 330 335
   Phe Lys Asp Lys Ala Thr Leu Thr Val Asn Lys Ser Ser Arg Thr Ala
   340 345 350
   Tyr Met Glu Leu Arg Ser Leu Thr Ser Glu Asp Ser Ala Val Tyr Phe
   355 360 365
   Cys Ala Gly Asp Tyr Trp Gly Gln Gly Thr Ser Val Thr Val Ser Ser
   370 375 380
   Ala Lys Thr Thr Pro Pro Ser Val Tyr Pro Leu Ala Pro Gly Ser Ala
   385 390 395 400
   Ala Gln Thr Asn Ser Met Val Thr Leu Gly Cys Leu Val Lys Gly Tyr
   405 410 415
   Phe Pro Glu Pro Val Thr Val Thr Trp Asn Ser Gly Ser Leu Ser Ser
   420 425 430
   Gly Val His Thr Phe Pro Ala Val Leu Gln Ser Asp Leu Tyr Thr Leu
   435 440 445
   Ser Ser Ser Val Thr Val Pro Ser Ser Thr Trp Pro Ser Glu Thr Val
   450 455 460
   Thr Cys Asn Val Ala His Pro Ala Ser Ser Thr Lys Val Asp Lys Lys
   465 470 475 480
   Ile
<210> 25
   <211> 14
   <212> PRT
   <213> Homo sapiens
<220>
   <221> misc_feature
   <223> Human Her2 (1223-1236)
<400> 25
   Trp Asp Gln Asp Pro Pro Glu Arg Gly Ala Pro Pro Ser Thr
   1 5 10
<210> 26
   <211> 26
   <212> PRT
   <213> Homo sapiens
<220>
   <221> misc_feature
   <223> human Her3 (1242-1267)
<400> 26
   Pro Leu His Pro Val Pro Ile Met Pro Thr Ala Gly Thr Thr Pro Asp
   1               5                   10                  15
   Glu Asp Tyr Glu Tyr Met Asn Arg Gln Arg
               20                  25
<210> 27
   <211> 18
   <212> DNA
   <213> Artificial Sequence
<220>
   <223> synthetized oligo nucleotide
<220>
   <221> misc_feature
   <222> (1)..(1)
   <223> N = Gly-Gly-Gly-Gly-Aminolinker-(Spacer C3)3
<220>
   <221> modified_base
   <222> (18)..(18)
   <223> 'A' = fluorescein labeld
<400> 27
   nagttctatc gtcgtcca 18
<210> 28
   <211> 20
   <212> DNA
   <213> Artificial Sequence
<220>
   <223> synthetized oligo nucleotide
<220>
   <221> modified_base
   <222> (1)..(1)
   <223> 'A' = fluorescein labeled
<220>
   <221> misc_feature
   <222> (20)..(20)
   <223> N = (Spacer C3)3-Aminolinker-'-Gly-Gly-Gly-Gly
<400> 28
   agtctattaa tgcttctgcn 20

## Description

### Background of the Invention

The present document relates to a bivalent binding agent capable of binding a polypeptide dimer the binding agent consisting of two monovalent binders that are linked to each other via a linker, wherein the first monovalent binder binds to an epitope of a first target polypeptide comprised in said dimer, wherein the second monovalent binder binds to an epitope of a second target polypeptide comprised in said dimer, wherein each monovalent binder has a Kdiss in the range of 5x10⁻³/sec to 10⁻⁴/sec, and wherein the bivalent binding agent has a Kdiss of 3x10⁻⁵/sec or less. A method of making such bivalent binding agent and the use of such bivalent agent in histological staining procedures are also disclosed.

Many proteins, especially many membrane-bound receptor molecules are inactive as monomers and activated by homo- and/or heterodimerization. The specific detection of homo- or heterodimers of such receptor molecules against the background of single, non-dimerized molecules is a tremendeous challenge.

US5635602 discloses a process in which two single-stranded complementary oligonucleotides (A and B) are synthesized, each of them with a thiolated 5' end. In two reactions, the single-stranded oligonucleotide A is coupled to a Fab'(1) fragment, and, separately from the former, the single-stranded oligonucleotide B is coupled to a Fab'(2) fragment. Following separation steps with anion exchange HPLC the two coupled fragments are combined to form a bipartite compound. Notably, the two different antibody fragments are bridged by a single doublestranded oligonucleotide, and each single-stranded member thereof is coupled to a single antibody fragment.

It surprisingly has now been found that a bivalent binding agent can be provided that is capable of binding to a homo- or heterodimer and at the same time exhibiting no significant binding to the monomeric form of the one polypeptide comprised in the homodimer or to any of the two polypeptides comprised in the heterodimer.

### Summary of the Invention

In one embodiment the present disclosure relates to a bivalent binding agent capable of binding a polypeptide dimer the binding agent consisting of two monovalent binders that are linked to each other via a linker, wherein the first monovalent binder binds to an epitope of a first target polypeptide comprised in said dimer, wherein the second monovalent binder binds to an epitope of a second target polypeptide comprised in said dimer, wherein each monovalent binder has a kdiss in the range of 5x10⁻³/sec to 10⁻⁴/sec, and wherein the bivalent binding agent has a Kdiss of 3x10⁻⁵/sec or less.

Also disclosed is a method for obtaining a bivalent binding agent that specifically binds a polypeptide dimer, the method comprising the steps of selecting a first monovalent binder that binds to a first target polypeptide with a Kdiss of between 5x10⁻³/sec to 10⁻⁴/sec, selecting a second monovalent binder that binds to a second target polypeptide with a Kdiss of 5x10⁻³/sec to 10⁻⁴/sec, coupling both monovalent binders by a linker, and selecting a bivalent binding agent having a Kdiss-value of 3x10⁻⁵/sec or less.

The use of the novel bivalent binding agent, especially in an immunohistochemical procedure is also described.

A first aspect of the invention is a bivalent binding agent of Formula I

A - a':a - S - b:b' - B (Formula I),

the bivalent binding agent being capable of binding a polypeptide dimer, the dimer consisting of two associated polypeptide chains,
a) wherein A is a first monovalent binder, binding to a single epitope of a first target polypeptide comprised in said dimer, wherein B is a second monovalent binder, binding to a single epitope of a second target polypeptide comprised in said dimer,
b) wherein each monovalent binder A and B is selected from the group consisting of a peptide, a peptide mimetic, an aptamer, a spiegelmer, a darpin, a lectin, an ankyrin repeat protein, a Kunitz type domain, a single domain antibody, and a monovalent fragment of an antibody, and has a Kdiss in the range of 5x10⁻³/sec to 10⁻⁴/sec,
c) wherein each of a':a and b:b' independently is a binding pair, wherein a':a and b:b' are different, wherein S is a spacer, wherein - represents a covalent bond,
d) wherein a - S - b is a linker having a length of 6 to 100 nm,
e) wherein the members of the binding pairs a':a and b:b' are hybridizing nucleic acid sequences, and
f) wherein the bivalent binding agent has a Kdiss of 3x10⁻⁵/sec or less.

A second aspect of the invention is a method for forming a bivalent binding agent that specifically binds a polypeptide dimer, the dimer consisting of two associated polypeptide chains, the method comprising the steps of
a) selecting a first monovalent binder selected from the group consisting of a peptide, a peptide mimetic, an aptamer, a spiegelmer, a darpin, a lectin, an ankyrin repeat protein, a Kunitz type domain, a single domain antibody, and a monovalent fragment of an antibody, that binds to a single epitope of a first target polypeptide comprised in said dimer with a Kdiss of between 5x10⁻³/sec to 10⁻⁴/sec,
b) selecting a second monovalent binder selected from the group consisting of a peptide, a peptide mimetic, an aptamer, a spiegelmer, a darpin, a lectin, an ankyrin repeat protein, a Kunitz type domain, a single domain antibody, and a monovalent fragment of an antibody, that binds to a single epitope of a second target polypeptide comprised in said dimer with a Kdiss of 5x10⁻³/sec to 10⁻⁴/sec,
c) coupling the first monovalent binder to a member of a first binding pair of hybridizing DNA sequences and the second monovalent binder to a member of a second binding pair of hybridizing DNA sequences, wherein the first and the second binding pair do not interfere with each other, and a linker having a length of 6 to 100 nm and comprising a spacer and the complementary binding pair members to the first and the second binding pair member, respectively are co-incubated, whereby a bivalent binding agent is formed,
d) selecting a bivalent binding agent having a Kdiss-value of 3x10⁻⁵/sec or less,
thereby forming a bivalent binding agent that specifically binds the polypeptide dimer.

A third aspect of the invention is a histological staining method for a polypeptide dimer the method comprising the steps of
a) providing a cell or tissue sample,
b) incubating said sample with a bivalent binding agent according to the invention, and
c) detecting the bivalent binding agent, thereby staining said sample for the polypeptide dimer.

A fourth aspect of the invention is the use of a bivalent binding agent according to the invention in the staining of a cell or tissue sample.

### Detailed Description of the Invention

In one embodiment the present document relates to a bivalent binding agent capable of binding a polypeptide dimer the binding agent consisting of two monovalent binders that are linked to each other via a linker,
a) wherein the first monovalent binder binds to an epitope of a first target polypeptide comprised in said dimer,
b) wherein the second monovalent binder binds to an epitope of a second target polypeptide comprised in said dimer,
c) wherein each monovalent binder has a Kdiss in the range of 5x10⁻³/sec to 10⁻⁴/sec, and
d) wherein the bivalent binding agent has a Kdiss of 3x10⁻⁵/sec or less.

The present invention provides a bivalent binding agent of Formula I

A - a':a - S - b:b' - B (Formula I),

the bivalent binding agent being capable of binding a polypeptide dimer, the dimer consisting of two associated polypeptide chains,
a) wherein A is a first monovalent binder, binding to a single epitope of a first target polypeptide comprised in said dimer, wherein B is a second monovalent binder, binding to a single epitope of a second target polypeptide comprised in said dimer,
b) wherein each monovalent binder A and B is selected from the group consisting of a peptide, a peptide mimetic, an aptamer, a spiegelmer, a darpin, a lectin, an ankyrin repeat protein, a Kunitz type domain, a single domain antibody, and a monovalent fragment of an antibody, and has a Kdiss in the range of 5x10⁻³/sec to 10⁻⁴/sec,
c) wherein each of a':a and b:b' independently is a binding pair, wherein a':a and b:b' are different, wherein S is a spacer, wherein - represents a covalent bond,
d) wherein a - S - b is a linker having a length of 6 to 100 nm,
e) wherein the members of the binding pairs a':a and b:b' are hybridizing nucleic acid sequences, and
f) wherein the bivalent binding agent has a Kdiss of 3x10⁻⁵/sec or less.

Sometimes a single polypeptide is sufficient for the protein to be active. As indicated above, often, however, two or more polypeptides need to interact to allow a protein to perform its particular function. If this is the case, one talks e.g. of a protein or polypeptide dimer in case two polypeptide chains associate with one another. The dimer formation results from interactions between two polypeptide chains and/or and can be triggered by binding of a ligand. Dimers are held together by hydrogen bonds, ionic bonds and, less commonly, hydrophobic interfaces and inter-chain disulphide bonds.

Receptor homo- and/or heterodimerization is an extremely important mechanism in the regulation of cellular activities and physiological and/or pathological processes.

A dimer in the sense of the present invention is present if two polypeptide chains are associated and form a biologically relevant complex. The dimer may be a membrane-bound dimer or it may be a dimer present in the circulation, i.e. a complex of two polypetides that is stable under physiological conditions. In one embodiment the two polypeptide chains in a dimer (as they occur in nature) are not covalently linked but held together by protein-protein interactions, e.g. based on ion bridges or on van der Wals forces.

The bivalent binding agent according to the present invention is a binding agent comprising exactly two monovalent binders.

In a preferred embodiment the kinetic rate properties of each monovalent binder and of the bivalent binding agent are characterized by Biacore™ SPR technology as described in detail in the examples.

As the skilled artisan will appreciate the bivalent binding agent described in the present invention can be isolated and purified as desired. In one embodiment the present invention relates to an isolated bivalent binding agent as disclosed herein. An "isolated" bivalent binding agent is one which has been identified and separated and/or recovered from e.g. the reagent mixture used in the synthesis of such bivalent binding agent. Unwanted components of such reaction mixture are e.g. monovalent binders that did not end up in the desired bivalent binding agent. In one embodiment, the bivalent binding agent is purified to greater than 80%. In some embodiments, the bivalent binding agent is purified to greater than 90%, 95%, 98% or 99% by weight, respectively. In case both monovalent binders are polypeptides purity is e.g. easily determined by SDS-PAGE under reducing or nonreducing conditions using, for example, Coomassie blue or silver stain in protein detection. In case purity is assessed on the nucleic acid level, size exclusion chromatography is applied to separate the bivalent binding agent from side products and the OD at 260 nm is monitored to assess its purity.

The articles "a" and "an" are used herein to refer to one or to more than one (i.e., to at least one) of the grammatical object of the article. By way of example, "an antibody" means one antibody or more than one antibody.

The term "oligonucleotide" or "nucleic acid sequence" as used herein, generally refers to short, generally single stranded, polynucleotides that comprise at least 8 nucleotides and at most about 1000 nucleotides. In a preferred embodiment an oligonucleotide will have a length of at least 9, 10, 11, 12, 15, 18, 21, 24, 27 or 30 nucleotides. In a preferred embodiment an oligonucleotide will have a length of no more than 200, 150, 100, 90, 80, 70, 60, 50, 45, 40, 35 or 30 nucleotides. The description given below for polynucleotides is equally and fully applicable to oligonucleotides.

The term oligonucleotide is to be understood broadly and includes DNA and RNA as well as analogs and modification thereof.

An oligonucleotide may for example contain a substituted nucleotide carrying a substituent at the standard bases deoxyadenosine (dA), deoxyguanosine (dG), deoxycytosine (dC), deoxythymidine (dT), deoxyuracil (dU). Examples of such substituted nucleobases are: 5-substituted pyrimidines like 5 methyl dC, aminoallyl dU or dC, 5-(aminoethyl-3-acrylimido)-dU, 5-propinyl-dU or -dC, 5 halogenated - dU or -dC; N substituted pyrimidines like N4-ethyl-dC; N substituted purines like N6-ethyl-dA, N2-ethyl-dG; 8 substituted purines like 8-[6-amino)-hex-1-yl]-8-amino-dG or -dA, 8 halogenated dA or dG, 8 -alkyl dG or dA; and 2 substituted dA like 2 amino dA.

An oligonucleotide may contain a nucleotide or a nucleoside analog. I.e. the naturally occurring nucleobases can be exchanged by using nucleobase analogs like 5-Nitroindol d riboside; 3 nitro pyrrole d riboside, deoxyinosine (dI), deoyxanthosine (dX); 7 deaza -dG, -dA, -dI or -dX; 7-deaza-8-aza -dG, -dA, -dI or -dX; 8-aza -dA, -dG, -dI or -dX; d Formycin; pseudo dU; pseudo iso dC; 4 thio dT; 6 thio dG; 2 thio dT; iso dG; 5-methyl-iso-dC; N8-linked 8-aza-7-deaza-dA; 5,6-dihydro-5-aza-dC; and etheno-dA or pyrollo-dC. As obvious to the skilled artisan, the nucleobase in the complementary strand has to be selected in such manner that duplex formation is specific. If, for example, 5-methyl-iso-dC is used in one strand (e.g. (a)) iso dG has to be in the complementary strand (e.g. (a')).

The oligonucleotide backbone may be modified to contain substituted sugar residues, sugar analogs, modifications in the internucleoside phosphate moiety, and/or be a PNA.

An oligonucleotide may for example contain a nucleotide with a substituted deoxy ribose like 2'-methoxy, 2'-fluoro, 2'-methylseleno, 2'-allyloxy, 4'-methyl dN (wherein N is a nucleobase, e.g., A, G, C, T or U).

Sugar analogs are for example Xylose; 2',4' bridged Ribose like (2'-O, 4'-C methylene)- (oligomer known as LNA) or (2'-O, 4'-C ethylene)- (oligomer known as ENA); L-ribose, L- d-ribose, hexitol (oligomer known as HNA); cyclohexenyl (oligomer known as CeNA); altritol (oligomer known as ANA); a tricyclic ribose analog where C3' and C5' atoms are connected by an ethylene bridge that is fused to a cyclopropane ring (oligomer known as tricycloDNA); glycerin (oligomer known as GNA); Glucopyranose (oligomer known as Homo DNA); carbaribose (with a cyclopentan instead of a tetrahydrofuran subunit); hydroxymethyl-morpholin (oligomers known as morpholino DNA).

A great number of modification of the internucleosidic phosphate moiety are also known not to interfere with hybridization properties and such backbone modifications can also be combined with substituted nucleotides or nucleotide analogs. Examples are phosphorthioate, phosphordithioate, phosphoramidate and methylphosphonate oligonucleotides.

PNA (having a backbone without phosphate and d-ribose) can also be used as a DNA analog.

The above mentioned modified nucleotides, nucleotide analogs as well as oligonucleotide backbone modifications can be combined as desired in an oligonucleotide in the sense of the present invention.

The terms "polypeptide" and "protein" are used inter-changeably. A polypeptide in the sense of the present invention consists of at least 5 amino acids linked by alpha amino peptidic bonds.

A "target polypeptide" is a polypeptide of interest for which a method for determination or measurement is sought. The target polypeptide of the present invention is a polypeptide known or suspected to form a homo- or a heterodimeric polypeptide complex.

A "monovalent binder" according to the present invention is a molecule interacting with the target polypeptide at a single binding site with a Kdiss of 5x10⁻³/sec to 10⁻⁴/sec. The biophysical characterization of kinetic binding rate properties, respectively the determination of the dissociation rate constant kd(1/s) according to a Langmuir model is preferably analyzed by biosensor-based surface plasmon resonance spectroscopy. Preferably the Biacore™ technology as described in detail in the Examples section is used.

Examples of monovalent binders are peptides, peptide mimetics, aptamers, spiegelmers, darpins, lectines, ankyrin repeat proteins, Kunitz type domains, single domain antibodies, (see: Hey, T. et al., Trends Biotechnol 23 (2005) 514-522) and monovalent fragments of antibodies.

As obvious to the skilled artisan, each monovalent binder according to the present invention only binds to a single epitope on a target polypeptide.

In certain preferred embodiments the monovalent binder is a monovalent antibody fragment, preferably a monovalent fragment derived from a monoclonal antibody.

Monovalent antibody fragments include, but are not limited to Fab, Fab'-SH (Fab'), single domain antibody, Fv, and scFv fragments, as provided below.

In a preferred embodiment at least one of the monovalent binders is a single domain antibody, an Fab-fragment or an Fab'-fragment of a monoclonal antibody.

It also represents a preferred embodiment that in the bivalent binding agent disclosed herein both the monovalent binders are derived from monoclonal antibodies and are Fab-fragments, or Fab'-fragments or an Fab-fragment and an Fab'-fragment.

Monoclonal antibody techniques allow for the production of extremely specific binding agents in the form of specific monoclonal antibodies or fragments thereof. Particularly well known in the art are techniques for creating monoclonal antibodies, or fragments thereof, by immunizing mice, rabbits, hamsters, or any other mammal with a polypeptide of interest. Another method of creating monoclonal antibodies, or fragments thereof, is the use of phage libraries of sFv (single chain variable region), specifically human sFv. (See e.g., Griffiths et al., U.S. Pat. No. 5,885,793; McCafferty et al., WO 92/01047; Liming et al., WO 99/06587).

Antibody fragments may be generated by traditional means, such as enzymatic digestion or by recombinant techniques. For a review of certain antibody fragments, see Hudson, P.J. et al., Nat. Med. 9 (2003) 129-134.

An Fv is a minimum antibody fragment that contains a complete antigen-binding site and is devoid of constant region. In one embodiment, a two-chain Fv species consists of a dimer of one heavy- and one light-chain variable domain in tight, noncovalent association. In one embodiment of a single-chain Fv (scFv) species, one heavy- and one light-chain variable domain can be covalently linked by a flexible peptide linker such that the light and heavy chains can associate in a dimeric structure analogous to that in a two-chain Fv species. For a review of scFv, see, e.g., Plueckthun, In: The Pharmacology of Monoclonal Antibodies, Vol. 113, Rosenburg and Moore (eds.), Springer-Verlag, New York (1994), pp. 269-315; see also WO 93/16185; and U.S. Patent Nos. 5,571,894 and 5,587,458. Generally, six hyper variable regions (HVRs) confer antigen-binding specificity to an antibody. However, even a single variable domain (or half of an Fv comprising only three HVRs specific for an antigen) has the ability to recognize and bind antigen.

An Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group.

Various techniques have been developed for the production of antibody fragments. Traditionally, antibody fragments were derived via proteolytic digestion of intact antibodies (see, e.g., Morimoto, K. et al., Journal of Biochemical and Biophysical Methods 24 (1992) 107-117; and Brennan et al., Science 229 (1985) 81-83). For example, papain digestion of antibodies produces two identical antigen-binding fragments, called "Fab" fragments, each with a single antigen-binding site, and a residual "Fc" fragment, whose name reflects its ability to crystallize readily.

Antibody fragments can also be produced directly by recombinant host cells. Fab, Fv and scFv antibody fragments can all be expressed in and secreted from E. coli, thus allowing the facile production of large amounts of these fragments. Antibody fragments can be isolated from the antibody phage libraries according to standard procedures. Alternatively, Fab'-SH fragments can be directly recovered from E. coli (Carter, P. et al., Bio/Technology 10 (1992) 163-167). Mammalian cell systems can be also used to express and, if desired, secrete antibody fragments.

In certain embodiments, a monovalent binder of the present invention is a single-domain antibody. A single-domain antibody is a single polypeptide chain comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., U.S. Patent No. 6,248,516 B1). In one embodiment, a single-domain antibody consists of all or a portion of the heavy chain variable domain of an antibody.

One of the two monovalent binders, the first monovalent binder, binds to an epitope on a first (target) polypeptide.

An "epitope" according to the present invention - the binding site on the target polypeptide bound by the corresponding monovalent binder - is composed of amino acids. The amino acids are naturally occurring amino acids and may cary one or more secondary modification. In one embodiment the amino acids are not secondarily modified. In one embodiment the monovalent binder either binds to a linear epitope, i.e. an epitope consisting of a stretch of 5 to 12 consecutive amino acids, or the monovalent binder binds to a tertiary structure formed by the spatial arrangement of the target polypeptide. Tertiary epitopes recognized by a binder, e.g. by the antigen recognition site or paratope of an antibody, can be thought of as three-dimensional surface features of an antigen molecule; these features fit precisely (in)to the corresponding binding site of the binder and thereby binding between binder and target polypeptide is facilitated.

In the bivalent binding agent as disclosed herein the first monovalent binder binds to an epitope on a first polypeptide and the second monovalent binder binds to a epitope on a second polypeptide. The first and the second polypeptide in a polypeptide dimer can have an identical sequence, i.e. this dimer is a homodimer, or the first and the second polypeptide can be different, i.e. this dimer is a heterodimer.

As mentioned above, for many receptor molecules the formation of a polypeptide homo- or heterodimer, respectively, is key to the regulation of cell signaling and protein activity. This is especially known and true for membrane-bound receptors, especially the so-called receptor tyrosine kinases (RTKs). As the nomenclature already suggests, at least part of the intracellular signaling of the RTKs is mediated by the phosphorylation status of certain tyrosine of the intracellur domain of such RTKs. In one preferred embodiment the present invention thus relates to a bivalent binding agent binding to a receptor polypeptide dimer. Obviously such bivalent binding agent is of great utility in the detection of active homo- or hetrodimeric receptor polypeptides.

In one preferred embodiment the present invention relates to a bivalent binding agent as disclosed herein above, wherein the target polypeptide dimer is selected from the group consisting of membrane-bound receptor molecules, preferably from homo- or heterodimers formed by association of two receptor tyrosine kinase polypeptides.

In some embodiments, the RTK polypeptide being part of a homo- or heterodimer is selected from the group consisting of: ALK, adhesion related kinase receptor (e.g., Axl), ERBB receptors (e.g., EGFR, ERBB2, ERBB3, ERBB4), erythropoietin-producing hepatocellular (EPH) receptors (e.g., EphA1; EphA2, EphA3, EphA4, EphA5, EphA6, EphA7, EphA8, EphB1, EphB2, EphB3, EphB4, EphB5, EphB6), fibroblast growth factor (FGF) receptors (e.g., FGFR1, FGFR2, FGFR3, FGFR4, FGFR5), Fgr, IGFIR, Insulin R, LTK, M-CSFR, MUSK, platelet-derived growth factor (PDGF) receptors (e.g., PDGFR-A, PDGFR-B), RET, ROR1, ROR2, ROS, RYK, vascular endothelial growth factor (VEGF) receptors (e.g., VEGFR1/FLT1, VEGFR2/FLK1, VEGF3), tyrosine kinase with immunoglobulin-like and EGF-like domains (TIE) receptors (e.g., TIE-1, TIE-2/TEK), Tec, TYRO10, insulin-like growth factor (IGF) receptors (e.g., INS-R, IGF-IR, IR-R), Discoidin Domain (DD) receptors (e.g., DDR1, DDR2), receptor for c-Met (MET), recepteur d'origine nantais (RON); also known as macrophage stimulating 1 receptor, Flt3 fins-related tyrosine kinase 3 (Flt3), colony stimulating factor 1(CSF1) receptor, receptor for c-kit (KIT, or SCFR) and insulin receptor related (IRR) receptors.

In one embodiment, the RTK being part of a homo- or heterodimer is selected from the group consisting of: ERBB receptors (e.g., EGFR, ERBB2, ERBB3, ERBB4), platelet-derived growth factor (PDGF) receptors (e.g., PDGFR-A, PDGFR-B) and vascular endothelial growth factor (VEGF) receptors and co-receptors (e.g., VEGFR1/FLT1, VEGFR2/FLK1, VEGF3, neuropilin-1, neuropilin-2), insulin-like growth factor (IGF) receptors (e.g., INS-R, IGF-IR, IR-R), and insulin receptor related (IRR) receptors.

In one embodiment, the RTK being part of a homo- or heterodimer is selected from the group consisting of: ERBB receptors (e.g., EGFR, ERBB2, ERBB3, ERBB4), platelet-derived growth factor (PDGF) receptors (e.g., PDGFR-A, PDGFR-B) and vascular endothelial growth factor (VEGF) receptors and co-receptors (e.g., VEGFR1/FLT1, VEGFR2/FLK1, VEGF3, neuropilin-1, neuropilin-2), insulin-like growth factor (IGF) receptors (e.g., INS-R, IGF-IR, IR-R), and insulin receptor related (IRR) receptors.

In one embodiment the present invention relates to a bivalent binding agent capable of binding a polypeptide dimer the binding agent consisting of two monovalent binders that are linked to each other via a linker, wherein the first monovalent binder binds to an epitope of a first target polypeptide comprised in said dimer, wherein the second monovalent binder binds to an epitope of a second target polypeptide comprised in said dimer, wherein each monovalent binder has a Kdiss in the range of 5x10⁻³/sec to 10⁻⁴/sec, wherein the bivalent binding agent has a Kdiss of 3x10⁻⁵/sec or less and wherein said dimer is a receptor polypeptide dimer selected from the group consisting of ERBB receptors (e.g., EGFR, ERBB2, ERBB3, ERBB4) and vascular endothelial growth factor (VEGF) receptors and co-receptors (e.g., VEGFR1/FLT1, VEGFR2/FLK1, VEGF3, neuropilin-1, neuropilin-2).

In one embodiment receptor homo- and/or heterodimerization relates to the polypeptides involved in VEGF-signaling. It includes the vascular endothelial growth factors and their corresponding receptors as one specific example (for full details see Otrock, Z. et al., Blood Cells, Molecules and Diseases: 38 (2007) 258-268).

VEGF signaling often represents a critical rate-limiting step in physiological angiogenesis. Angiogenesis is the sprouting of new blood vessels from the pre-existing ones. This process is important for the growth of new blood vessels during fetal development and tissue repair; however, uncontrolled angiogenesis promotes neoplastic diseases and other disorders. The successful implementation of this process depends upon the balance of growth promoting factors and growth inhibitory factors. One of the most specific and crucial regulators of angiogenesis is VEGF.

The VEGF family comprises seven secreted glycoproteins that are designated VEGF-A, VEGF-B, VEGF-C, VEGF-D, VEGF-E, placental growth factor (P1GF) and VEGF-F. The VEGF family members bind their cognate receptors. The receptors identified so far are designated VEGFR-1, VEGFR-2, VEGFR-3 and the neuropilins (NP-1 and NP-2).

VEGF-A exerts its biologic effect through interaction with cell-surface receptors. These receptors are transmembrane tyrosine kinase receptors and they include VEGF receptor-1 (VEGFR-1; Flt-1) and VEGFR-2 (kinase insert domain-containing receptor/Flk-1), selectively expressed on vascular endothelial cells, and the neuropilin receptors (NP-1 and NP-2), expressed on vascular endothelium and neurons. Upon binding of VEGF-A to the extracellular domain of the receptor, a cascade of downstream proteins is activated after the dimerization and autophosphorylation of the intracellular receptor tyrosine kinases. VEGFR-2 appears to be the main receptor responsible for mediating the proangiogenic effects of VEGF-A.

VEGFR-1 (fins-like tyrosine kinase; Flt-1) is composed of seven extracellular immunoglobulin (Ig) homology domains, a single transmembrane region and an intracellular tyrosine kinase domain. VEGFR-1 binds VEGF-A, VEGF-B and P1GF with high affinity.

VEGFR-2 (KDR, human; Flk-1, mouse) was first isolated in 1991 and was named Kinase-insert domain containing receptor (KDR). Like VEGFR-1, VEGFR-2 bears an extracellular region with seven immunoglobulin (Ig)-like domains, a transmembrane domain and a tyrosine kinase domain with about 70-amino-acid insert. VEGFR-2 binds VEGF-A, VEGF-C, VEGF-D and VEGF-E.

VEGFR-3 (fins-like tyrosine kinase 4, Flt4), a member of the endothelial cells receptor tyrosine kinases, has only six Ig-homology domains [121]. VEGFR-3 preferentially binds VEGF-C and VEGF-D.

The VEGF receptors 1, 2 and 3 upon ligand binding can form homo-dimers that are active and exert their effects via the intracellular tyrosine kinase domain. However, homodimer formation is not the only way of action and the so-aclled neuropilins play an important role as co-receptors.

Neuropilin NP-1 was identified initially as a 130- to 140-kDa cell-surface glycoprotein that served as a receptor for the semaphorin/collapsins, a large family of secreted and transmembrane proteins that serve as repulsive guidance signals in axonal and neuronal development. NP-1 binds VEGF-A, VEGF-B and P1GF while NP-2 binds VEGF-A, VEGF-C and P1GF.

NP-1 acts as a co-receptor enhancing VEGF-A-VEGFR-2 interactions, forming complexes with VEGFR-1 and augmenting tumor angiogenesis in vivo. In order to investigate this pathway the specific detection of a VEGFR-2/NP-1 heterodimer is required.

Recent studies have linked NP-2 to lymphatic vessel development.

As has been pointed out above the members of VEGF family of receptors as long as they are monomeric are not active, however, upon ligand binding and homo- and/or heterodimerization an array of different biochemical pathways, leading to different modes of action is induced. The capability to differentiate between (inactive) monomers and the various homo- and/or heterodimers is of utmost importance in elucidating the physiological mode of action of the VEGF/VEGF receptor system and the effect of drugs targeting one or more members of this system. In one embodiment the present invention relates to a bivalent binding agent according to the invention, the bivalent binding agent being capable of binding a polypeptide dimer, the binding agent consisting of two monovalent binders that are linked to each other via a linker, wherein the first monovalent binder binds to an epitope of a first target polypeptide comprised in said dimer, wherein the second monovalent binder binds to an epitope of a second target polypeptide comprised in said dimer, wherein each monovalent binder has a Kdiss in the range of 5x10⁻³/sec to 10⁻⁴/sec, wherein the bivalent binding agent has a Kdiss of 3x10⁻⁵/sec or less and wherein the homo- and/or heterodimer is part of the VEGF/VEGF receptor system. In such binder in one embodiment one monovalent binder binds to a VEGF selected from the group consisting of VEGF-A, VEGF-B, VEGF-C, VEGF-D, VEGF-E, placental growth factor (P1GF) and VEGF-F and the other monovalent binder binds to a receptor selected from the group consisting of VEGFR-1, VEGFR-2, VEGFR-3 and the neuropilins (NP-1 and NP-2). Or, such binder in one embodiment has one monovalent binder that binds to a to a receptor polypeptide selected from the group consisting of VEGFR-1, VEGFR-2 and VEGFR-3 and the other monovalent binder binds to a neuropilin (NP-1 or NP-2).

As discussed a monovalent binder for use in the construction of a bivalent binding agent as disclosed herein has to have a kdiss from 5x10⁻³/sec to 10⁻⁴/sec.

Preferably, the first monovalent binder is specifically binding to an epitope on the first polypeptide. I.e. this binder binds to an epitope that is either not present on the second target polypeptide as is the case for a heterodimer bivalent binding agent or it binds to an epitope that overlaps with or is identical to an epitope present on the second target polypeptide as is the case for for a homodimer bivalent binding agent.

Specific binding to an epitope is acknowledged if said binder has a Kdiss that is at least 20 times lower for the epitope on the target polypeptide as compared to any (similar or completely un-related) epitope on any other peptide (e.g. on the second target polypeptide if the first and the second target polypeptide are different). Polypeptides in a heterodimer are acknowleged to be different if a monovalent binder specifically binds to only one of the polypeptides. Also preferred the Kdiss of the first monovalent binder to the epitope on the first target polypeptide is at least 30-, 40-, 50-, 80-, 90-, 95- or at least 100-fold lower as compared to any epitope on any other polypeptide.

In one preferred embodiment the bivalent binding agent disclosed herein binds to a heterodimer, comprising two different polypeptides. In this case the the second monovalent binder is preferably selected to have a Kdiss that is at least 20 times lower for the first polypeptide as compared to the Kdiss for its binding to the second target polypeptide. Also preferred the Kdiss of the second monovalent binder to the second target polypeptide is at least 30-, 40-, 50-, 80- 90-, 95- or at least 100-fold lower as compared to the Kdiss for any epitope on the first target polypeptide.

As mentioned above the bivalent binding agent according to the present invention will have a Kdiss of at most 3x10⁻⁵/sec or lower, i.e. better.

In one embodiment in the bivalent binding agent according to this invention each monovalent binder has a Kdiss from 2x10⁻³/sec to 10⁻⁴/sec.

In one embodiment in the bivalent binding agent according to this invention each monovalent binder has a Kdiss from 10⁻³/sec to 10⁻⁴/sec.

The automatic immunohistochemistry staining machines distributed by Ventana Medical Systems Inc. Tucson employ rather stringent washing conditions. An antibody used on the BenchMark® analyzer series should have a Kdiss of at most 5x10⁻⁵/sec in order to give a reasonable staining intensity. The lower the Kdiss, the better the staining intensity will be. The bivalent binding agent as disclosed herein has a Kdiss of at most 3x10⁻⁵/sec. In a further embodiment the bivalent binding agent as disclosed herein has a Kdiss of 2x10⁻⁵/sec or less or also preferred of 10⁻⁵/sec or less.

In one embodiment the kinetic rate properties of each monovalent binder and of the bivalent binding agent are characterized by Biacore™ SPR technology as described in detail in the examples.

The bivalent binding agent according to the present invention contains a linker. The linker can either covalently link the two monovalent binders or, according to the invention, the linker and the monovalent binders can be bound by two different specific binding pairs a:a' and b:b'.

The linker may for example be composed of appropriate monomers, linked together and to the two monovalent binders by co-valent bonds. Preferably the linker may contain sugar moieties, nucleotide moieties, nucleoside moieties and/or amino acids. In certain preferred embodiments the linker may essentially consist of nucleotides, nucleotide analogues or amino acids.

In aspects as disclosed in here, the linker covalently linking, or, according to the invention binding the two monovalent binders via binding pairs has a length of 6 to 100 nm. Also preferred the linker has a length of 6 to 50 nm or of 6 to 40 nm. In an also preferred embodiment the linker will have a length of 10 nm or longer or of 15 nm or longer. In one embodiment the linker comprised in a bivalent binding agent according to the present invention has between 10 nm and 50 nm in length.

The length of non-nucleosidic entities of a given linker (a-S-b) in theory and by complex methods can be calculated by using known bond distances and bond angles of compounds which are chemically similar to the non-nucleosidic entities.

Such bond distances are summarized for some molecules in standard text books: CRC Handbook of Chemistry and Physics, 91st edition, 2010-2011, section 9. However, exact bond distances vary for each compound. There is also variability in the bond angles.

It is therefore more practical to use an average parameter (an easy to understand approximation) in such calculation.

In the calculation of a spacer or a linker length the following approximations apply: a) for calculating lenghts of nonnucleosidic entities an average bond length of 130 pm with an bond angle of 180° independently of the nature of the linked atoms is used; b) one nucleotide in a single strand is calculated with 500 pm and c) one nucleotide in a double strand is calculated with 330 pm.

The value of 130 pm is based on calculation of the distance of the two terminal carbonatoms of a C(sp3)-C(sp3)-C(sp3) chain with a bond angle of 109°28' and a distance of 153 pm between two C(sp3) which is approx 250 pm which translates with an assumed bond angle of 180° to and bond distance between two C(Sp3) with 125 pm. Taking in account that heteroatoms like P and S and sp2 and sp1 C atoms could also be part of the spacer the value 130 pm is taken. If a spacer comprises a cyclic structure like cycloalkyl or aryl the distance is calculated in analogous manner, by counting the number of the bonds of said cyclic structure which are part of the overall chain of atoms that are defining the distance

As mentioned above, the linker can either covalently link the two monovalent binders or, according to the invention, the linker and the monovalent binders can be bound by two different specific binding pairs a:a' and b:b'. Therefore, the bivalent binding agent according to the present invention, binding to a polypeptide dimer, can be also depicted by the below Formula I:

A - a':a - S - b:b' - B,

wherein A is a first monovalent binder, binding to an epitope of said first target polypeptide, wherein B is a second monovalent binder, binding to an epitope of said second target polypeptide, wherein each monovalent binder A and B has a Kdiss in the range of 5x10⁻³/sec to 10⁻⁴/sec, wherein according to the invention a':a as well as b:b' independently are a binding pair, wherein according to the invention a':a and b:b' are different, wherein S is a spacer, wherein - represents a covalent bond, wherein the linker a - S - b has a length of 6 to 100 nm and wherein the bivalent binding agent has a Kdiss of 3x10⁻⁵/sec or less.

The linker L consisting of a - S - b has a length of 6 to 100 nm. Preferably the linker L consisting of a - S - b has a length of 6 to 80 nm. Also preferred the linker has a length of 6 to 50 nm or of 6 to 40 nm. In an also preferred embodiment the linker will have a length of 10 nm or longer or of 15 nm in length or longer. In one embodiment the linker has between 10 nm and 50 nm in length. In one embodiment a and b, respectively, are binding pair members and have a length of at least 2.5 nm each.

The spacer S can be construed as required to e.g. provide for the desired length as well as for other desired properties. The spacer can e.g. be fully or partially composed of naturally occurring or non-naturally occurring amino acids, of phosphate-sugar units e.g. a DNA like backbone without nucleobases, of glyco-peptidic structures, or at least partially of saccharide units or at least partially of polymerizable subunits like glycols or acryl amide.

The length of spacer S in a compound according to the present invention may be varied as desired. In order to easily make available spacers of variable length, a library, it is preferred to have a simple synthetic access to the spacers of such library. A combinatorial solid phase synthesis of a spacer is preferred. Since spacers have to synthesized up to a length of about 100 nm, the synthesis strategy is chosen in such a manner that the monomeric synthetic building blocks are assembled during solid phase synthesis with high efficiency. The synthesis of deoxy oligonucleotides based on the assembly of phosphoramidite as monomeric building blocks perfectly meet this requirements. In such spacer monomeric units within a spacer are linked in each case via a phosphate or phosphate analog moiety.

The spacer S can contain free positively or/and negatively charged groups of polyfunctional amino-carboxylic acids, e.g. amino, carboxylate or phosphate. For example the charge carriers can be derived from trifunctional aminocarboxylic acids which contain a) an amino group and two carboxylate groups or b) two amino groups and one carboxylate group. Examples of such trifunctional aminocarboxylic acids are lysine, ornithine, hydroxylysine, α,β-diamino propionic acid, arginine, aspartic acid and glutamic acid, carboxy glutamic acid and symmetric trifunctional carboxylic acids like those described in EP-A-0 618 192 or US-A-5,519,142. Alternatively one of the carboxylate groups in the trifunctional aminocarboxylic acids a) can be replaced by a phosphate, sulphonate or sulphate group. An example of such a trifunctional amino acid is phosphoserine.

The spacer S can also contain uncharged hydrophilic groups. Preferred examples of uncharged hydrophilic groups are ethylene oxide or polyethylene oxide groups with preferably at least three ethylene oxide units, sulphoxide, sulphone, carboxylic acid amide, carboxylic acid ester, phosphonic acid amide, phosphonic acid ester, phosphoric acid amide, phosphoric acid ester, sulphonic acid amide, sulphonic acid ester, sulphuric acid amide and sulphuric acid ester groups. The amide groups are preferably primary amide groups, particularly preferably carboxylic acid amide residues in amino acid side groups e.g. the amino acids asparagine and glutamine. The esters are preferably derived from hydrophilic alcohols, in particular C1-C3 alcohols or diols or triols.

In one embodiment the spacer S is composed of one type of monomer. For example, the spacer is composed exclusively of amino acids, of sugar residues, of diols, of phospho-sugar units or it can be a nucleic acid, respectively.

In one embodiment, the spacer is DNA. In one preferred embodiment the spacer is the _{L}-stereoisomer of DNA also known as beta- _{L}-DNA, _{L}-DNA or mirror image DNA. _{L}-DNA features advantages like orthogonal hybridization behaviour, which means that a duplex is formed only between two complementary single strands of L-DNA but no duplex is formed between a single strand of L-DNA and the complementary DNA strand, nuclease resistance and ease of synthesis even of a long spacer. As pointed out ease of synthesis and variability in spacer length are important for a spacer library. Spacers of variable length are extremely utile in identifying the bivalent dual binder according to the present invention having a spacer of optimal length thus providing for the optimal distance between the two monovalent binders.

Spacer building blocks, as the name says, can be used to introduce a spacing moiety into the spacer S or to build the spacer S of the linker a-S-b.

Different numbers and kinds of non-nucleotidic as well nucleotidic spacer building blocks are at hand for introducing spacing moieties.

Many different non nucleotidic bifunctional spacer building blocks are known in literature and a great variety is commercially available. The choice of the non nucleotidic bifunctional spacer building is influencing the charge and flexibility of the spacer molecule.

In bifunctional spacer building blocks a hydroxyl group which is protected with an acid labile protecting group is connected to a phosphoramidite group.

Bifunctional spacer building blocks in one embodiment are non-nucleosidic compounds. For example, such spacers are C2 - C18 alkyl, alkenyl, alkinyl carbon chains, whereas said alkyl, alkenyl, alkinyl chains may be interrupted by additional ethyleneoxy and/or amide moieties or quarternized cationic amine moieties in order to increase hydrophilicity of the linker. Cyclic moieties like C5-C6-cycloalkyl, C4N, C5N, C4O,C5O- heterocycloalkyl, phenyl which are optionally substituted with one or two C1-C6 alkyl groups can also be used as nonnucleosidic bifunctional spacer moieties. Preferred bifunctional building blocks comprise C3-C6 alkyl moieties and tri- to hexa- ethyleneglycol chains. Table I shows some examples of nucleotidic bifunctional spacer building blocks with different hydrophilicity, different rigidity and different charges. One oxygen atom is connected to a acid labile protecting group preferably dimethoxytrityl and the other is part of a phosphoramidite.

**Table I: Examples of non-nucleotidic bifunctional spacer building blocks**

| Non-nucleotidic bifunctional spacer building blocks | Reference |
|---|---|
| 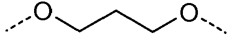 | Seela, F., Nucleic Acides Research 15 (1987) 3113-3129 |
| 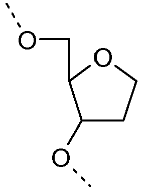 | Iyer, R.P., Nucleic Acids Research 18 (1990) 2855-2859 |
| 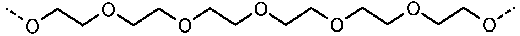 | WO 89/02931 A1 |
| 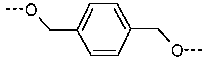 | EP 1 538 221 |
| 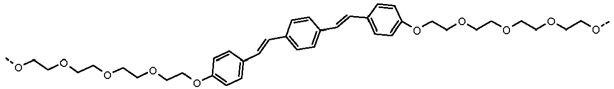 | US 2004/224372 |
| 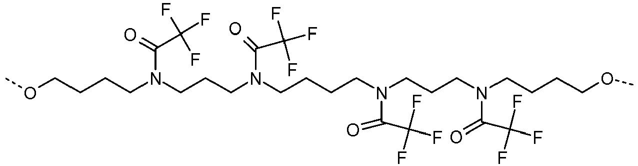 | WO 2007/069092 |

A simple way to build the spacer S or to introduce spacing moieties into the spacer S is to use standard D or L nucleoside phosphoramidite building blocks. In one embodiment a single strand stretch of dT is used. This is advantageous, because dT does not carry a base protecting group.

Hybridization can be used in order to vary the spacer length (distance between the binding pair members a and b) and the flexibility of the spacer, because the double strand length is reduced compared to the single strand and the double strand is more rigid than a single strand.

For hybridization in one embodiment oligonucleotides modified with a functional moiety X are used. The oligonucleotide used for hybridization can have one or two terminal extentions not hybridizing with the spacer and/or is branched internally. Such terminal extensions that are not hybridizing with the spacer (and not interfering with the binding pairs a:a' and b:b') can be used for further hybridization events. In one embodiment an oligonucleotide hybridizing with a terminal extension is labeled oligonucleotide. This labeled oligonucleotide again may comprise terminal extensions or being branched in order to allow for further hybridization, thereby a polynucleotide aggregate or dendrimer can be obtained. A poly-oligonucleic acid dendrimer is preferably used in order to produce a polylabel. or in order to get a high local concentration of X.

In one embodiment the spacer S has a backbone length of 1 to 100 nm. With other words here the groups a and b of Formula I are between 1 and 100 nm apart. In one embodiment a and b, respectively, each are a binding pair member and the spacer S has a backbone length of 1 to 95 nm.

"a':a" as well as "b:b"' each independently represent a binding pair, respectively.

"a':a" as well as "b:b"' are different. The term different indicates that the binding of a to a' (intra-binding pair-binding according to the invention) does not interfere with the intra-binding pair-binding of the other pair b to b', and vice versa.

In an embodiment separate from the claimed invention, "a':a" as well as "b:b"' signify covalent coupling. Thus, the binding of a to a' (covalent coupling) does not interfere with the covalent coupling of the other pair b to b', and vice versa.

In the separate embodiment as disclosed herewith, either a':a or b:b' are bound covalently and the other, i.e., b:b' or a':a, respectively, represents a binding pair.

In one further separate embodiment as disclosed herewith, both a':a and b:b' are bound covalently.

In the separate embodiments the coupling chemistry between a':a and b:b' is different from one another and selected from standard protocols. Depending on the nature of the binding partner and of the spacer, appropriate conjugation chemistries are chosen.

The chemistry used in coupling (a') to (a), i.e. in coupling A-(a') to a linker comprising (a) does not interfere with the chemistry used in coupling (b) to (b'), i.e. in coupling (b')-B to a linker comprising (b). As the skilled artisan will appreciate, the reactive sites (a), (a'), (b) and (b'), respectively, leading to the covalent bond a':a as well as b:b', respectively, preferably also do not to interfere with any functional group that might be present on a monovalent binder (A and/or B of Formula I).

In case at least one of the monovalent binders is a protein, a peptide or a peptide mimic, it likely carries one or more OH, COOH, NH2 and/or SH groups, which could potentially react with certain coupling reagents. Such (side-)reaction can be avoided by selecting e.g. one of the coupling chemistries given in Table II.

Table II provides an overview over routinely used reactive groups for binding A-(a') and (b')-B, respectively, to (a) and (b), respectively, both being covalently bound to the linker (a-S-b).

**Table II:**

| **reactive site (a') within A-(a')** | **reactive site (a) of the linker L: (a)-S-(b)** | **reactive site (b) of the linker L: (a)-S-(b)** | **reactive site (b') within (b')-B** |
|---|---|---|---|
| ONH2 (aminoxy) | C(H)=O (aldehyde) | -C=C (alkyne) or triphenylphosphin carboxylic ester | N3 (azide) |
| C(H)=O (aldehyde) | ONH2 (aminoxy) | N3 (azide) | -C=C (alkyne) or triphenylphosphin carboxylic ester |
| ONH2 (aminoxy) | C(H)=O (aldehyde) | Dien | Dienophil |
| C(H)=O (aldehyde) | ONH2 (aminoxy) | Dienophil | Dien |
| Dien | Dienophil | N3 (azide) | -C=C (alkyne) or triphenylphosphin carboxylic ester |
| Dienophil | Dien | N3 (azide) | -C=C (alkyne) or triphenylphosphin carboxylic ester |
| Dienophil | Dien | --C=C (alkyne) or triphenylphosphin carboxylic ester | N3 (azide) |
| Dien | Dienophil | -C=C (alkyne) or triphenylphosphin carboxylic ester | N3 (azide) |

The above bi-orthogonal coupling chemistries are e.g. appropriate if at least one of the monovalent binders is a polypeptide. If the two binding partners are not carrying certain reactive functional groups, e.g. in the case of combination of two aptamers, as the monovalent binders A an B, respectively, there is more freedom in selection of the reactive sites (a'), (a), (b) and (b'), respectively. Therefore in addition or in combination with the pairs of corresponding reactive sites given in the above table, amino/active ester (e.g. NHS ester), and SH/SH or SH/maleinimido can be used for orthogonal coupling.

As obvious from the above examples at least one of the covalent bonds between a':a and between b:b', respectively is not an alpha amino peptide bond. Also preferred both covalent bonds are not alpha amino peptide bonds.

According to the invention, both a':a and b:b' are a binding pair. Consequently, an embodiment of the present invention comprises an at least bispecific binding agent of the Formula I: A - a':a - S - b:b' - B; wherein A is a first monovalent binder, binding to an epitope of a first target polypeptide, wherein B is a second monovalent binder, binding to an epitope on a second target polypeptide, wherein each monovalent binder A and B has a Kdiss in the range of 5x10⁻³/sec to 10⁻⁴/sec, wherein a':a as well as b:b' independently are a binding pair and are different, wherein S is a spacer, wherein - represents a covalent bond, wherein the linker a - S - b has a length of 6 to 100 nm and wherein the bivalent binding agent has a Kdiss of 3x10⁻⁵/sec or less.

In this embodiment a and a' are the members of the binding pair a':a and b and b' are the members of the binding pair b:b', respectively. Preferably each member of a binding pair is of a molecular weight of 10 kD or below. In further also preferred embodiments the molecular weight of each binder of such binding pair is 8, 7, 6, 5 or 4 kD or below.

According to the invention, a':a and b:b' are binding pairs and the members of the binding pairs a':a and b:b' are hybridizing nucleic acid sequences. According to the invention, both binding pairs are hybridizing nucleic acid sequences.

In case a:a' or b':b represents a binding pair, the binding affinity for (within) such binding pair is at least 10⁸ l/mol. Both binding pairs are different. For a binding pair difference is e.g. acknowledged if the affinity for the reciprocal binding, e.g. binding of a as well as a' to b or b' is 10% of the affinity within the pair a:a' or lower. Also preferred, the reciprocal binding, i.e. binding of a as well as a' to b or b', respectively, is 5% of the affinity within the pair a:a' or lower, or if it is 2% of the affinity within the pair a:a' or lower. In one embodiment the difference is so pronounced that the reciprocal (cross-reactive) binding is 1% or less as compared to the specific binding affinity within a binding pair.

In an embodiment separate from the claimed invention a':a and b:b' are leucine zipper domain dimers. In such an embodiment both binding pairs represent leucine zipper domain dimers.

The term "leucine zipper domain" is used to denote a commonly recognized dimerization domain characterized by the presence of a leucine residue at every seventh residue in a stretch of approximately 35 residues. Leucine zipper domains are peptides that promote oligomerization of the proteins in which they are found. Leucine zippers were originally identified in several DNA-binding proteins (Landschulz, W.H. et al., Science 240 (1988) 1759-1764), and have since been found in a variety of different proteins. Among the known leucine zippers are naturally occurring peptides and derivatives thereof that dimerize or trimerize. Examples of leucine zipper domains suitable for producing soluble multimeric proteins are described in PCT application WO 94/10308, and the leucine zipper derived from lung surfactant protein D (SPD) described in Hoppe, H.J. et al., FEBS Lett. 344 (1994) 191-195.

Leucine zipper domains form dimers (binding pairs) held together by an alpha-helical coiled coil. A coiled coil has 3.5 residues per turn, which means that every seventh residue occupies an equivalent position with respect to the helix axis. The regular array of leucines inside the coiled coil stabilizes the structure by hydrophobic and Van der Waals interactions.

If leucine zipper domains form the first binding pair (a':a) and the second binding pair (b:b'), both leucine zipper sequences are different, i.e. sequences a and a' do not bind to b and b'. Leucine zipper domains may be isolated from natural proteins known to contain such domains, such as transcription factors. One leucine zipper domain may e.g. come from the transcription factor fos and a second one from the transcription factor jun. Leucine zipper domains may also be designed and synthesized artificially, using standard techniques for synthesis and design known in the art.

In one preferred embodiment both members of the binding pairs a':a and b:b', i.e. a, a', b and b' represent leucine zipper domains and the spacer S consists of amino acids. In this embodiment production of the construct a-S-b is easily possible. Varying the length of such spacer S as desired is straightforward for a person skilled in the art. Such polypeptide can be synthesized or recombinantly produced.

E.g., recombinant fusion proteins comprising a spacer polypeptide fused to a leucine zipper peptide at the N-terminus and to a leucine zipper peptide at the C-terminus can be expressed in suitable host cells according to standard techniques. A DNA sequence coding for a desired peptide spacer can be inserted between a sequence coding for a member of a first leucine zipper domain a and in the same reading frame a DNA sequence coding for a member of a second leucine zipper domain b.

The spacer S, if the linker a-S-b is a polypeptide in one embodiment comprises once or several times a GGGGS (G4S) (SEQ ID NO:13) amino acid sequence motif. The spacer S may also comprise a tag sequence. The tag sequence may be selected from commonly used protein recognition tags such as YPYDVPDYA (HA-Tag) (SEQ ID NO:14) or GLNDIFEAQKIEWHE (Avi-Tag) (SEQ ID NO:15).

According to the invention, both binding pairs (a':a) and (b:b') are hybridizing nucleic acid sequences.

As indicated already by nomenclature, a and a' as well as b and b' hybridize to one another, respectively. The nucleic acid sequences comprised in a and a' one the one hand and in b and b' on the other hand are different. With other words the sequences of in the binding pair a':a do not bind to the sequences of the binding pair b:b', respectively, and vice versa. In one embodiment the present invention relates to an at least dual binding agent of Formula I, wherein the binding pairs a:a' and b:b', respectively, both are hybridizing nucleic acid sequences and wherein the hybridizing nucleic acid sequences of the different binding pairs a':a and b:b' do not hybridize with one another. With other words a and a' hybridize to each other but do not bind to any of b or b' or interfere with their hybridization and vice versa. Hybridization kinetics and hybridization specificity can easily be monitored by melting point analyses. Specific hybridization of a binding pair (e.g. a:a') and non-interference (e.g. with b or b') is acknowleged, if the melting temperature for the pair a:a' as compared to any possible combination with b or b', respectively, (i.e. a:b; a:b'; a':b and a':b') is at least 20°C higher.

The nucleic acid sequences forming a binding pair, e.g. (a:a') or any other nucleic acid sequence-based binding pair, may compromise any naturally occurring nucleobase or an analogue thereto and may have a modified or an un-modified backbone as described above, provided it is capable of forming a stable duplex via multiple base pairing. Stable means that the melting temperature of the duplex is higher than 37°C. Preferably the double strand consists of two fully complementary single strands. However mismatches or insertions are possible as long as the a stability at 37°C is given.

As the skilled artisan will appreciate a nucleic acid duplex can be further stabilized by interstrand crosslinking. Several appropriate cross-linking methods are known to the skilled artisan, e.g. methods using psoralen or based on thionucleosides.

The nucleic acid sequences representing the members of a binding pair preferably consist of between 12 and 50 nucleotides. Also preferred such nucleic acid sequences will consist of between 15 and 35 nucleotides.

RNAses are ubiquitous and special care has to be taken to avoid unwanted digestion of RNA-based binding pairs and/or spacer sequences. While it certainly is possible to use, e.g. RNA-based binding pairs and/or spacers, binding pairs and/or spacers based on DNA represent a preferred embodiment.

Appropriate hybridizing nucleic acid sequences can easily be designed to provide for more than two pairs of orthogonal complementary oligonucleotides, allowing for an easy generation and use of more than two binding pairs. Another advantage of using hybridizing nucleic acid sequences in a dual binding agent of the present invention is that modifications can be easily introduced into a nucleic acid sequences. Modified building blocks are commercially available which e.g. allow for an easy synthesis of a linker comprising a functional moiety. Such functional moiety can be easily introduced at any desired position and in any of the structures a and a' as well as b and b' and/or S, provided they represent an oligonucleotide. In one preferred embodiment the spacer S comprised in a binding agent according to Formula I is a nucleic acid. In one preferred embodiment both binding pairs are hybridizing nucleic acid sequences and the spacer S also is a nucleic acid. In this embodiment the linker L consisting of a - S - b is an oligonucleotide.

In case the spacer S as well as the sequences a, a', b and b' all are oligonucleotide sequences it is easily possible to provide for and synthesize a single oligonucleotide representing the linker L comprising S and the members a and b of the binding pairs a':a and b:b', respectively. In case the monovalent binders A and B, respectively, are polypeptides, they can each be coupled easily to the hybridizing nucleic acid sequences a' and b', respectively. The length of the spacer S comprised in such construct can easily be varied in any desired manner. Based on the three constructs a-S-b, A-a' and b'-B the binding agent of Formula I can be most easily obtained according to standard procedures by hybridization between a':a and b:b', respectively. When spacers of different length are used, the resulting constructs, provide for otherwise identical dual binding agents, yet having a different distance in between the monovalent binders A and B. This allows for optimal distance and/or flexibility.

In one preferred embodiment the spacer S as well as the sequences a, a', b and b' are DNA.

The enantiomeric L-DNA, is known for its orthogonal hybridization behavior, its nuclease resistance and for ease of synthesis of oligonucleotides of variable length. This ease of variability in linker length via designing appropriate spacers is important for optimizing the binding of a binding agent as disclosed herein to its antigen or antigens.

In one preferred embodiment the linker L (= a-S-b) is enantiomeric L-DNA or L-RNA. In one preferred embodiment linker a-S-b is enantiomeric L-DNA. In one preferred embodiment a, a', b and b' as well as the spacer S are enantiomeric L-DNA or L-RNA. In one preferred embodiment a, a', b and b' as well as the spacer S are enantiomeric L-DNA.

In one embodiment the spacer S is an oligonucleotide and is synthesized in two portions comprising ends hybridizable with each other. In this case the spacer S can be simply constructed by hybridization of these hybridizable ends with one another. The resulting spacer construct comprises an oligonucleotide duplex portion. As obvious, in case the spacer is construed that way, the sequence of the hybridizable oligonucleotide entity forming said duplex is chosen in such a manner that no hybridization or interference with the binding pairs a:a' and b:b' can occur.

As indicated above the bivalent binding agent according to the present invention in one embodiment binds to a polypeptide homodimer. In case the two target polypeptides form a homodimer it is preferable to use the same monovalent binder twice. In one embodiment the present invention relates to a bivalent binding agent capable of binding to a protein homodimer according to Formula I, wherein S is a polynucleotide spacer wherein the monovalent binder A binds to a first target polypeptide, wherein the second monovalent binder - usually B - is also A and binds to a second target polypeptide, wherein a':a and b:b' both represent a polynucleotide binding pair. As obvious to the skilled artisan the epitope bound by the monovalent binder A must be only present once on the target polypeptide or the epitopes recognized by two different monovalent binders A and B, respectively, must be present only once and must be overlapping to avoid binding of the two monovalent binders on a single monomeric target polypeptide. In one embodiment the bivalent binding agent binds to a homodimer and both monovalent binders bind to an overlapping epitope. In one embodiment the bivalent binding agent binds to a homodimer and both monovalent binders bind to the same epitope.

In case the bivalent binding agent according to the present invention binds to a polypeptide homodimer, a simplified way can also be used to construct such bivalent binding agent. In this embodiment the monovalent binder A can be coupled to only a single species of a hybridizable polynucleotide (a') and the linker can be construed to provide for two ends, each hybridizable to the construct A-a'. In this embodiment the linker may be of the form a-S-a. In the example given herein such special linker has been termed adaptor.

As mentioned, in one embodiment the spacer S is an oligonucleotide and is synthesized in two portions comprising ends hybridizable with each other. In this case the spacer S can be simply constructed by hybridization these hybridizable ends with one another and the resulting spacer construct comprises an oligonucleotide duplex portion.

As indicated above the bivalent binding agent according to the present invention in one embodiment binds to a polypeptide heterodimer. In case the two target polypeptides form a heterodimer it is preferable to use two different binding pairs a':a and b:b', respectively. In one embodiment the present invention relates to a bivalent binding agent according to Formula I, herein S is a polynucleotide spacer wherein the monovalent binder A binds to a first target polypeptide, wherein the second monovalent binder B binds to a second target polypeptide, wherein A and B specifically bind to the first an the second target polypeptide, respectively, wherein a':a and b:b' both represent a polynucleotide binding pair, wherein a':a and b:b' do not bind to or interfere with one another.

As already described above the monovalent specific binders A and B of Formula I may be nucleic acids. In one embodiment of the present invention a', a, b, b', A, B and S all are oligonucleotide sequences. In this embodiment the sub-units A-a', a-S-b and b'-B of Formula I can easily and independently be synthesized according to standard procedures and combined by hybridization according to convenient standard procedures.

As discussed in detail above, the coupling can be either co-valent or it can be via specific binding pairs.

As the skilled artisan will readily appreciate, the bivalent binding agent according to the present invention may be further modified to carry one or more functional moieties. Such functional moiety X is preferably selected from the group consisting of a binding group, a labeling group, an effector group and a reactive group.

If more than one functional moiety X is present, each such functional moiety can in each case be independently a binding group, a labeling group, an effector group or a reactive group.

In one embodiment the functional moiety X is preferably selected from the group consisting of a binding group, a labeling group and an effector group.

In one embodiment the group X is a binding group. As obvious to a person skilled in the art, the binding group X will be selected to have no interference with the pairs a':a and b:b'.

Examples of binding groups are the partners of a bioaffine binding pair which can specifically interact with the other partner of the bioaffine binding pair. Suitable bioaffine binding pairs are hapten or antigen and antibody; biotin or biotin analogues such as aminobiotin, iminobiotin or desthiobiotin and avidin or streptavidin; sugar and lectin, oligonucleotide and complementary oligonucleotide, receptor and ligand, e.g., steroid hormone receptor and steroid hormone. In one embodiment X is a binding group and is covalently bound to at least one of a', a, b, b' or S of the compound of Formula I. Preferably the smaller partner of a bioaffine binding pair, e.g. biotin or an analogue thereto, a receptor ligand, a hapten or an oligonucleotide is covalently bound to at lest one of a', a, S, b or b' as defined above.

In one embodiment functional moiety X is a binding group selected from hapten; biotin or biotin analogues such as aminobiotin, iminobiotin or desthiobiotin; oligonucleotide and steroid hormone.

In one embodiment the functional moiety X is a reactive group. The reactive group can be selected from any known reactive group, like Amino, Sulfhydryl, Carboxylate, Hydroxyl, Azido, Alkinyl or Alkenyl. In one embodiment the reavtive group is selected from Maleinimido, Succinimidyl, Dithiopyridyl, Nitrophenylester, Hexafluorophenylester.

In one embodiment the functional moiety X is a labeling group. The labeling group can be selected from any known detectable group. The skilled artisan will choose the number of labels as approporiate for best sensitivity with least quenching.

The labeling group can be selected from any known detectable group. In one embodiment the labeling group is selected from dyes like luminescent labeling groups such as chemiluminescent groups e.g. acridinium esters or dioxetanes or fluorescent dyes e.g. fluorescein, coumarin, rhodamine, oxazine, resorufin, cyanine and derivatives thereof, luminescent metal complexes such as ruthenium or europium complexes, enzymes as used for CEDIA (Cloned Enzyme Donor Immunoassay, e.g. EP 0 061 888), microparticles or nanoparticles e.g. latex particles or metal sols, and radioisotopes.

In one embodiment the labeling group is a luminescent metal complex and the compound has a structure of the general formula (II):

[M(L₁L₂L₃)]ₙ-Y-XₘA (II)

in which M is a divalent or trivalent metal cation selected from rare earth or transition metal ions, L₁, L₂ and L₃ are the same or different and denote ligands with at least two nitrogen-containing heterocycles in which L₁, L₂ and L₃ are bound to the metal cation via nitrogen atoms, X is a reactive functional group which is covalently bound to at least one of the ligands L₁, L₂ and L₃ via a linker Y, n is an integer from 1 to 10, preferably 1 to 4, m is 1 or 2 and preferably 1 and A denotes the counter ion which may be required to equalize the charge.

The metal complex is preferably a luminescent metal complex i.e. a metal complex which undergoes a detectable luminescence reaction after appropriate excitation. The luminescence reaction can for example be detected by fluorescence or by electrochemiluminescence measurement. The metal cation in this complex is for example a transition metal or a rare earth metal. The metal is preferably ruthenium, osmium, rhenium, iridium, rhodium, platinum, indium, palladium, molybdenum, technetium, copper, chromium or tungsten. Ruthenium, iridium, rhenium, chromium and osmium are particularly preferred. Ruthenium is most preferred.

The ligands L₁, L₂ and L₃ are ligands with at least two nitrogen-containing heterocycles. Aromatic heterocycles such as bipyridyl, bipyrazyl, terpyridyl and phenanthrolyl are preferred. The ligands L₁, L₂ and L₃ are particularly preferably selected from bipyridine and phenanthroline ring systems.

The complex can additionally contain one or several counter ions A to equalize the charge. Examples of suitable negatively charged counter ions are halogenides, OH⁻, carbonate, alkylcarboxylate, e.g. trifluoroacetate, sulphate, hexafluorophosphate and tetrafluoroborate groups. Hexafluorophosphate, trifluoroacetate and tetrafluoroborate groups are particularly preferred. Examples of suitable positively charged counter ions are monovalent cations such as alkaline metal and ammonium ions.

In a further preferred embodiment the functional moiety X is an effector group. A preferred effector group is a therapeutically active substance.

Therapeutically active substances have different ways in which they are effective, e.g. in inhibiting cancer. They can damage the DNA template by alkylation, by cross-linking, or by double-strand cleavage of DNA. Other therapeutically active substances can block RNA synthesis by intercalation. Some agents are spindle poisons, such as vinca alkaloids, or anti-metabolites that inhibit enzyme activity, or hormonal and anti-hormonal agents. The effector group X may be selected from alkylating agents, antimetabolites, antitumor antibiotics, vinca alkaloids, epipodophyllotoxins, nitrosoureas, hormonal and antihormonal agents, and toxins.

Currently more preferred alkylating agents may be exemplified by cyclophosphamide, chlorambucil, busulfan, Melphalan, Thiotepa, ifosphamide, Nitrogen mustard.

Currently more preferred antimetabolites may be exemplified by methotrexate, 5-Fluorouracil, cytosine arabinoside, 6-thioguanine, 6-mercaptopurin.

Currently more preferred antitumor antibiotics may be exemplified by doxorubicin, daunorubicin, idorubicin, nimitoxantron, dactinomycin, bleomycin, mitomycin, and plicamycin.

Currently more preferred spindle poisons may be exemplified by maytansine and maytansinoids, vinca alkaloids and epipodophyllotoxins may be exemplified by vincristin, vinblastin, vindestin, Etoposide, Teniposide.

Currently more preferred nitrosoureas may be exemplified by carmustin, lomustin, semustin, streptozocin.

Currently more preferred hormonal and antihormonal agents may be exemplified by adrenocorticorticoids, estrogens, antiestrogens, progestins, aromatase inhibitors, androgens, antiandrogens.

Additional preferred random synthetic agents may be exemplified by dacarbazin, hexamethylmelamine, hydroxyurea, mitotane, procarbazide, cisplastin, carboplatin.

A functional moiety X is bound either covalently or via an additional binding pair, e.g., to at least one of (a'), (a), (b), (b') or S. The functional moiety X can occur once or several (n) times. (n) is an integer and 1 or more than one. Preferably (n) is between 1 and 100. Also preferred (n) is 1 - 50. In certain embodiments n is 1 to 10, or 1 to 5. In further embodiments n is 1 or 2.

For covalent binding of the functional moiety X to at least one of a', a, b, b' or S any appropriate coupling chemistry can be used. The skilled artisan can easily select such coupling chemistry from standard protocols. It is also possible to incorporate a functional moiety by use of appropriate building blocks when synthesizing a', a, b, b' or S.

In one preferred embodiment functional moiety X is bound to a, b, or S of the binding agent as defined by Formula I.

In one preferred embodiment functional moiety X is bound to the spacer S of the binding agent as defined by Formula I.

In one preferred embodiment functional moiety X is covalently bound to a, b, or S of the binding agent as defined by Formula I.

If a functional moiety X is located within the a hybridizing oligonucleotide representing a, a', b or b', respectively, preferably such functional moiety is bound to a modified nucleotide or is attached to the internucleosidic P atom (WO 2007/059816). Modified nucleotides which do not interfere with the hybridization of oligonucleotides are incorporated into those oligonucleotides. Such modified nucleotides preferably are C5 substituted pyrimidines or C7 substituted 7deaza purines.

Oligonucleotides can be modified internally or at the 5' or 3' terminus with non-nucleotidic entities which are used for the introduction of functional moiety. Preferably such non-nucleotidic entities are located within the spacer S, i.e. between the two binding pair members a and b.

Many different non-nucleotidic modifier building blocks for construction of a spacer are known in literature and a great variety is commercially available. For the introduction of a functional moiety either non-nucleosidic bifunctional modifier building blocks or non-nucleosidic trifunctional modified building blocks are either used as CPG for terminal labeling or as phosphroamidite for internal labeling (see: Wojczewski, C. et al., Synlett 10 (1999) 1667-1678).

### Bifunctional modifier building blocks

Bifunctional modifier building blocks connect a functional moiety or a - if necessary - a protected functional moiety to a phosphoramidite group for attaching the building block at the 5' end (regular synthesis) or at the 3'end (inverted synthesis) to the terminal hydroxyl group of a growing oligonucleotide chain.

Bifunctional modifier building blocks are preferably non-nucleosidic compounds. For example, such modified building blocks are C2 - C18 alkyl, alkenyl, alkynyl carbon chains, whereas said alkyl, alkenyl, alkynyl chains may be interrupted by additional ethyleneoxy and/or amide moieties in order to increase hydrophilicity of the spacer and thereby of the whole linker structure. Cyclic moieties like C5-C6-cycloalkyl, C4N, C5N, C4O,C5O- heterocycloalkyl, phenyl which are optionally substituted with one or two C1-C6 alkyl groups can also be used as non-nucleosidic bifunctional modified building blocks. Preferred modified bifunctional building blocks comprise C3-C6 alkyl moieties and tri- to hexa- ethyleneglycol chains. Non-limiting, yet preferred examples of bifunctional modifier building blocks are given in Table III below.

**Table III:**

| **Bifunctional non-nucleosidic modifier building block** | **Introduction of** | **Reference** |
|---|---|---|
| 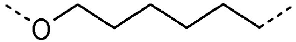 | 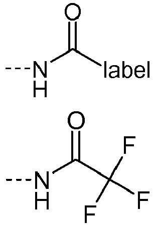 | Pon, R.T., Tetrahedron Letters 32 (1991) 1715-1718 |
| | | Theisen, P. et al., Nucleic Acids Symposium Series (1992), 27 (Nineteenth Symposium on Nucleic Acids Chemistry, 1992), 99-100 |
| | | EP 0 292 128 |
| 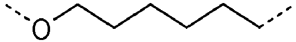 | 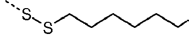 | EP 0 523 978 |
| 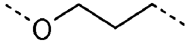 | 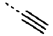 | Meyer, A. et al., Journal of Organic Chemistry 75 (2010) 3927-3930 |
| 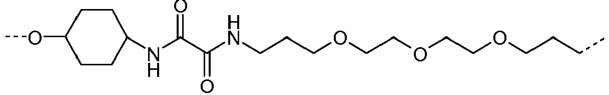 | 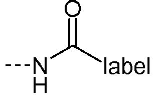 | Morocho, A.M. et al., Nucleosides, Nucleotides & Nucleic Acids 22 (2003) 1439-1441 |
| 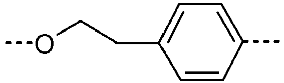 | 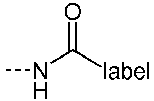 | Cocuzza, A.J., Tetrahedron Letters 30 (1989) 6287-6290 |

### Trifunctional modifier building blocks

Trifunctional building blocks connect (i) a functional moiety or a - if necessary - a protected functional moiety, (ii) a phosphoramidite group for coupling the reporter or the functional moiety or a - if necessary - a protected functional moiety, during the oligonucleotide synthesis to a hydroxyl group of the growing oligonucleotide chain and (iii) a hydroxyl group which is protected with an acid labile protecting group preferably with a dimethoxytrityl protecting group. After removal of this acid labile protecting group a hydroxyl group is liberated which can react with further phosphoramidites. Therefore trifunctional building blocks allow for positioning of a functional moiety to any location within an oligonucleotide. Trifunctional building blocks are also a prerequisite for synthesis using solid supports, e.g. controlled pore glass (CPG), which are used for 3' terminal labeling of oligonucleotides. In this case, the trifunctional building block is connected to a functional moiety or a - if necessary - a protected functional moiety via an C2 - C18 alkyl, alkenyl, alkinyl carbon chains, whereas said alkyl, alkenyl, alkyinyl chains may be interrupted by additional ethyleneoxy and/or amide moieties in order to increase hydrophilicity of the spacer and thereby of the whole linker structure and comprises a hydroxyl group which is attached via a cleavable spacer to a solid phase and a hydroxyl group which is protected with an acid labile protecting group. After removal of this protecting group a hydroxyl group is liberated which could then react with a phosphoramidite.

Trifunctional building blocks may be non-nucleosidic or nucleosidic.

Non-nucleosidic trifunctional building blocks are C2 - C18 alkyl, alkenyl, alkynyl carbon chains, whereas said alkyl, alkenyl, alkynyl are optionally interrupted by additional ethyleneoxy and/or amide moieties in order to increase hydrophilicity of the spacer and thereby of the whole linker structure. Other trifunctional building blocks are cyclic groups like C5-C6-cycloalkyl, C4N, C5N, C4O, C5O heterocycloalkyl, phenyl which are optionally substituted with one ore two C1-C6 alkyl groups. Cyclic and acyclic groups may be substituted with one -(C1-C18)alkyl-O-PG group, whereas said C1-C18 alkyl comprises (Ethyleneoxy)n, (Amide)m moieties with n and m independently from each other = 0 - 6 and PG is an acid labile protecting group. Preferred trifunctional building blocks are C3-C6 alkyl, cycloalkyl, C5O heterocycloalkyl moieties optionally comprising one amide bond and substituted with a C1 - C6 alkyl O-PG group, wherein PG is an acid labile protecting group, preferably monomethoxytrityl, dimethoxytrityl, pixyl, xanthyl most preferred dimethoxytrityl.

Non-limiting, yet preferred examples for non-nucleosidic trifunctional building blocks are e.g. summarized in Table IV.

**Table IV: Examples for non-nucleosidic trifunctional modifier building blocks**

| **Trifunctional** | **Introduction of** | **Reference** |
|---|---|---|
| 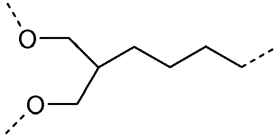 | 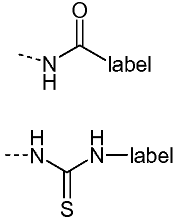 | Nelson, P.S. et al., Nucleic Acids Research 20 (1992) 6253-6259 |
| | | EP 0 313 219; US 5,585,481; US 5,451,463; EP 0 786 468; WO 92/11388; WO 89/02439 |
| 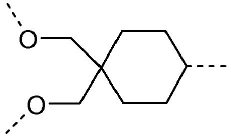 | 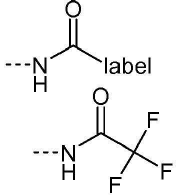 | Su, S.-H. et al., Bioorganic & Medicinal Chemistry Letters 7 (1997) 1639-1644 |
| | | WO 97/43451 |
| 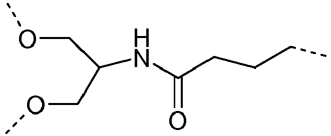 | 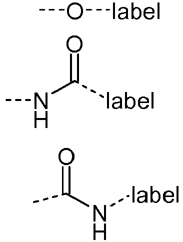 | Putnam, W.C. et al., Nucleosides, Nucleotides & Nucleic Acids 24 (2005) 1309-1323 |
| | | US 2005/214833; EP 1 186 613 |
| 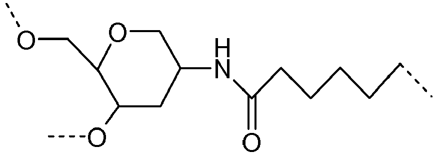 | 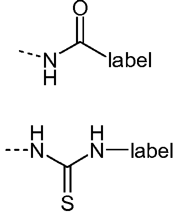 | EP 1 431 298 |
| 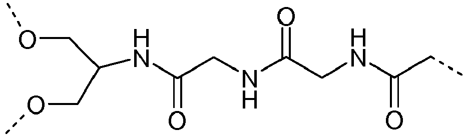 | 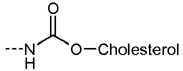 | WO 94/04550 |
| | | Huynh, V. et al., Nucleic Acids Symposium Series (1993), 29 (Second International Symposium on Nucleic Acids Chemistry), 19-20 |
| 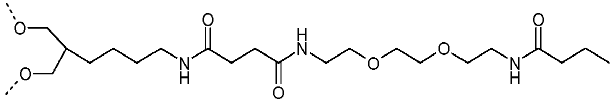 | 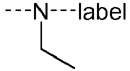 | WO 2003/019145 |
| 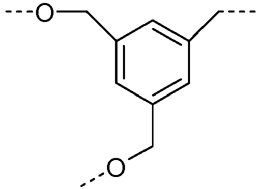 | 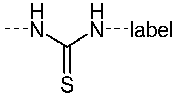 | Behrens, C. and Dahl, O., Nucleosides & Nucleotides 18 (1999) 291-305 |
| | | WO 97/05156 |
| 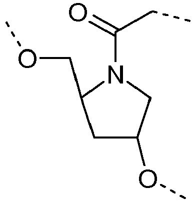 | 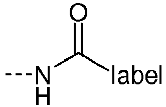 | Prokhorenko, I.A. et al., Bioorganic & Medicinal Chemistry Letters 5 (1995) 2081-2084 |
| | | WO 2003/104249 |
| 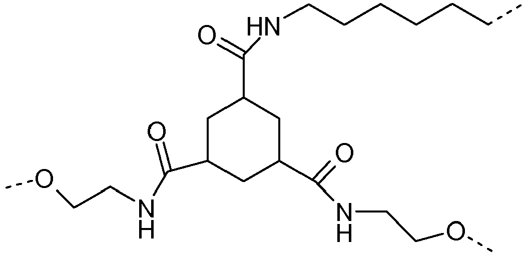 | 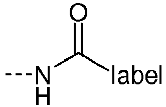 | US 5,849,879 |

### Nucleosidic modifier building blocks:

Nucleosidic modifier building blocks are used for internal labeling whenever it is necessary not to influence the oligonucleotide hybridization properties compared to a non modified oligonucleotide. Therefore nucleosidic building blocks comprise a base or a base analog which is still capable of hybridizing with a complementary base. The general formula of a labeling compound for labeling a nucleic acid sequence of one or more of a, a', b, b' or S comprised in a binding agent according to Formula I of the present invention is given in Formula II. 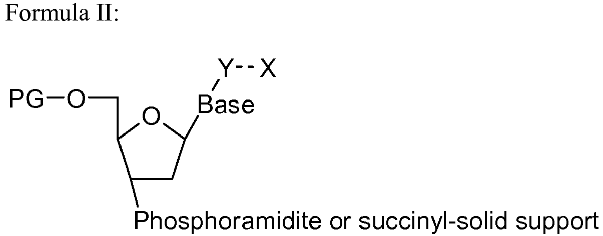 wherein PG is an acid labile protecting group, preferably monomethoxytrityl, dimethoxytrityl, pixyl, xanthyl most preferred dimethoxytrityl, wherein Y is C2 - C18 alkyl, alkenyl alkinyl, wherein said alkyl, alkenyl, alkinyl may comprise ethyleneoxy and/or amide moieties, wherein Y preferably is C4 - C18 alkyl, alkenyl or alkinyl and contains one amide moiety and wherein X is a functional moiety to which a label can be bound.

Specific positions of the base may be chosen for such substitution to minimize the influence on hybridization properties. Therefore the following positions are preferred for substitution: a) with natural bases: Uracil substituted at C5; Cytosine substituted at C5 or at N4; Adenine substituted at C8 or at N6 and Guanine substituted at C8 or at N2 and b) with base analogs: 7 deaza A and 7 deaza G substituted at C7; 7 deaza 8 Aza A and 7 deaza 8 Aza G substituted at C7; 7 deaza Aza 2 amino A substituted at C7; Pseudouridine substituted at N1 and Formycin substituted at N2.

Non-limiting, yet preferred examples for nucleosidic trifunctional building blocks are given in Table V.

**Table V:**

| **Trifunctional nucleosidic** | **A** | **Reference** |
|---|---|---|
| 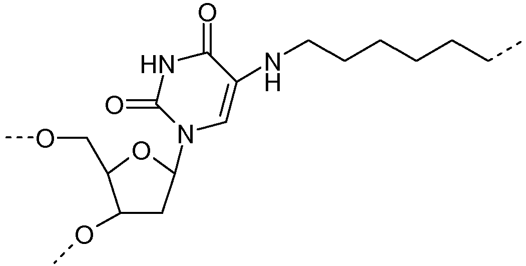 | 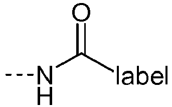 | Roget, A. et al., Nucleic Acids Research 17 (1989) 7643-7651 |
| | | WO 89/12642; WO 90/08156; WO 93/05060 |
| 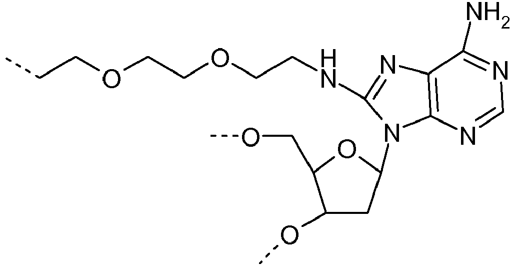 | 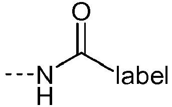 | Silva, J.A. et al., Biotecnologia Aplicada 15 (1998) 154-158 |
| 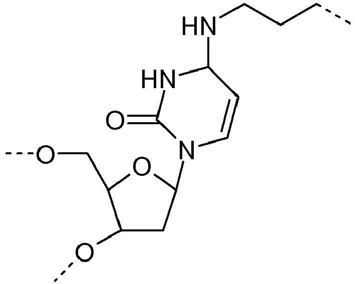 | 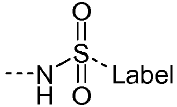 | US 6,531,581 |
| | | EP 0 423 839 |
| 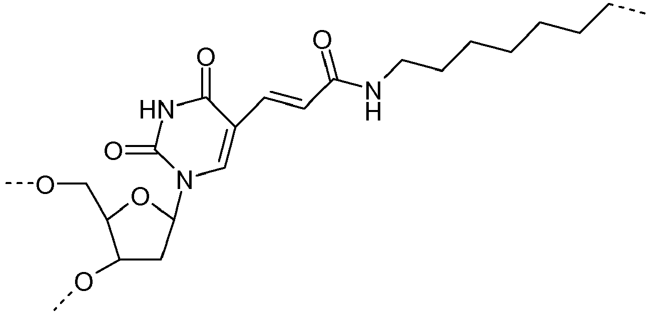 | 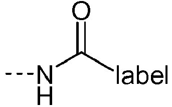 | US 4,948,882; US 5,541,313; US 5,817,786 |
| 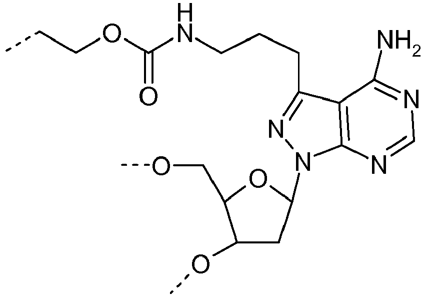 | 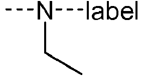 | WO 2001/042505 |
| 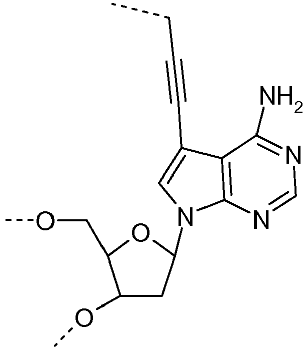 | 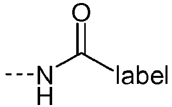 | McKeen, C.M. et al., Organic & Biomolecular Chemistry 1 (2003) 2267-2275 |
| 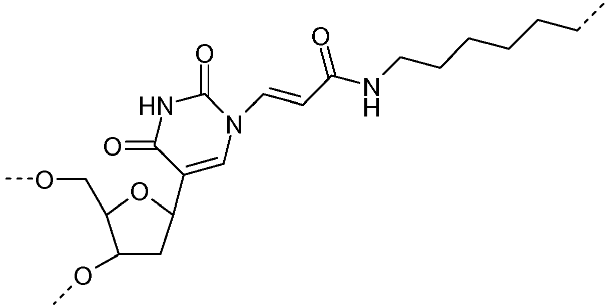 | 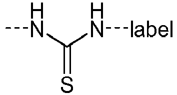 | Ramzaeva, N. et al., Helvetica Chimica Acta 83 (2000)1108-1126 |

In Tables III, IV and V, one of the terminal oxygen atom of a bifunctional moiety or one of the terminal oxygen atoms of a trifunctional moiety is part of a phosphoramidite that is not shown in full detail but obvious to the skilled artisan. The second terminal oxygen atom of trifunctional building block is protected with an acid labile protecting group PG, as defined for Formula II above.

Post-synthetic modification is another strategy for introducing a covalently bound functional moiety into a linker or a spacer molecule. In this approach an amino group is introduced by using bifunctional or trifunctional building block during solid phase synthesis. After cleavage from the support and purification of the amino modified oligonucleotide is reacted with an activated ester of a functional moiety or with a bifunctional reagent wherein one functional group is an active ester. Preferred active esters are NHS ester or pentafluor phenyl esters.

Post-synthetic modification is especially useful for introducing a functional moiety which is not stable during solid phase synthesis and deprotection. Examples are modification with triphenylphosphincarboxymethyl ester for Staudinger ligation (Wang, C.C. et al., Bioconjugate Chemistry 14 (2003) 697-701), modification with digoxigenin or for introducing a maleinimido group using commercial available sulfo SMCC.

The functional moiety X in one embodiment is bound to at least one of a', a, b, b' or S via an additional binding pair.

The additional binding pair to which a functional moiety X can be bound is preferably a leucine zipper domain or a hybridizing nucleic acid. In case the functional moiety X is bound to at least one of a', a, b, b' or S via an additional binding pair member, the binding pair member to which X is bound and the binding pairs a':a and b:b', respectively, all are selected to have different specificity. The binding pairs a:a', b:b' and the binding pair to which X is bound each bind to (e.g. hybridize with) their respective partner without interfering with the binding of any of the other binding pairs.

### Covalent coupling of a member of a binding pair to a monovalent binder

Depending on the biochemical nature of the binder different conjugation strategies are at hand.

In case the binder is a naturally occurring protein or a recombinat polypeptide of between 50 to 500 amino acids, there are standard procedures in text books describing the chemistry for synthesis of protein conjugates, which can be easily followed by the skilled artisan (Hackenberger, C.P. and Schwarzer, D., Angew. Chem., Int. Ed., 47 (2008) 10030-10074).

In one embodiment the reaction of a maleinimido moiety with a cystein residue within the protein is used. This is a preferred coupling chemistry in case e.g. an Fab or Fab'-fragment of an antibody is used a monovalent binder. Alternatively in one embodiment coupling of a member of a binding pair (a' or b', respectively, of Formula I) to the C-terminal end of the binder polypeptide is performed. C-terminal modification of a protein, e.g. of an Fab-fragment can e.g. be performed as described by Sunbul, M. et al., Organic & Biomolecular Chemistry 7 (2009) 3361-3371).

In general site specific reaction and covalent coupling of a binding pair member to a monovalent polypeptidic binder is based on transforming a natural amino acid into an amino acid with a reactivity which is orthogonal to the reactivity of the other functional groups present in a protein. For example, a specific cystein within a rare sequence context can be enzymatically converted in an aldehyde (see Formylglycine aldehyde tag -protein engineering through a novel post-translational modification (Frese, M.-A. et al., ChemBioChem 10 (2009) 425-427). It is also possible to obtain a desired amino acid modification by utilizing the specific enzymatic reactivity of certain enzymes with a natural amino acid in a given sequence context (see e.g.: Taki, M. et al., Protein Engineering, Design & Selection 17 (2004) 119-126; Gautier, A. et al., Chemistry & Biology 15 (2008) 128-136; Protease-catalyzed formation of C-N bonds is used by Bordusa, F., Highlights in Bioorganic Chemistry (2004) 389-403; and Sortase-mediated protein ligation is used by Mao, H. et al., in J. Am Chem Soc. 126 (2004) 2670-2671 and reviewed by Proft, T., in Biotechnol. Lett 32 (2010) 1-10).

Site specific reaction and covalent coupling of a binding pair member to a monovalent polypeptidic binder can also be achieved by the selective reaction of terminal amino acids with appropriate modifying reagents.

The reactivity of an N-terminal cystein with benzonitrils (Ren, Hongjun et al., Angewandte Chemie, International Edition 48 (2009) 9658-9662) can be used to achieve a site-specific covalent coupling.

Native chemical ligation can also rely on C-terminal cystein residues (Taylor, E. et al., Nucleic Acids and Molecular Biology 22 (2009) 65-96).

EP 1 074 563 describes a conjugation method which is based on the faster reaction of a cystein within a stretch of negatively charged amino acids with a cystein located in a stretch of positively charged amino acids.

The monovalent binder may also be a synthetic peptide or peptide mimic. In case a polypeptide is chemically synthesized, amino acids with orthogonal chemical reactivity can be incorporated during such synthesis (de Graaf, A.J. et al., Bioconjugate Chemistry 20 (2009) 1281-1295). Since a great variety of orthogonal functional groups is at stake and can be introduced into a synthetic peptide, conjugation of such peptide to a linker is standard chemistry.

In order to obtain a mono-labeled protein the conjugate with 1:1 stoichiometry may be separated by chromatography from other conjugation products. This procedure is facilated by using a dye labeled binding pair member and a charged spacer. By using this kind of labeled and highly negatively charged binding pair member, mono conjugated proteins are easily separated from non labeled protein and proteins which carry more than one linker, since the difference in charge and molecular weight can be used for separation. The fluorescent dye is valuable for purifying the bivalent bindig agent from un-bound components, like a labeled monovalent binder.

Therefore in one embodiment it is preferred to use a binding pair member (a' and/or b', respectively of Formula I) which is labeled with a fluroescent dye (e.g. synthesized using a bifunctional or trifunctional modifier building block in combination with bifunctional spacer building blocks during synthesis) for forming the bivalent binding agent of the present invention. In one preferred embodiment the spacer S as well as the sequences a, a', b and b' are DNA and at least one of a' or b', respectively, is labeled with a fluorescent dye. In one preferred embodiment the spacer S as well as the sequences a, a', b and b' are DNA and both a' and b', respectively, are labeled each with a different fluorescent dye.

In one embodiment a method of producing a bivalent binding agent that specifically binds a polypeptide dimer is disclosed. The method comprises the steps of (a) selecting a first monovalent binder that binds to a first target polypeptide with a Kdiss of between 5x10⁻³/sec to 10⁻⁴/sec, (b) selecting a second monovalent binder that binds to a second target polypeptide with a Kdiss of between 5x10⁻³/sec to 10⁻⁴/sec, c) coupling both monovalent binders by a linker, and d) selecting a bivalent binding agent having a Kdiss-value of 3x10⁻⁵/sec or less.

Using a method as disclosed in the present invention it is now rather easy to produce various bivalent binding agents each comprising a linker of different length and to select those bivalent binding agents having the desired binding properties, i.e. a Kdiss-value of 3x10⁻⁵/sec or less. Selection of a bivalent binding agent with the desired Kdiss in one embodiment is performed by Biacore™-analysis as disclosed in Example 2.8.

In one embodiment the present invention relates to a method of forming a bivalent binding agent according to the present invention, wherein a first monovalent binder that binds to a first target polypeptide with a Kdiss of between 10⁻³/sec to 10⁻⁴/sec and is coupled to a member of a first binding pair, a second monovalent binder that binds to a second target polypeptide with a Kdiss of 10⁻³/sec to 10⁻⁴/sec and is coupled to a member of a second binding pair, wherein the first and the second binding pair do not interfere with each other and a linker comprising a spacer and the complementary binding pair members to the first and the second binding pair member, respectively are co-incubated, whereby a bivalent binding agent having a Kdiss-value of 3x10⁻⁵/sec or less is formed.

As the skilled artisan will appreciate the Kdiss is a temperature-dependent value. Logically, the Kdiss-values of both the monovalent binders as well as of the bivalent binding agent according to the present invention are determined at the same temperature. As will be appreciated preferably a Kdiss-value is determined at the same temperature at which the bivalent binding agent shall be used, e.g., an assay shall be performed. In one embodiment the Kdiss-values are established at room temperature, i.e. at 20°C, 21°C, 22°C, 23°C, 24°C or 25°C, respectively. In one embodiment the Kdiss-values are established at 4 or 8°C, respectively. In one embodiment the Kdiss-values are established at 25°C. In one embodiment the Kdiss-values are established at 37°C. In one embodiment the Kdiss-values are established at 40°C. In one preferred embodiment Kdiss determinations, i.e. those for each monovalent binder and the Kdiss determination for the dual binder are made at 37°C.

In one embodiment the above method further comprises the step of isolating the bivalent binding agent.

The preferred stoichiometry for forming or assembling the bivalent binding agent according to the present invention is 1:1:1.

In one preferred embodiment the method of producing a bivalent binding reagent according to the present invention makes use of an L-DNA-linker. In one preferred embodiment the method of producing a bivalent binding reagent according to the present invention makes use of two specific binding pairs consisting of DNA, preferably L-DNA, and of an L-DNA-linker.

The formation and stoichiometry of the formed bivalent binding agent can be analyzed by Size Exclusion Chromatography according to state of the art procedures. If desired, the formed complexes can also be analyzed by SDS-PAGE.

The bivalent binding agent disclosed in this invention, if used in an immunohistochemical staining procedure only significantly binds and is not washed off during the various incubation steps of such procedure if it has a Kdiss of 3x10⁻⁵/sec or better. This Kdiss can only be achieved, if both monovalent binder bind to their corresponding binding site. In case only one epitope on one of the two target polypeptides is present, i.e. in case the polypeptides are monomeric and not dimeric no significant staining will be found. Thus, and this is of great advantage, immunohistochemical staining will be only observed if a polypeptide dimer is present in the sample.

In a preferred embodiment the present invention therefore relates to a histological staining method the method comprising the steps of (a) providing a cell or tissue sample, (b) incubating said sample with a bivalent binding agent consisting of two monovalent binders that are linked to each other via a linker, wherein the first monovalent binders binds to a first polypeptide the second monovalent binder binds to a second polypeptide, each monovalent binder has a Kdiss in the range of 5x10⁻³/sec to 10⁻⁴/sec and wherein the bivalent binding agent has a Kdiss of 3x10⁻⁵/sec or less, and (c) detecting the bivalent binding agent, thereby staining said sample for a polypeptide dimer of interest.

Use of a bivalent binding agent according to the present invention in the staining of a cell or tissue sample by an immunohistochemical method represents a further preferred embodiment.

In more general terms the present invention relates to a bivalent binding agent consisting of two monovalent binders that are linked to each other via a linker, which binding agent binds a target polypeptide dimer with a Kdiss meeting the requirements of an (automated) assay system or better, wherein (a) the first monovalent binder that binds to a first target polypeptide with a Kdiss of at least 10-fold above (worse than) the requirements of the (automated) assays system, (b) the second monovalent binder binds to a second target polypeptide with a Kdiss of at least 10-fold above the requirements of the (automated) assays system, and (c) wherein the product of the Kdiss-values of the two monovalent binders (a) and (b) has at least the Kdiss required by the (automated) system or less.

In general terms a method for obtaining a bivalent binding agent is described that specifically binds a polypeptide dimer, comprising two target polypeptides, with a Kdiss at least meeting the minimal assay requirements of an (automated) assay system or better, the method comprising the steps of (a) selecting a first monovalent binder that binds to a first target polypeptide with a Kdiss of at least 10-fold above the minimal assay requirements of the (automated) assays system, (b) selecting a second monovalent binder that binds to a second target polypeptide with a Kdiss of at least 10-fold above the minimal assay requirements of the (automated) assays system, wherein the product of the Kdiss-values of the two monovalent binders in steps (a) and (b) is at least the Kdiss required by the (automated) system or less and (c) coupling both monovalent binders by a linker.

In one embodiment the automated system is the BenchMark® analyzer as distributed by Ventana Medical Systems Inc., Tucson.

The following examples, sequence listing and figures are provided to aid the understanding of the present invention.

### Description of the Sequence Listing

### 1. Antibody fragments

**SEQ ID NO:1** V_{H} (mAb 1.4.168):
**SEQ ID NO:2** V_{L} (mAb 1.4.168):
**SEQ ID NO:3** V_{H} (mAb 8.1.2):
**SEQ ID NO:4** V_{L} (mAb 8.1.2):

### 2. Sequences of ssDNA

a) 19mer ssDNA (covalently bound with 3' end to Fab' of anti-TroponinT MAB b or Fab' 8.1.2 to phosphorylated IGF-1R, respectively)
   5'-A GTC TAT TAA TGC TTC TGC-3' (SEQ ID NO:5)
b) 17mer ssDNA (covalently bound with 5' end to Fab' of anti-TroponinT MAB a or Fab' 1.4.168 to IGF-1R, respectively)
   5'-AGT TCT ATC GTC GTC CA-3'(SEQ ID NO:6)
c) complementary 19mer ssDNA (used as part of a linker)
   5'-G CAG AAG CAT TAA TAG ACT-3' (SEQ ID NO:7)
d) complementary 17mer ssDNA (used as part of a linker)
   5'- TGG ACG ACG ATA GAA CT-3' (SEQ ID NO:8)

### 3. Sequences of Troponin T epitopes:

SEQ ID NO:9 = ERAEQQRIRAEREKEUUSLKDRIEKRRRAERAEamide, wherein U represents β-Alanin. (The epitope "A" for antibody anti-Troponin antibody a.)
SEQ ID NO:10 = SLKDRIERRRAERAEOOERAEQQRIRAEREKEamide, wherein O represents Amino-trioxa-octanoic-acid. (The epitope "B" for antibody anti-Troponin antibody b.)

### 4. Sequences of IGF-1R/IR epitopes:

SEQ ID NO:11 = FDERQPYAHMNGGRKNERALPLPQSST; IGF-1R (1340-1366)
SEQ ID NO:12 = YEEHIPYTHMNGGKKNGRILTLPRSNPS; hIR(1355-1382)

### 5. Protein linker and tag-sequences:

SEQ ID NO:13 = GGGGS motif (e.g. as prt of a polypeptide linker)
SEQ ID NO:14 = YPYDVPDYA (HA-Tag)
SEQ ID NO:15 = GLNDIFEAQKIEWHE (Avi-Tag)

### 6. Sequences used for compiling the bivalent binding agent to a polypeptide homodimer

SEQ ID NO:16 = ACC TGC TGC TAT CTT GA; this oligonucleotide was FAM-labeled at the 5' end and for coupling modified to carry a maleimido at the 3' end.
SEQ ID NO:17 = G CAG AAG CAT TAA TAG AC- T(Biotin)-TGG ACG ACG ATA GAA CT; the linker oligonucleotide
SEQ ID NO:18 = GTC TAT TAA TGC TTC TGC - C3 - TGG ACG ACG ATA GAA CT; the adaptor oligonucleotide

### 7. Sequences used in a dual-binder to HER2/HER3 heterodimers for use in immunohistochemistry

SEQ ID NO:19 = EFEVQLQESGGGLVQPKGSLQLSCAASGFTFNTYAMHWVRQAPGKGLEW VARIRTESSDYATDYADSVKDRFIISRDDSQNMLYLQMNNLKSEDTAIYYC VRSSGFDYWGQGTTLTVSSS; 7.2.32 variable region heavy chain (HC)
SEQ ID NO:20 = DIQMTQSPSLPVSLGDQASISCRSSQSLVHDNGNTYLHWFLQKPGQSPKLLI YKVSNRFSGVPDRFGGSGSGTDFTLKISGVEAEDLGVYFCSQGTHVPTFGG GTKLEIK; 7.2.32 variable region light chain (LC)
SEQ ID NO:21 = EVQLQQSGPVLVKPGASVKMSCRPSGYTFTDYNMHWVKQSHEKSLEWIG YINPKNGIISYNQKFKDKATLTVNKSSRTAYMELRSLTSEDSAVYFCAGDY WGQGTSVTVSS; 4.1.43 variable region heavy chain (HC):
SEQ ID NO:22 = DVVMTQTPLSLPVSLGDQASISCRSSQSLVHSNGNTYLHWYLQKPGQSPKL LISKVSNRFSGVPDRFSGSGSGTDFTLKISRVEAEDLGVYFCSQSIHLPWTFG GGTKLEIK; 4.1.43 variable region light chain (LC):
SEQ ID NO:23 = KLLPETGGGSGS; Sortase cleavage-Tag
SEQ ID NO:24 = MGLFCSHPGDPLATTMGWSCIILFLVATATGVHSEFEVQLQESGGGLVQPK GSLQLSCAASGFTFNTYAMHWVRQAPGKGLEWVARIRTESSDYATDYAD SVKDRFIISRDDSQNMLYLQMNNLKSEDTAIYYCVRSSGFDYWGQGTTLT VSSSKTTPPSVYPLAPGCGDTTGSSVTLGCLVKGYFPESVTVTWNSGSLSSS VHTFPALLQSGLYTMSSSVTVPSSTWPSQTVTCSVAHPASSTTVDKKLKL( GGGGS)_{X}YPYDVPDYA(GGGGS)_{X}GTEVQLQQSGPVLVKPGASVKMSCRPS GYTFTDYNMHWVKQSHEKSLEWIGYINPKNGIISYNQKFKDKATLTVNKS SRTAYMELRSLTSEDSAVYFCAGDYWGQGTSVTVSSAKTTPPSVYPLAPGS AAQTNSMVTLGCLVKGYFPEPVTVTWNSGSLSSGVHTFPAVLQSDLYTLS SSVTVPSSTWPSETVTCNVAHPASSTKVDKKI; HC Dual binder, _{X} = 1, 2, 3 or 4 and indicates that amino acid sequence motive Gly-Gly-Gly-Gly-Ser (= GGGGS or G4S) is present 1, 2, 3 or 4 times.
SEQ ID NO:25 = WDQDPPERGAPPST; human HER2 (1223-1236)
SEQ ID NO:26 = PLHPVPIMPTAGTTPDEDYEYMNRQR; human HER3 (1242-1267)
SEQ ID NO:27 = 5'-(Gly)₄- Aminolinker-(Spacer C3)3-AGT TCT ATC GTC GTC CA-Fluorescein-3'; 17mer-Oligo
SEQ ID NO:28 = 5'-Fluorescein-AGT CTA TTA ATG CTT CTG C-(Spacer C3)3-Aminolinker-'-(Gly)4-3'; 19mer-Oligo

### Description of the Figures

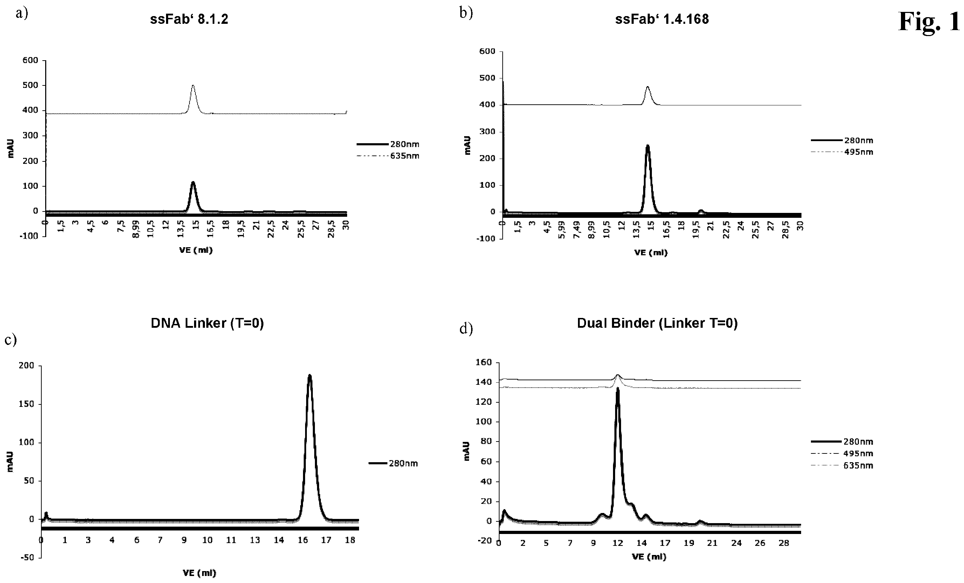

- **Figure 1**: Analytical gel filtration experiments assessing efficiency of the anti-pIGF1-R dual binder assembly. Diagrams a, b and c show the elution profile of the individual dual binder components (flourescein-ssFab' 1.4.168, Cy5-ssFab' 8.1.2 and Linker DNA (T=0); ssFab' denotes an Fab'-fragment conjugated to a single-stranded oligonucleotide). Diagram d shows the elution profile after the 3 components needed to form the bivalent binding agent had been mixed in a 1:1:1 molar ratio. The thicker (bottom) curve represents absorbance measured at 280nm indicating the presence of the ssFab' proteins or the linker DNA, respectively. The thinner top curve in b) and d) (absorbance at 495 nm) indicates the presence of fluorescein and the thinner top curve in a) and the middle curve in d) (absorbance at 635 nm) indicates the presence of Cy5. Comparison of the elution volumes of the single dual binder components (VE_{ssFab' 1.4.168} ∼15 ml; VE_{ssFab' 8.1.2} ∼15 ml; VEₗᵢₙₖₑᵣ ∼ 16 ml) with the elution volume of the reaction mix (VEₘᵢₓ ∼ 12 ml) demonstrates that the dual binder assembly reaction was successful (rate of yield: ∼ 90%). The major 280nm peak that represents the eluted dual binder nicely overlaps with the major peaks in the 495nm and 635nm channel, proving the presence of both ssFab' 8.1.2 and ssFab'1.4.168 in the peak representing the bivalent binding agent.

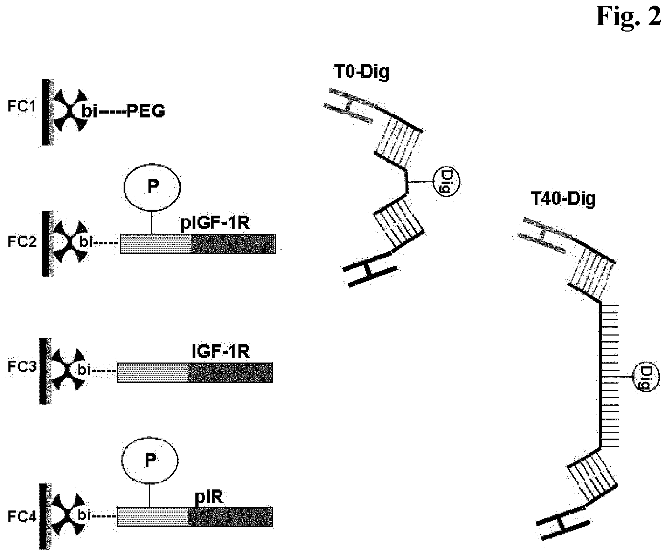

- **Figure 2**: Scheme of the Biacore™ experiment. Schematically and exemplarily, two binding molecules in solution are shown: The T0-T-Dig (linker 16), bivalent binding agent and the T40-T-Dig (linker 15), bivalent binding agent. Both these bivalent binding agents only differ in their linker-length (a central digoxigenylated T with no additional T versus 40 additional Ts (20 on each side of the central T-Dig), between the two hybridizing nucleic acid sequences). Furthermore, ssFab' fragments 8.1.2 and 1.4.168 were used.

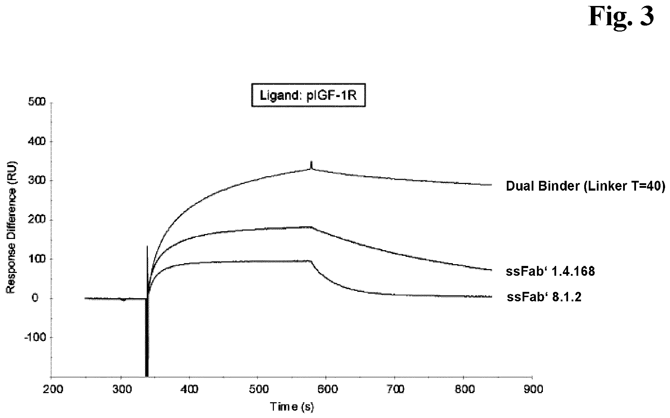

- **Figure 3**: Biacore™ sensorgram with overlay plot of three kinetics showing the interaction of 100 nM bivalent binding agent (consisting of ssFab' 8.1.2 and ssFab' 1.4.168 hybridized on the T40-T-Dig ssDNA-linker, i.e. linker 15) with the immobilized peptide pIGF-1R compared to the binding characteristics of 100 nM ssFab' 1.4.168 or 100 nM ssFab' 8.1.2 to the same peptide. Highest binding performance is obtained with the Dual Binder construct, clearly showing, that the cooperative binding effect of the Dual Binder increases affinity versus the target peptide pIGF-1R.

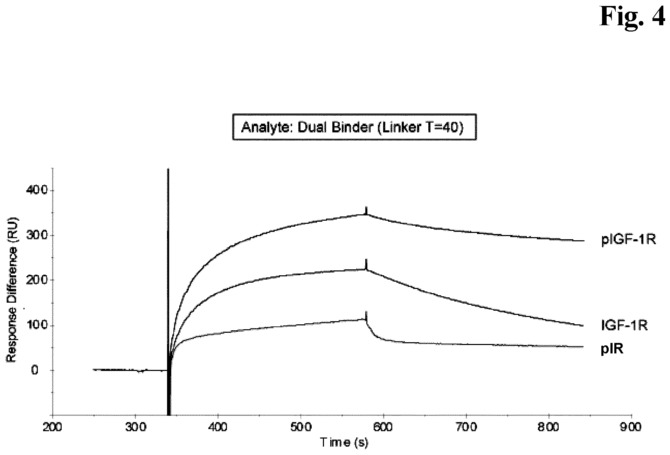

- **Figure 4**: Biacore™ sensorgram with overlay plot of three kinetics showing the interactions of the bivalent binding agent consisting of ssFab' 8.1.2 and ssFab' 1.4.168 hybridized on the T40-T-Dig ssDNA-linker, i.e. linker 15, with immobilized peptides pIGF-1R (phosphorylated IGF-1R), IGF-1R or pIR (phosphorylated insulin receptor). Highest binding performance is obtained with the pIGF-1R peptide, clearly showing, that the cooperative binding effect of the Dual Binder increases specificity versus the target peptide pIGF-1R as compared to e.g. the phosphorylated insulin receptor peptide (pIR).

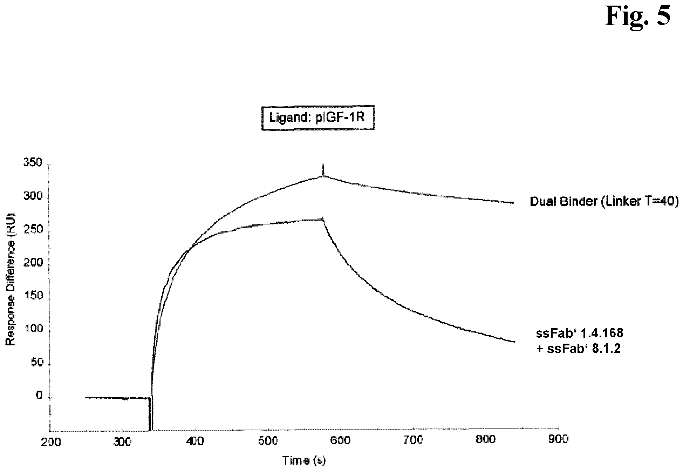

- **Figure 5**: Biacore™ sensorgram with overlay plot of two kinetics showing the interactions of 100 nM bivalent binding agent consisting of ssFab' 8.1.2 and ssFab' 1.4.168 hybridized on the T40-T-Dig ssDNA-linker, i.e. linker 15, and a mixture of 100 nM ssFab' 8.1.2 and 100 nM ssFab' 1.4.168 without linker DNA. Best binding performance is only obtained with the bivalent binding agent, whereas the mixture of the ssFab's without linker doesn't show an observable cooperative binding effect, despite the fact that the total concentration of these ssFab's had been at 200 nM.

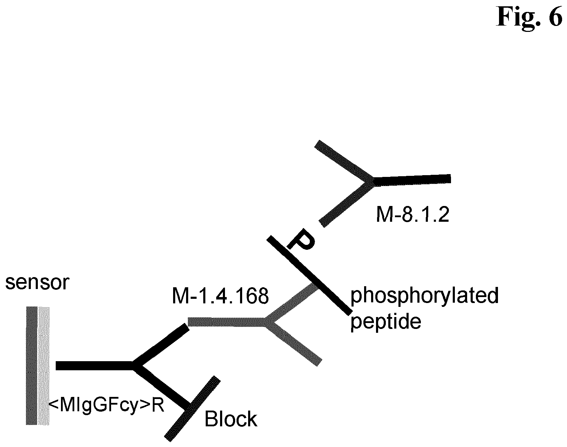

- **Figure 6**: Schematic drawing of a Biacore™ sandwich assay. This assay has been used to investigate the epitope accessibility for both antibodies on the phosphorylated IGF-1R peptide. <MIgGFcy>R presents a rabbit anti-mouse antibody used to capture the murine antibody M-1.4.168. M-1.4.168 then is used to capture the pIGF-1R peptide. M-8.1.2 finally forms the sandwich consisting of M-1.4.168, the peptide and M-8.1.2.

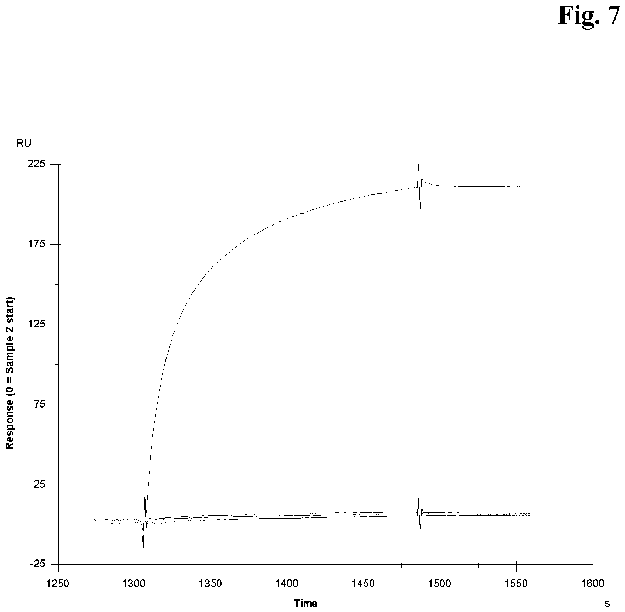

- **Figure 7**: Biacore™ sensorgram showing the binding signal (thick line) of the secondary antibody 8.1.2. to the pIGF-1R peptide after this was captured by antibody 1.4.168 on the Biacore™ chip. The other signals (thin lines) are control signals: given are the lines from top to bottom 500 nM 8.1.2, 500 nM 1.4.168; 500 nM target unrelated antibody <CKMM>M- 33-IgG; and 500 nM target unrelated control antibody <TSH>M-1.20-IgG, respectively. No binding event could be detected in any of these controls.

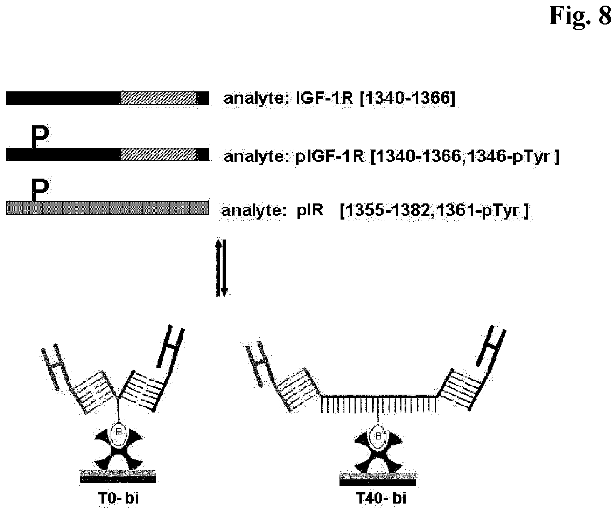

- **Figure 8**: Schematic drawing of the Biacore™ assay, presenting the biotinylated dual binders on the sensor surface. On Flow Cell 1 (=FC1) (not shown) amino-PEO-biotin was captured. On FC2, FC3 and FC4 bivalent binding agents with increasing linker length were immobilized (shown are the dual binders on FC2 (T0-bi = only one central T-Bi) and FC4 (T40-bi = one central T-Bi and 20 Ts each up- and downstream), respectively). Analyte 1: IGF-1R-peptide containing the M-1.4.168 ssFab' epitope at the right hand end of the peptide (top line) - the M-8.1.2 ssFab' phospho-epitope is not present, because this peptide is not phosphorylated; analyte 2: pIGF-1R peptide containing the M-8.1.2 ssFab' phospho-epitope (P) and the M-1.4.168 ssFab' epitope (second line); analyte 3: pIR peptide, containing the cross reacting M-8.1.2 ssFab' phospho-epitope, but not the epitope for M-1.4.168 (third line).

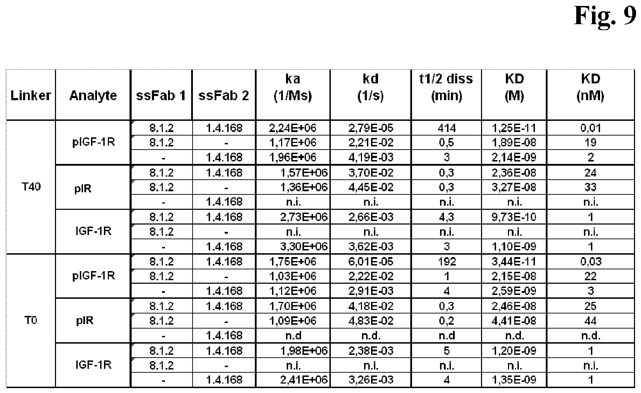

- **Figure 9**: Kinetic data of the Dual Binder experiment. T40-T-Bi linker Dual Binder with ssFab' 8.1.2 and ssFab' 1.4.168 (= T40 in the Figure) shows a 1300-fold lower off-rate (kd = 2.79E-05/s) versus pIGF-1R when compared to pIR (kd = 3.70E-02/s).

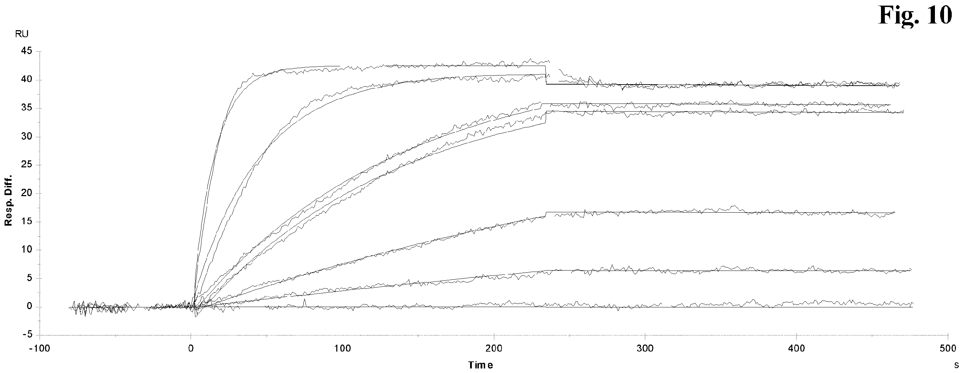

- **Figure 10**: Biacore™ sensorgram, showing concentration dependent measurement of the T40-T-Bi dual binding agent vs. the pIGF-1R peptide (the phosphorylated IGF-1R peptide). The assay setup was as depicted in Fig. 8. A concentration series of the pIGF-1R peptide was injected at 30 nM, 10 nM, 2x 3.3 nM, 1.1 nM, 0.4 nM, 0 nM. The corresponding data are given in the table of Fig. 9.

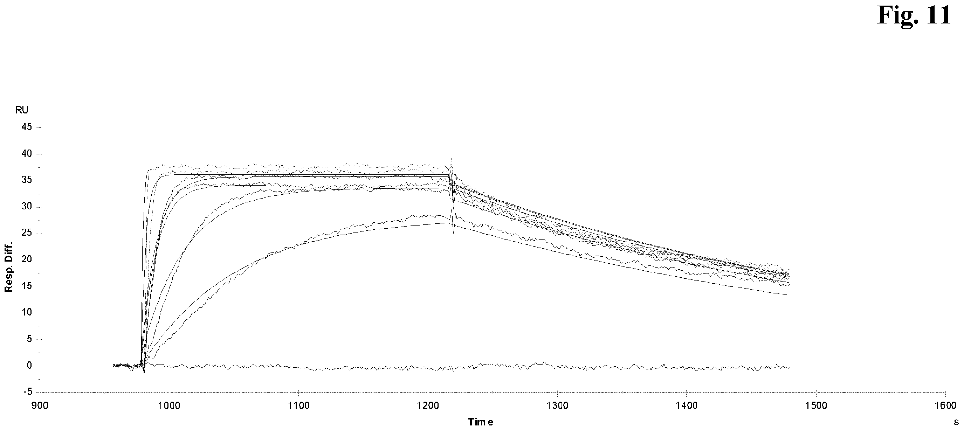

- **Figure 11**: Biacore™ sensorgram, showing concentration dependent measurement of the T40-T-Bi dual binding agent vs. the IGF-1R peptide (the non-phosphorylated IGF-1R peptide). The assay setup was as depicted in Fig. 8. A concentration series of the IGF-1R peptide was injected at 300 nM, 100 nM, 2x 33 nM, 11 nM, 4 nM, 0 nM. The corresponding data are given in the table of Fig. 9.

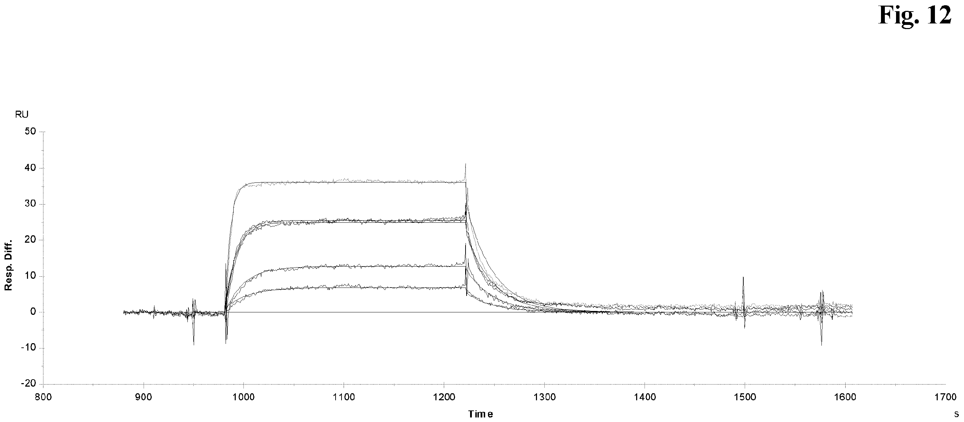

- **Figure 12**: Biacore™ sensorgram, showing concentration dependent measurement of the T40-T-Bi dual binding agent vs. the pIR peptide (the phosphorylated insulin receptor peptide). The assay setup was as depicted in Fig. 8. A concentration series of the pIR peptide was injected at 100 nM, 2x 33 nM, 11 nM, 4 nM, 0 nM. The corresponding data are given in the table depicted as Fig. 9.

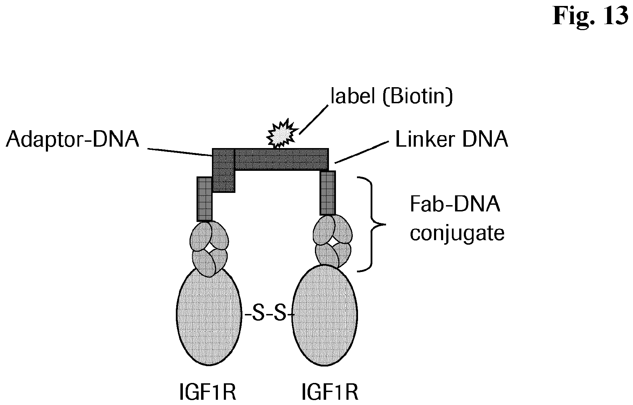

- **Figure 13**: Dual-binding agent binding the IGF1-receptor. Two Fab' fragments of mAbl.4.168 are conjugated with single stranded DNA and hybridized with an adaptor DNA and a linker DNA. As depicted, the linker DNA carries a biotin label, which can be detected in immunhistochemical stainings.

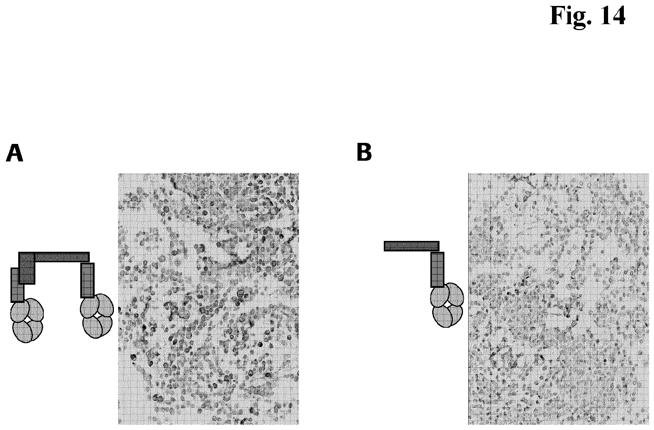

- **Figure 14**: IHC detection of the IGF1 receptor by the dual binding agent. The dual binding agent (1µg/mL) stains stably transfected NIH3T3 cells expressing the IGF1 receptor (A). A negative (monovalent) control (Fab'-ssDNA) without the adaptor DNA is washed off and does not stain the cells (B).

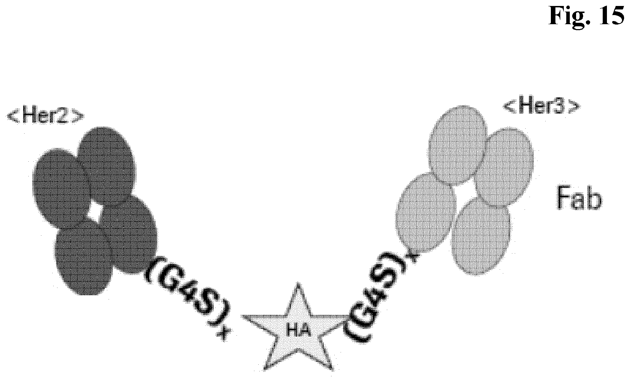

- **Figure 15**: The dual binder for HER2/HER3 heterodimer detection. The dual binder consists of two Fab fragments, one recognizing HER2 (=<Her2> in the Figure) and one recognizing HER3 (=<Her3> in the Figure). The Fab fragments are joined by a peptide linker that consists of a hemagglutinin-Tag (HA) and units of Gly-Gly-Gly-Gly-Ser (G4S) amino acid residue motives. The length of the linker can easily be varied by the introduction of several G4S units (x=1, 2, 3, 4...) to optimize the distance between the Fab fragments for dimer detection. The dual binder used in example 4 has two G4S units (x=1).

### Example 1

### Bivalent binding agent to troponin T

### 1.1 Monoclonal antibodies and Fab'-fragments

Two monoclonal antibodies binding to human cardiac Troponin T at different, nonoverlapping epitopes, epitope A' and epitope B', respectively, were used. Both these antibodies are used in the current Roche Elecsys™ Troponin T assay, wherein Troponin T is detected in a sandwich immuno assay format.

Purification of the monoclonal antibodies from culture supernatant was carried out using state of the art methods of protein chemistry.

The purified monoclonal antibodies are protease digested with either pre-activated papain (anti-epitope A' MAb) or pepsin (anti-epitope B' MAb) yielding F(ab')2 fragments that are subsequently reduced to Fab'-fragments, i.e. A and B, respectively, in Formula I (A-a':a-S-b:b'-B), with a low concentration of cysteamin at 37°C. The reaction is stopped by separating the cysteamin on a Sephadex G-25 column (GE Healthcare) from the polypeptide-containing part of the sample.

### 1.2 Conjugation of Fab'-fragments to ssDNA-oligonucleotides

The Fab'-fragments are conjugated with the below described activated ssDNAa and ssDNAb oligonucleotides, respectively.

### Preparation of the Fab'-fragment-ssDNA conjugates A" and B", respectively:

### a) Fab'-anti-Troponin T <epitope A'>-ssDNA-conjugate (=A")

For preparation of the Fab'-anti-Troponin T <epitope A'>-ssDNAa-conjugate A" a derivative of SED ID NO:5 is used, i.e. 5'-AGT CTA TTA ATG CTT CTG C(=SEQ ID NO:5)-XXX-Y-Z-3', wherein X = propylene-phosphate introduced via Phosphoramidite C3 (3-(4,4'-Dimethoxytrityloxy)propyl-1-[(2-cyanoethyl)-(N,N-diisopropyl)]-phosphoramidite (Glen Research), wherein Y = 3 "-Amino-Modifier C6 introduced via 3'-Amino Modifier TFA Amino C-6 lcaa CPG (ChemGenes) and wherein Z = 4[N-maleinimidomethyl]cyclohexane-1-carboxy introduced via Sulfosuccinimidyl 4-[N-maleimidomethyl]cyclohexane-1-carboxylate (ThermoFischer).

### b) Fab'-anti-Troponin T <epitope B'>-ssDNAb-conjugate (=B")

For the preparation of the Fab'-anti-Troponin T <epitope B'>-ssDNA-conjugate (B") a derivative of SEQ ID NO:6 is used, i.e. 5'-Y-Z-XXX-AGT TCT ATC GTC GTC CA-3', wherein X = propylene-phosphate introduced via Phosphoramidite C3 (3-(4,4'-Dimethoxytrityloxy)propyl-1-[(2-cyanoethyl)-(N,N-diisopropyl)]-phosphoramidite (Glen Research), wherein Y = 5'-Amino-Modifier C6 introduced via (6-(4-Monomethoxytritylamino)hexyl-(2-cyanoethyl)-(N,N-diisopropyl)-phosphoramidite (Glen Research), and wherein Z = 4[N-maleinimidomethyl]cyclohexane-1-carboxy introduced via Sulfosuccinimidyl 4-[N-maleimidomethyl]cyclohexane-1-carboxylate (ThermoFischer).

The oligonucleotides of SEQ ID NO:5 or 6, respectively, have been synthesized by state of the art oligonucleotide synthesis methods. The introduction of the maleinimido group was done via reaction of the amino group of Y with the succinimidyl group of Z which was incorporated during the solid phase oligonucleotide synthesis process.

The single-stranded DNA constructs shown above bear a thiol-reactive maleimido group that reacts with a cysteine of the Fab' hinge region generated by the cysteamine treatment. In order to obtain a high percentage of single-labeled Fab'-fragments the relative molar ratio of ssDNA to Fab'-fragment is kept low. Purification of single-labeled Fab'-fragments (ssDNA:Fab' = 1:1) occurs via anion exchange chromatography (column: MonoQ, GE Healthcare). Verification of efficient labeling and purification is achieved by analytical gel filtration chromatography and SDS-PAGE.

### 1.3 Biotinylated linker molecules

The oligonucleotides used in the ssDNA linkers L1, L2 and L3, respectively, have been synthesized by state of the art oligonucleotide synthesis methods and employing a biotinylated phosphoramidite reagent for biotinylation.
Linker 1 (=L1), a biotinylated ssDNA linker 1 with no spacer has the following composition:
   5'-GCA GAA GCA TTA ATA GAC T (Biotin-dT)-TGG ACG ACG ATA GAA CT-3'. It comprises ssDNA oligonucleotides of SEQ ID NO:7 and 8, respectively, and was biotinylated by using Biotin-dT (=T-Bi) (5'-Dimethoxytrityloxy-5-[N-((4-t-butylbenzoyl)-biotinyl)-aminohexyl)-3-acrylimido]-2'-deoxyUridine-3'-[(2-cyanoethyl)-(N,N-diisopropyl)]-phosphoramidite (Glen Research) in the middle of the spacer.
Linker 2 (=L2), a biotinylated ssDNA linker 2 with a 11mer thymidine-based spacer has the following composition:
   5'-GCA GAA GCA TTA ATA GAC T T5-(Biotin-dT)-T5 TGG ACG ACG ATA GAA CT-3'. It comprises ssDNA oligonucleotides of SEQ ID NO:7 and 8, respectively, twice oligonucleotide stretches of five thymidines each and was biotinylated by using Biotin-dT (5'-Dimethoxytrityloxy-5-[N-((4-t-butylbenzoyl)-biotinyl)-aminohexyl)-3-acrylimido]-2'-deoxyUridine-3'-[(2-cyanoethyl)-(N,N-diisopropyl)]-phosphoramidite (Glen Research) in the middle of the spacer.
Linker 3 (=L3), a biotinylated ssDNA linker 3 with a 31mer thymidine-based spacer has the following composition:
   5'-GCA GAA GCA TTA ATA GAC T T15-(Biotin-dT)-T15 TGG ACG ACG ATA GAA CT-3'. It comprises ssDNA oligonucleotides of SEQ ID NO:7 and 8, respectively, twice oligonucleotide stretches of fifteen thymidines each and was biotinylated by using Biotin-dT (5'-Dimethoxytrityloxy-5-[N-((4-t-butylbenzoyl)-biotinyl)-aminohexyl)-3-acrylimido]-2'-deoxyUridine-3'-[(2-cyanoethyl)-(N,N-diisopropyl)]-phosphoramidite (Glen Research).

### 1.4 Epitopes for monovalent Troponin T binders A and B, respectively

Synthetic peptides have been construed that individually only have a moderate affinity to the corresponding Fab'-fragment derived from the anti-Troponin T antibodies a and b, respectively.

### a) The epitope A' for antibody a is comprised in:

SEQ ID NO:9 = ERAEQQRIRAEREKEUUSLKDRIEKRRRAERAEamide, wherein U represents β-Alanin.

### b) The epitope B' for antibody b is comprised in:

SEQ ID NO:10 = SLKDRIERRRAERAEOOERAEQQRIRAEREKEamide, wherein O represents Amino-trioxa-octanoic-acid

As the skilled artisan will appreciate it is possible to combine these two epitope-containing peptides in two ways and both variants have been designed and prepared by linear combining the epitopes A' and B'. The sequences of both variants, the linear sequences of epitopes A'-B' (= TnT-1) and B'-A' (=TnT-2), respectively have been prepared by state of the art peptide synthesis methods.

The sequences for epitopes A' and B', respectively, had been modified compared to the original epitopes on the human cardiac Troponin T sequence (P45379/UniProtKB) in order to reduce the binding affinity for each of the Fabs thereto. Under these circumstances the dynamics of the effect of hetero-bivalent binding is better visible, e.g. by analyzing binding affinity with the Biacore™ Technology.

### 1.5 Biomolecular Interaction Analysis

For this experiment a Biacore™ 3000 instrument (GE Healthcare) was used with a Biacore™ SA sensor mounted into the system at T = 25°C. Preconditioning was done at 100 µl/min with 3x1 min injection of 1 M NaCl in 50 mM NaOH and 1 min 10 mM HCl.

HBS-ET (10 mM HEPES pH 7.4, 150 mM NaCl, 1mM EDTA, 0.05% Tween® 20 was used as system buffer. The sample buffer was identical to the system buffer.

The Biacore™ 3000 System was driven under the control software V1.1.1. Flow cell 1 was saturated with 7 RU D-biotin. On flow cell 2, 1063 RU biotinylated ssDNA linker L1 was immobilized. On flow cell 3, 879 RU biotinylated ssDNA linker L2 was immobilized. On flow cell 4, 674 RU biotinylated ssDNA linker L3 was captured.

Thereafter, Fab' fragment DNA conjugate A" was injected at 600 nM. Fab' fragment DNA conjugate B" was injected into the system at 900 nM. The conjugates were injected for 3 min at a flow rate of 2 µl/min. The conjugates were consecutively injected to monitor the respective saturation signal of each Fab' fragment DNA conjugate on its respective linker. Fab' combinations were driven with a single Fab' fragment DNA conjugate A", a single Fab' fragment DNA conjugate B" and both Fab' fragment DNA conjugates A" and B" present on the respective linker. Stable baselines were generated after the linkers have been saturated by the Fab' fragment DNA conjugates, which was a prerequisite for further kinetic measurements.

The artificial peptidic analytes TnT-1 and TnT-2 were injected as analytes in solution into the system in order to interact with the surface presented Fab' fragments.

TnT-1 was injected at 500 nM, TnT-2 was injected at 900 nM analyte concentration. Both peptides were injected at 50 µl/min for 4 min association time. The dissociation was monitored for 5 min. Regeneration was done by a 1 min injection at 50 µl/min of 50 mM NaOH over all flow cells.

Kinetic data was determined using the Biaevaluation software (V.4.1). The dissociation rate kd (1/s) of the TnT-1 and TnT-2 peptides from the respective surface presented Fab' fragment combinations was determined according to a linear Langmuir 1:1 fitting model. The complex halftime in min were calculated according to the solution of the first order kinetic equation : ln(2)/ (60*kd).

### Results:

The experimental data given in Tables 1 and 2, respectively demonstrate an increase in complex stability between analyte (TnT-1 or TnT-2), respectively, and the various hetero bivalent Fab'-Fab' dimers A"-B" as compared to the monovalent dsDNA Fab' A" or B" conjugate, respectively. This effect is seen in each Table in line 1 compared to lines 2 and 3.

**Table 1: Analysis data using TnT-1 with linkers of various length**

| **a) Linker L1** | | | |
|---|---|---|---|
| **Fab' fragment DNA conjugate A"** | **Fab' fragment DNA conjugate B"** | **kd (1/s)** | **t1/2 diss (min)** |
| x | x | 6.6E-03 | 1.7 |
| x | - | 3.2E-02 | 0.4 |
| - | x | 1.2E-01 | 0.1 |

| **b) Linker L2** | | | |
|---|---|---|---|
| **Fab' fragment DNA conjugate A"** | **Fab' fragment DNA conjugate B"** | **kd (1/s)** | **t1/2 diss (min)** |
| x | x | 4.85E-03 | 2.4 |
| x | - | 2.8E-02 | 0.4 |
| - | x | 1.3E-01 | 0.1 |

| **c) linker L3** | | | |
|---|---|---|---|
| **Fab' fragment DNA conjugate A"** | **Fab' fragment DNA conjugate B"** | **kd (/1/s)** | **t1/2 diss (min)** |
| x | x | 2.0E-03 | 5.7 |
| x | - | 1.57E-02 | 0.7 |
| - | x | 1.56E-02 | 0.7 |

**Table 2: Analysis data using TnT-2 with linkers of various length**

| **a) Linker L1** | | | |
|---|---|---|---|
| **Fab' fragment DNA conjugate A"** | **Fab' fragment DNA conjugate B"** | **kd (/1/s)** | **t1/2 diss (min)** |
| x | x | 1.4E-02 | 0.8 |
| x | - | 4.3E-02 | 0.3 |
| - | x | 1.4E-01 | 0.1 |

| **b) Linker L2** | | | |
|---|---|---|---|
| **Fab' fragment DNA conjugate A"** | **Fab' fragment DNA conjugate B"** | **kd (/1/s)** | **t1/2 diss (min)** |
| x | x | 4.9E-03 | 2.3 |
| x | - | 3.5E-02 | 0.3 |
| - | x | 1.3E-01 | 0.1 |

| **c) Linker L3** | | | |
|---|---|---|---|
| **Fab' fragment DNA conjugate A"** | **Fab' fragment DNA conjugate B"** | **kd (/1/s)** | **t1/2 diss (min)** |
| x | x | 8.0E-03 | 1.5 |
| x | - | 4.9E-02 | 0.2 |
| - | x | 3.2E-01 | 0.04 |

The avidity effect is further dependent on the length of the linker. In the sub-tables shown under Table 1, i.e. for the artificial analyte TnT-1, the linker L3 comprising a 31mer spacer shows the lowest dissociation rate or highest complex stability.

In the sub-tables shown under Table 2 the linker L2 comprising an 11mer spacer exhibits the lowest dissociation rate or highest complex stability for the artificial analyte TnT-2.

These data taken together demonstrate that the flexibility in linker length as inherent to the approach given in the present invention is of great utility and advantage.

### Example 2

### Bivalent binding agent to phosphorylated IGF-1R

### 2.1 Monoclonal antibody development (mAb 8.1.2 and mAb 1.4.168)

### a) Immunization of mice

BALB/C mice are immunized at week 0, 3, 6 and 9, respectively. Per immunization 100 µg of the conjugate comprising the phosphorylated peptide pIGF-1R (1340-1366) (SEQ ID NO:11) is used. This peptide had been phosphorylated at tyrosine 1346 (=1346-pTyr) and coupled to KLH via the C-terminal cysteine (=Aoc-Cys-MP-KLH-1340) to yield the conjugate used for immunization. At weeks 0 and 6, respectively, the immunization is carried out intraperitoneally and at weeks 3 and 9, respectively, subcutanuosly at various parts of the mouse body.

### b) Fusion and cloning

Spleen cells of immunized mice are fused with myeloma cells according to Galfre G., and Milstein C., Methods in Enzymology 73 (1981) 3-46. In this process ca 1x10⁸ spleen cells of an immunized mouse are mixed with 2x10⁷ myeloma cells a(P3X63-Ag8653, ATCC CRL1580) and centrifuged (10 min at 250 g and 37°C). The cells are then washed once with RPMI 1640 medium without fetal calf serum (FCS) and centrifuged again at 250 g in a 50 ml conical tube. The supernatant is discarded, the cell sediment is gently loosened by tapping, 1 ml PEG (molecular weight 4000, Merck, Darmstadt) is added and mixed by pipetting. After 1 min incubation in a water bath at 37°C, 5 ml RPMI 1640 without FCS is added dropwise at room temperature within a period of 4-5 min. This step is repeated with additional 10 ml RPMI 1640 without FCS. Afterwards 25 ml RPMI 1640 containing 10% FCS is added followed by an incubation step at 37°C, 5% CO₂ for 30 minutes. After centrifugation for 10 min at 250 g and 4°C the sedimented cells are taken up in RPMI 1640 medium containing 10% FCS and seeded out in hypoxanthine-azaserine selection medium (100 mmol/l hypoxanthine, 1 µg/ml azaserine in RPMI 1640+10% FCS). Interleukin 6 at 100 U/ml is added to the medium as a growth factor. After 7 days the medium is exchanged with fresh medium. On day 10, the primary cultures are tested for specific antibodies. Positive primary cultures are cloned in 96-well cell culture plates by means of a fluorescence activated cell sorter.

### c) Immunoglobulin isolation from the cell culture supernatants

The hybridoma cells obtained are seeded out at a density of 1x 10⁷ cells in CELLine 1000 CL flasks (Integra). Hybridoma cell supernatants containing IgGs are collected twice a week. Yields typically range between 400 µg and 2000 µg of monoclonal antibody per 1 ml supernatant. Purification of the antibody from culture supernatant was carried out using conventional methods of protein chemistry (e.g. according to Bruck, C., Methods in Enzymology 121 (1986) 587-695).

### 2.2 Synthesis of hybridizable oligonucleotides

The following amino modified precursors, comprising the sequences given in SEQ ID NOs: 5 and 6, respectively, were synthesized according to standard methods. The below given oligonucleotides not only comprise the so-called aminolinker, but also a fluorescent dye. As the skilled artisan will readily appreciate, this fluorescent dye is very convenient to facilitate purification of the oligonucleotide as such, as well as of components comprising them.
a) 5'-Fluorescein-AGT CTA TTA ATG CTT CTG C-(Spacer C3)3-C7Aminolinker-;
b) 5'-Cy5 AGT CTA TTA ATG CTT CTG C-(Spacer C3)3-C7Aminolinker-;
c) 5'-Aminolinker-(Spacer C3)3-AGT TCT ATC GTC GTC CA-Fluorescein-3';
d) 5'-Fluorescein-(beta L AGT CTA TTA ATG CTT CTG C)-(Spacer C3)3-C7Aminolinker-; (beta L indicates that this is an L-DNA oligonucleotide) and
e) 5'-Aminolinker-(Spacer C3)3-(beta L-AGT TCT ATC GTC GTC CA)-Fluorescein-3' (beta L indicates that this is an L-DNA oligonucleotide).

Synthesis was performed on an ABI 394 synthesizer at a 10 µmol scale in the trityl on (for 5' amino modification) or trityl off mode (for 3' amino modification) using commercially available CPGs as solid supports and standard dA(bz), dT, dG (iBu) and dC(Bz) phosphoramidites (Sigma Aldrich).

The following amidites, amino modifiers and CPG supports were used to introduce the C3-spacer, a dye and amino moieties, respectively, during oligonucleotide synthesis:
Spacer Phosphoramidite C3 (3-(4,4'-Dimethoxytrityloxy)propyl-1-[(2-cyanoethyl)-(N,N-diisopropyl)]-phosphoramidite (Glen Research);
5' amino modifier is introduced by using 5'-Amino-Modifier C6 (6-(4-Monomethoxytritylamino)hexyl-(2-cyanoethyl)-(N,N-diisopropyl)-phosphoramidite (Glen Research);
5'-Fluorescein Phosphoramidite 6-(3',6'-dipivaloylfluoresceinyl-6-carboxamido)-hexyl-1-O-(2-cyanoethyl)-(N,N-diisopropyl)-phosphoramidite (Glen Research);
Cy5™ Phosphoramidite 1-[3-(4-monomethoxytrityloxy)propyl]-1'-[3-[(2-cyanoethyl)-(N,N-diisopropyl phosphoramidityl]propyl]-3,3,3',3'-tetramethylindodicarbocyanine chloride (Glen Research);
LightCycler Fluoresceine CPG 500 A (Roche Applied Science); and
3'-Amino Modifier TFA Amino C-6 lcaa CPG 500 A (Chemgenes),

For Cy5 labeled oligonucleotides, dA(tac), dT, dG(tac) dC(tac) phosphoramidites, (Sigma Aldrich), were used and deprotection with 33% ammonia was performed for 2h at room temperature.

L- DNA oligonucleotides were synthesized by using beta-L-dA(bz), dT, dG (iBu) and dC(Bz) phosphoramidites (Chemgenes)

Purification of fluorescein modified hybridizable oligonucleotides was performed by a two step procedure: First the oligonucleotides were purified on reversed-phase HPLC (Merck-Hitachi-HPLC; RP-18 column; gradient system [A: 0.1 M (Et3NH)OAc (pH 7.0)/MeCN 95:5; B: MeCN]: 3 min, 20% B in A, 12 min, 20-50% B in A and 25 min, 20% B in A with a flow rate of 1.0 ml/min, detection at 260 nm. The fractions (monitored by analytical RP HPLC) containing the desired product were combined and evaporated to dryness. (Oligonucleotides modified at the 5' end with monomethoxytrityl protected alkylamino group are detriylated by incubating with 20% acetic acid for 20 min). The oligomers containing fluorescein as label were purified again by IEX chromatography on a HPLC [Mono Q column: Buffer A: Sodium hydroxide (10 mM/l; pH ∼12) Buffer B 1M Sodium chloride dissolved in Sodium hydroxide (10 mM/l; pH ∼12) gradient: in 30 minutes from 100% buffer A to 100% buffer B flow 1 ml/min detection at 260 nm]. The product was desalted via dialysis.

Cy5 labeled oligomers were used after the first purification on reversed-phase HPLC (Merck-Hitachi-HPLC; RP-18 column; gradient system [A: 0.1 M (Et3NH)OAc (pH 7.0)/MeCN 95:5; B: MeCN]: 3 min, 20% B in A, 12 min, 20-50% B in A and 25 min, 20% B in A with a flow rate of 1.0 ml/min, detection at 260 nm. The oligomers were desalted by dialysis and lyophilized on a Speed-Vac evaporator to yield solids which were frozen at -24°C.

### 2.3 Activation of hybridizable oligonucleotides

The amino modified oligonucleotides from Example 2 were dissolved in 0,1 M sodium borate buffer pH 8.5 buffer (c= 600 µmol) and reacted with a 18-fold molar excess of Sulfo SMCC (Sulfosuccinimidyl 4-[N-maleimidomethyl]cyclohexane-1-carboxylate dissolved in DMF (c= 3mg/100µl) from Thermo Scientific, The reaction product was thoroughly dialyzed against water in order to remove the hydrolysis product of sulfoSMCC 4-[N-maleimidomethyl]cyclohexane-1-carboxylate.

The dialysate was concentrated by evaporation and directly used for conjugation with a monovalent binder comprising a thiol group.

### 2.4 Synthesis of linker oligonucleotides comprising hybridizable oligonucleotides at both ends

Oligonucleotides were synthesized by standard methods on an ABI 394 synthesizer at a 10 µmol scale in the trityl on mode using commercially available dT-CPG as solid supports and using standard dA(bz), dT, dG (iBu) and dC(Bz) phosphoramidites (Sigma Aldrich).

L- DNA oligonucleotides were synthesized by using commercially available beta L-dT-CPG as solid support and beta-L-dA(bz), dT, dG (iBu) and dC(Bz) phosphoramidites (Chemgenes)

Purification of the oligonucleotides was performed as described under Example 2.3 on a reversed-phase HPLC. The fractions (analyzed/monitored by analytical RP HPLC) containing the desired product were combined and evaporated to dryness. Detriylation was performed by incubating with 80% acetic acid for 15 min) The acetic acid was removed by evaporation. The reminder was dissolved in water and lyophilized

The following amidites and CPG supports were used to introduce the C18 spacer, digoxigenin and biotin group during oligonucleotide synthesis:
Spacer Phosphoramidite 18 (18-O-Dimethoxytritylhexaethyleneglycol,1-[(2-cyanoethyl)-(N,N-diisopropyl)]-phosphoramidite (Glen Research);
Biotin-dT (5'-Dimethoxytrityloxy-5-[N-((4-t-butylbenzoyl)-biotinyl)-aminohexyl)-3-acrylimido]-2'-deoxyUridine-3'-[(2-cyanoethyl)-(N,N-diisopropyl)]-phosphoramidite (Glen Research);
Biotin Phosphoramidite1-Dimethoxytrityloxy-2-(N-biotinyl-4-aminobutyl)-propyl-3-O-(2-cyanoethyl)-(N,N-diisopropyl)-phosphoramidite and
5'-Dimethoxytrityl-5-[N-(trifluoroacetylaminohexyl)-3-acrylimido]- 2'-deoxy uridine, 3'-[(2-cyanoethyl)-(N,N-diisopropyl)]-phosphoramidite for amino modification and postlabeling with Digoxigenin-N-Hydroxyl-succininimidyl ester.

The following bridging constructs or linkers were synthesized:
Linker 1: 5'-G CAG AAG CAT TAA TAG ACT-TGG ACG ACG ATA GAA CT-3'
Linker 2: 5'-G CAG AAG CAT TAA TAG ACT-(T40)-TGG ACG ACG ATA GAA CT-3'
Linker 3: 5'-[B-L]G CAG AAG CAT TAA TAG ACT-(Biotin-dT)-TGG ACG ACG ATA GAA CT-3'
Linker 4: 5'-[B-L]G CAG AAG CAT TAA TAG ACT-T5-(Biotin-dT)-T5-TGG ACG ACG ATA GAA CT-3'
Linker 5: 5'-[B-L]G CAG AAG CAT TAA TAG ACT-T20-(Biotin-dT)-T20-TGG ACG ACG ATA GAA CT-3'
Linker 6: 5'-[B-L] G CAG AAG CAT TAA TAG ACT-T30-(Biotin-dT)-T30-TGG ACG ACG ATA GAA CT-3'
Linker 7: 5'-GCA GAA GCA TTA ATA GAC T T5-(Biotin-dT)-T5 TG GAC GAC GAT AGA ACT-3'
Linker 8: 5'-GCA GAA GCA TTA ATA GAC T T10-(Biotin-dT)-T10 TGG ACG ACG ATA GAA CT-3'
Linker 9: 5'-GCA GAA GCA TTA ATA GAC T T15-(Biotin-dT)-T15 TGG ACG ACG ATA GAA CT-3'
Linker 10: 5'-GCA GAA GCA TTA ATA GAC T T20-(Biotin-dT)-T20 TGG ACG ACG ATA GAA CT-3'
Linker 11: 5'-G CAG AAG CAT TAA TAG ACT-Spacer C18- (Biotin-dT)-Spacer C18-TGG ACG ACG ATA GAA CT-3'
Linker 12: 5'-G CAG AAG CAT TAA TAG ACT-(Spacer C18)2-(Biotin-dT)-(Spacer C18)2-TGG ACG ACG ATA GAA CT-3'
Linker 13: 5'-G CAG AAG CAT TAA TAG ACT-(Spacer C18)3-(Biotin-dT)-(Spacer C18)3-TGG ACG ACG ATA GAA CT-3'
Linker 14: 5'-G CAG AAG CAT TAA TAG ACT-(Spacer C18)4-(Biotin-dT)-(Spacer C18)4-TGG ACG ACG ATA GAA CT-3'
Linker 15: 5'-G CAG AAG CAT TAA TAG ACT-T20-(Dig-dT)-T20-TGG ACG ACG ATA GAA CT-3'
Linker 16: 5'-G CAG AAG CAT TAA TAG ACT-(Dig-dT)-TGG ACG ACG ATA GAA CT-3'
Linker 17: 5'-G CAG AAG CAT TAA TAG ACT-(Biotin-dT)-TGG ACG ACG ATA GAA CT-3'

The above bridging construct examples comprise at least a first hybridizable oligonucleotide and a second hybridizable oligonucleotide. Linkers 3 to 17 in addition to the hybridizable nucleic acid stretches comprise a central biotinylated or digoxigenylated thymidine, respectively, or a spacer consisting of thymidine units of the length given above.

The 5'-hybridizable oligonucleotide corresponds to SEQ ID NO:7 and the 3'-hybridizable oligonucleotide corresponds to SEQ ID NO:8, respectively. The oligonucleotide of SEQ ID NO:7 will readily hybridize with the oligonucleotide of SED ID NO:5. The oligonucleotide of SEQ ID NO:8 will readily hybridize with the oligonucleotide of SED ID NO:6.

In the above bridging construct examples [B-L] indicates that an L-DNA oligonucleotide sequence is given; spacer C18, Biotin and Biotin dT respectively, refer to the C18 spacer, the Biotin and the Biotin-dT as derived from the above given building blocks; and T with a number indicates the number of thymidine residues incorporated into the linker at the position given.

### 2.5 Assembly of dual binder construct

### A) Cleavage of IgGs and labeling of Fab' fragments with ssDNA

Purified monoclonal antibodies were cleaved with the help of pepsin protease yielding F(ab')2 fragments that are subsequently reduced to Fab' fragments by treatment with low concentrations of cysteamine at 37°C. The reaction is stopped via separation of cysteamine on a PD 10 column. The Fab' fragments are labeled with an activated oligonucleotide as produced according to Example 3. This single-stranded DNA (=ssDNA) bears a thiol-reactive maleimido group that reacts with the cysteines of the Fab' hinge region. In order to obtain high percentages of single-labeled Fab' fragments the relative molar ratio of ssDNA to Fab'-fragment is kept low. Purification of single-labeled Fab' fragments (ssDNA: Fab' = 1:1) occurs via ion exchange chromatography (column: Source 15 Q PE 4.6/100, Pharmacia/GE). Verification of efficient purification is achieved by analytical gel filtration and SDS-PAGE.

### B) Assembly of an anti-pIGF-1R dual binder.

The anti-pIGF-1R dual binder is based on two Fab' fragments that target different epitopes of the intracellular domain of IGF-1R: Fab' 8.1.2 detects a phosphorylation site (pTyr 1346) and Fab' 1.4.168 a non-phospho site of the said target protein. The Fab' fragments have been covalently linked to single-stranded DNA (ssDNA): Fab' 1.4.168 to a 17mer ssDNA comprising SEQ ID NO:6 and containing fluorescein as an fluorescent marker and Fab' 8.1.2 to a 19mer ssDNA comprising SEQ ID NO:5 and containing Cy5 as fluorescent marker. In the following, these Fab's with covalently bound 17mer or 19mer ssDNA are named ssFab' 1.4.168 and ssFab' 8.1.2 respectively. Dual binder assembly is mediated by a linker (i.e. a bridging construct comprising two complementary ssDNA oligonucleotides (SEQ ID NOs:7 and 8, respectively) that hybridize to the corresponding ssDNAs of the ssFab' fragments. The distance between the two ssFab' fragments of the dual binder can be modified by using spacers, e.g. C18-spacer or DNAs of different length, respectively.

For assembly evaluation the dual binder components ssFab' 8.1.2, ssFab' 1.4.168 and the linker constructs (I) (= linker 17 of example 2.4) 5'-G CAG AAG CAT TAA TAG ACT T(-Bi)-TGG ACG ACG ATA GAA CT-3' and (II) (= linker 10 of example 2.4) 5'-G CAG AAG CAT TAA TAG ACT-(T20)-T(-Bi)-(T20)-TGG ACG ACG ATA GAA CT-3' were mixed in equimolar quantities at room temperature. After a 1 minute incubation step the reaction mix was analyzed on an analytical gel filtration column (Superdex™ 200, 10/300 GL, GE Healthcare). Comparison of the elution volumes (V_{E}) of the single dual binder components with the V_{E} of the reaction mix demonstrates that the dual binder has been formed successfully (Figl). (The biotinylated thymidine (T-(Bi)) in the middle of both of the linkers is without function in these experiments.)

### 2.6 Biacore™ experiment assessing binding of anti-pIGF-1R dual binder to immobilized IGF-1R and IR peptides

For this experiment a Biacore™ 2000 instrument (GE Healthcare) was used with a Biacore™ SA sensor mounted into the system at T = 25°C. Preconditioning occurred at 100 µl/min with 3x1 min injection of 1 M NaCl in 50 mM NaOH and 1 min 10 mM HCl.

HBS-ET (10 mM HEPES pH 7.4, 150 mM NaCl, 1mM EDTA, 0.05% Tween® 20 was used as system buffer. The sample buffer was identical with the system buffer. The Biacore™ 2000 System was driven under the control software V1.1.1.

Subsequently biotinylated peptides were captured on the SA surface in the respective flow cells. 16 RU of IGF-1R(1340-1366)[1346-pTyr; Glu(Bi-PEG-1340]amid (i.e. the - 1346 tyrosine phosphorylated - peptide of SEQ ID NO:11 comprising a PEG-linker bound via glutamic acid corresponding to position 1340 and being biotinylated at the other end of the linker) was captured on flow cell 2. 18 RU of IGF-1R(1340-1366); Glu(Bi-PEG-1340]amid (i.e. the - 1346 tyrosine non-phosphorylated - peptide of SEQ ID NO:11 comprising a PEG-linker bound via glutamic acid corresponding to position 1340 and being biotinylated at the other end of the linker) was captured on flow cell 3. 20 RU of hIR(1355-1382)[1361-pTyr; Glu(Bi-PEG-1355]amid (i.e. the - 1361 tyrosine phosphorylated - peptide of SEQ ID NO:12 comprising a PEG-linker bound via glutamic acid corresponding to position 1355 of human insulin receptor and being biotinylated at the other end of the linker) was captured on flow cell 4. Finally all flow cells were saturated with d-biotin.

For the Dual Binder formation the assembly protocol as described in Example 2.5 was used. When individual runs with only one of the two ssFab's were performed, the absence or presence of linker DNA did not affect the association or dissociation curves (data not shown).

100 nM of analyte (i.e. in these experiments a bivalent dual binding agent) in solution was injected at 50 µl/min for 240 sec association time and dissociation was monitored for 500 sec. Efficient regeneration was achieved by using a 1 min injection step at 50 µl /min with 80 mM NaOH. Flow cell 1 served as a reference. A blank buffer injection was used instead of an antigen injection to double reference the data by buffer signal subtraction.
In each measurement cycle one of the following analytes in solution was injected over all 4 flow cells: 100 nM ssFab' 8.1.2, 100 nM ssFab' 1.4.168, a mixture of 100 nM ssFab' 8.1.2 and 100 nM ssFab', 100 nM bivalent binding agent consisting of ssFab' 8.1.2 and ssFab' 1.4.168 hybridized on linker (III) (5'-G CAG AAG CAT TAA TAG ACT-T(20)-T(-Dig)-(T20)-TGG ACG ACG ATA GAA CT-3'(= linker 15 of example 2.4)), and 100 nM bivalent binding agent consisting of ssFab' 8.1.2 and ssFab' 1.4.168 hybridized on linker (IV) (5'-G CAG AAG CAT TAA TAG ACT-T(-Dig) -TGG ACG ACG ATA GAA CT-3'(= linker 16 of example 2.4)), respectively. (The digoxigenylation of the middle thymidine (T(-Dig)) in the above linkers is without relevance to these experiments.)

### The signals were monitored as time-dependent Biacore™ sensorgrams.

Report points were set at the end of the analyte association phase (Binding Late, BL) and at the end of the analyte dissociation phase (Stability Late, SL) to monitor the response unit signal heights of each interaction. The dissociation rates kd (1/s) were calculated according to a linear 1:1 Langmuir fit using the Biacore™ evaluation software 4.1. The complex halftimes in minutes were calculated upon the formula ln(2)/(60*kd).

The sensorgrams (Fig 2-5) show a gain in both specificity and complex stability in pIGF-1R binding when ssFab' 1.4.168 and ssFab' 1.4.168 are used in form of a dual binder (= bivalent binding agent), probably due to the underlying cooperative binding effect. Fab' 1.4.168 alone shows no cross reactivity for the pIR peptide but does not discriminate between the phosphorylated and unphosphorylated form of IGF-1R (T1/2 dis = 3 min in both cases). Fab' 8.1.2, however, binds only to the phosphorylated version of the IGF1-R peptide but exhibits some undesired cross reactivity with phosphorylated Insulin Receptor. The dual binder discriminates well between the pIGF-1R peptide and both other peptides (see Fig. 4) and thus helps to overcome issues of unspecific binding. Note that the gain in specificity is lost when both Fab's are applied without linker DNA (Fig. 5). The gain in affinity of the dual binder towards the pIGF-1R peptide manifests in increased dissociation half times compared to individual Fab's and the Fab' mix omitting the linker DNA (Fig 3 and Fig. 5). Although the tested dual binders with two different DNA linker lengths share an overall positive effect on target binding specificity and affinity, the longer linker ((III) with T40-T-Dig as a spacer) (i.e. linker 15 of example 2.4) seems to be advantageous with respect to both criteria.

### 2.7 Biacore™ assay sandwich of M-1.4.168-IgG and M-8.1.2-IgG

A Biacore™ T100 instrument (GE Healthcare) was used with a Biacore™ CM5 sensor mounted into the system. The sensor was preconditioned by a 1 min injection at 100 µl/min of 0.1 % SDS, 50 mM NaOH, 10 mM HCl and 100 mM H3PO4.

The system buffer was HBS-ET (10 mM HEPES pH 7.4, 150 mM NaCl, 1mM EDTA, 0.05% Tween® 20). The sample buffer was the system buffer.

The Biacore™ T100 System was driven under the control software V1.1.1. Polyclonal rabbit IgG antibody <IgGFCγM>R (Jackson ImmunoResearch Laboratories Inc.) at 30 µg/ml in 10 mM Na-Acetate pH 4.5 was immobilized at 10 000 RU on the flow cells 1, 2, 3, and 4, respectively, via EDC/NHS chemistry according to the manufacturer's instructions. Finally, the sensor surface was blocked with 1M ethanolamine. The complete experiment was driven at 13°C.

500 nM primary mAb M-1.004.168-IgG was captured for 1 min at 10 µl /min on the <IgGFCγM>R surface. 3 µM of an IgG fragment mixture (of IgG classes IgG1, IgG2a, IgG2b, IgG3) containing blocking solution was injected at 30 µl/min for 5 min. The peptide IGF-IR(1340-1366)[1346-pTyr; Glu(Bi-PEG-1340]amid was injected at 300 nM for 3 min at 30 µl/min. 300 nM secondary antibody M-8.1.2-IgG was injected at 30 µl min. The sensor was regenerated using 10 mM Glycine-HCl pH 1.7 at 50 µl/min for 3 min.

Fig.6 describes the assay setup. In Fig 7. the measurement results are given. The measurements clearly indicate, that both monoclonal antibodies are able to simultaneously bind two distinct, unrelated epitopes on their respective target peptide. This is a prerequisite to any latter experiments with the goal to generate cooperative binding events.

### 2.8 Biacore™ assay Dual Binder on sensor surface

A Biacore™ 3000 instrument (GE Healthcare) was used with a Biacore™ SA sensor mounted into the system at T = 25°C. The system was preconditioned at 100 µl/min with 3x1 min injection of 1 M NaCl in 50 mM NaOH and 1 min 10 mM HCl.

The system buffer was HBS-ET (10 mM HEPES pH 7.4, 150 mM NaCl, 1mM EDTA, 0.05% Tween® 20). The sample buffer was the system buffer.

### The Biacore™ 3000 System was driven under the control software V4.1.

124 RU amino-PEO-biotin were captured on the reference flow cell 1. 1595 RU biotinylated 14.6 kDa T0-Bi 37-mer ssDNA-Linker (I) (5'-G CAG AAG CAT TAA TAG ACT-T(-Bi)-TGG ACG ACG ATA GAA CT-3') (= linker 17 of example 2.4) and 1042 RU biotinylated 23.7 kDa T40-Bi 77-mer ssDNA-Linker (II) (5'-G CAG AAG CAT TAA TAG ACT-T(20)- (Biotin-dT)-(T20)-TGG ACG ACG ATA GAA CT-3'= linker 10 of example 2.4) were captured on different flow cells.

300 nM ssFab' 8.1.2 and 300 nM ssFab' 1.004.168 were injected into the system at 50 µl/min for 3 min. As a control only 300 nM ssFab' 8.1.2 or 300 nM ssFab' 1.004.168 was injected to test the kinetic contribution of each ssFab. As a control, buffer was injected instead of the ssFabs. The peptides pIR(1355-1382)[1361-pTyr]amid and IGF-1R(1340-1366)amid, respectively, were injected into system at 50 µl/min for 4 min, free in solution, in concentration steps of 0 nM, 4 nM, 11 nM, 33 nM (twice), 100nM and 300 nM. In another set of experiments to measure the affinities versus the peptide pIGF-1R(1340-1366)[1346-pTyr]amid the concentration steps of 0 nM, 0.4 nM, 1.1 nM, 3.3 nM (twice), 10 nM and 30 nM were used.

The dissociation was monitored at 50 µl/min for 5.3 min. The system was regenerated after each concentration step with a 12 sec pulse of 250 mM NaOH and was reloaded with ssFab' ligand.

Figure 8 schematically describes the assay setup on the Biacore™ instrument. The table given in Figure 9 shows the quantification results from this approach. Figures 10, 11 and 12 depict exemplary Biacore™ results from this assay setup using the T40 dual binding agent.

The table in Figure 9 demonstrates the benefits of the dual binder concept. The T40 dual binding agent (a dual binding agent with linker 10 of example 2.4, i.e. a linker with a spacer of T20-Biotin-dT-T20) results in a 2-fold improved antigen complex halftime (414 min) and a 3-fold improved affinity (10 pM) as compared to the T0 dual binding agent (i.e. a dual binding agent with linker 16 of example 2.4) with 192 min and 30 pM, respectively. This underlines the necessity to optimize the linker length to generate the optimal cooperative binding effect.

The T40 dual binding agent (i.e. the dual binding agent comprising the T40-T-Bi linker (linker 10 of example 2.4)) exhibits a 10 pM affinity versus the phosphorylated IGF-1R peptide (table in Fig. 9, Fig.10). This is a 2400-fold affinity improvement versus the phosphorylated insulin receptor peptide (24 nM) and a 100-fold improvement versus the non-phosphorylated IGF-1R peptide. Therefore, the goal to increase specificity and affinity by the combination of two distinct and separated binding events is achieved.

The cooperative binding effect especially becomes obvious from the dissociation rates against the phosphorylated IGF-1R peptide, where the dual binder shows 414 min antigen complex halftime, versus 0.5 min with the monovalent binder 8.1.2 alone and versus 3 min with the monovalent binder 1.4.168 alone, respectively.

Furthermore, the fully assembled construct roughly multiplies its dissociation rates kd (1/s), when compared to the singly Fab' hybridized constructs (Figs. 10, 11, 12 and table in Fig. 9). Interestingly, also the association rate ka (1/Ms) slightly increases when compared to the single Fab' interaction events, this may be due to an increase of the construct's molecular flexibility.

A diagnostic system using an intense washing procedure should definitely foster the high performance of the T40 dual binding agent, in contrast to individual (monovalent) Fab' molecules. The hybridized construct, i.e. a bivalent binding agent according to the present invention, generates a specific and quite stable binding event, while the monovalent binders more rapidly dissociate, e.g. they are more rapidly washed away.

### Example 3:

### Bivalent binding agent - binding to the homodimeric form of insulin-like

### growth factor 1

The insulin-like growth factor 1 receptor (IGF1-R) belongs to the family of tyrosin kinase receptors and it is activated by IGF1. The receptor is homodimeric.

An IGF1-R monomer consists of one extracellular α subunit which is covalently linked to a transmembrane β subunit via a disulfide bridge. Two monomers are covalently linked by a disulfite bridge between the α subunits of two monomers. The IGF1 receptor plays an important role in cancer, aging and insulin signalling.

The dimer-binder, we developed, comprises two identical Fab'-fragments. Both these Fab'-fragments - when used individually - bind to their target peptide (IGF1-R) only with low complex stability. In order to produce a bivalent binding agent the Fab'-fragment is conjugated to a hybridizable oligonucleotide. An adaptor DNA capable of hybridizing with both the linker DNA as well as with this oligonucleotide is used as a bridge to hybridize with the Fab'-DNA and the Linker-DNA, to provide for a stable bivalent binding agent also featuring the appropriate distance for the to monovalent binders employed (a schematic is given in Figure 13). This anti-IGF1-R dual binder was tested in an automated immunhistochemistry staining system (Ventana BenchMark® XT).

### 3.1 Development of a low-affinity monoclonal antibody against IGF1 receptor

### a) Immunization of mice

Balb/c mice were immunized with a peptide of IGF1R(1340-1366)[1346-pTyr; Aoc-Dys-MP-KLH-1340] amide. (The non-phophorylated amino cid sequence is given as SEQ ID NO:11.) The initial immunization dosis was 100 µg. The mice were further immunized with 100 µg immunogen after 4, 8 and 12 weeks.

### b) Fusion and cloning

Spleen cells of aforementioned mice were fused with myeloma cells according to Galfre, G. and Milstein, C. Methods in Enzymology 73 (1981) 3-46. In this process, 1x10⁸ spleen cells of the immunized mouse were mixed with 2x10⁷ myeloma cells (P3x63-Ag8-653, ATCC CRL1580) and centrifuged for 10 min at 250g and 37°C. The supernatant was discarded and the cell pellet was loosened by tapping. 1mL PEG (molecular weight 4000, Merck, Darmstadt) was added drop by drop within 1 minute at 37°C in a water bath. Afterwards, 5 mL RPMI 1640 without FCS was added drop by drop within 5 min, followed by further 10 mL RPMI 1640 without FCS, which were added drop by drop within 10 minutes. As a next step, 25 mL RPMI 1640 with 10% FCS, 100 mmol/l hypoxanthine, 1 µg/ml azaserine and 50u IL-6 were added and the cells were incubated at 37°C, 5% CO₂ for 60 min and then centrifuged at 250g for 10 min. The cell pellet was resuspended in 30 mL RPMI 1640 with 10% FCS, 100 mmol/l hypoxanthine, 1 µg/ml azaserine and 50u IL-6, and incubated at 37°C, 5% CO₂.

### c) Immunoglobulin isolation from the cell culture supernatants

After ca. 10 days the primary cultures were tested for antigen-specificity. The positive primary cultures were cloned in 96-well cell culture plates by means of a fluorescence activated cell sorter. After subsequent grow in a 24-well plate, a 6-well plate and a T75 flask (Corning) in Hyclone medium (Theromo Scientific) with Nutridoma supplements (Roche), the hybridoma cells expressing the desired low-affinity antibody were eventually cultured in a CeLLine bioreactor (Integra biosciences) according to manufacturer's instructions. The most promising antibody was named mAb 1.4.168.

### d) Biophysical characterization of mAb 1.4.168

The kinetic properties of the interaction between mAB 1.4.168 and the IGF1 receptor were investigated by surface plasmon resonance using Biacore™ technology.

A Biacore™ T100 instrument (GE Healthcare) was used with a Biacore™ CM5 sensor mounted into the system at T = 25°C and was preconditioned by a 1 min injection at 100 µl/min of 0.1 % SDS, 50 mM NaOH, 10 mM HCl and 100 mM H₃PO₄. The system buffer was HBS-ET (10 mM HEPES pH 7.4, 150 mM NaCl, 1mM EDTA, 0.05% Tween® 20). The sample buffer was the system buffer.

The Biacore™ T100 System was driven under the control software V1.1.1. The polyclonal rabbit IgG antibody <IgGFCγM>R (Jackson ImmunoResearch Laboratories Inc.) at 30 µg/ml in 10 mM Na-Acetate pH 4.5 was immobilized at 8000 RU on the flow cells 1,2,3,4 via EDC/NHS chemistry according to the manufacturer's instructions. Finally, the sensor surface was blocked with 1M ethanolamine.

250 nM mAb M-1.004.168-IgG were captured for 1 min at 10 µl /min on the <IgGFCγM>R surface.

A concentration series of 900 nM, 300 nM, 150 nM, 50 nM, 17 nM, 6 nM, and 0 nM of the peptide IGF-1R(1340-1366)[1346-pTyr; Glu(Bi-PEG-1340]amide was used as analyte in solution and was injected at 100 µl / min for 3 min association time. The dissociation was monitored for 10 min at 100 µl/min. As a control, one concentration step was analyzed twice to ensure the reproducibility of the assay. Flow cell 1 served as a reference cell. A blank buffer injection was used instead of an antigen injection to double reference the data by buffer signal subtraction. The capture system was regenerated using a 3 min injection at 10 µl /min with 10 mM glycine pH 1.7.

The data was evaluated according to a 1:1 binary Langmuir interaction model in order to calculate the association rate constant ka [1/Ms], the dissociation rate constant kd [1/s] and the resulting affinity constant K_{D} [M] using the Biacore™ T100 evaluation software V.1.1.1. The dissociation rate constant for mAb 1.4.168 was 3.6 x 10⁻³ 1/s and, consequently, within the range of antibodies with low complex stability, required for our dual-binder approach.

Further, surface plasmon resonance was used to determine the antigen specificity. A Biacore™ 2000 instrument (GE Healthcare) was used with a Biacore™ SA sensor mounted into the system at T = 25°C and was preconditioned at 100 µl/min with 3x1 min injection of 1 M NaCl in 50 mM NaOH and 1 min 10 mM HCl. The system buffer was HBS-ET (10 mM HEPES pH 7.4, 150 mM NaCl, 1 mM EDTA, 0.05% Tween® 20). The sample buffer was the system buffer + 1mg/ml CMD (Carboxymethyldextrane, Sigma).

Subsequently, biotinylated peptides were captured on the SA surface in the respective flow cells. The flow cells finally were saturated with 2 µM d-biotin. Hybridoma Clone Culture supernatant was diluted 1:2 and was injected for 2 min at 50 µl/min, the dissociation was monitored for 5 min. Regeneration at 30 µl/min with 1.5 min injection with 10 mM Gly/HCl, pH 1.7. Flow cell 1 served as a reference. A blank buffer injection was used instead of an antigen injection to double reference the data by buffer signal subtraction. The data was evaluated according to the Biacore™ evaluation software 1.1.1. The signal height was measured at the end of the injection time, Binding Late (RU). The below Table 3 clearly shows, that mAb 1.4.168 recognized the phosphorylated and unphosphorylated IGF-1R peptide (SEQ ID NO:11) equally well, but it did not interact with the phosphorylated insulin receptor peptide (SEQ ID NO:12).

**Table 3: Specificity assay**

| **FC** | **Ligand** | **RU** | **Analyte** | **Binding Late (RU** |
|---|---|---|---|---|
| 1 | Bi | 7 | 1.004.168 | - |
| 2 | IGF-1R(1340-1366)PEG-Bi | 87 | | 199 |
| 3 | IGF-1R(1340-1366)ph1346-PEG-Bi | 97 | | 265 |
| 4 | Bi-InsulinR(1356-1382)p1361 | 80 | | 0 |

FC = flow cell, ligand = biotin and biotinylated peptides, respectively, captured on the SA coated chip, RU = resonace unit (correlates to the mass of bi-peptides captured as ligand on the SA surface), analyte = mAb 1.4.168 in solution, binding late (RU) = signal height at the end of the analyte injection phase.

### 3.2 Antibody purification, Ig cleavage and generation of Fab'-fragments

### a) Purification

The mAb 1.4.168 was first purified by acid precipitation with 2 N acetic acid to a final pH 4.75. After 15 min incubation time and centrifugation at 4°C and 13,000 rpm, the supernatant was buffered to pH 7.0 with 1 M K₃PO₄. Afterwards, the antibody was further purified by ammonium sulphate precipitation (30.5g /100 mL). After centrifugation at 9000 rpm for 30 min at 4°C, the supernatant was discarded and the pellet was resuspended in 10 mM Na-citrate, 20 mM NaCl, pH 5.5. The antibody was then dialyzed against 10 mM Na-citrate, 20 mM NaCl, pH 5.5 and centrifuged at 4°C, 13,000 rpm for 30 min and the cell pellet was discarded. The antibody was further purified by ion exchange chromatography on a SP sepharose column (GE healthcare), equilibrated in 10 mM Na-citrate, 20 mM NaCl, pH 5.5. The antibody was eluted with a gradient of NaCl from 20 mM to 250 mM over 6 column volumes. The eluate was collected with a fraction collector and fractions containing protein were analyzed by HPLC and SDS-PAGE to positively identify the antibody. The fractions containing the antibody were pooled and concentrated by ultrafiltration (Amicon Ultra 10 kDa MWCO) according to manufacturer's instructions and dialyzed against 50 mM potassium phosphate, 150 mM NaCl, pH 7.5.

### b) Papain digestion to generate Fab'₂ molecules

The purified antibody was cleaved with 25 mU Papain /mg IgG at 37°C for 80 min. The reaction was quenched by the addition of 0.1 times the reaction volume of 270 mM iodine acetamide, 75 mM potassium phosphate, 150 mM NaCl and 2 mM EDTA. Afterwards, the reaction mix was dialyzed against 10 mM potassium phosphate, 20 mM NaCl, pH 7.0. The Fab'₂ was then purified by DEAE ion exchange chromatography. The DEAE column (GE healthcare) was equilibrated with 10 mM potassium phosphate, 20 mM NaCl, pH 7.0 and, after loading the dialyzed reaction mix, the Fab'₂ was eluted with 10 mM potassium phosphate, 1 M NaCl, pH 7.0. Further, the Fab'₂ was purified by immunoaffinity chromatography as the flow-through of a column loaded with polyclonal anti-papain antibodies and subsequently a column loaded with polyclonal antibodies against M-Fcγ. PBS was used as a running buffer. The final flow-through was concentrated with an Amicon Ultra 10 kDa MWCO centrifugational filter and the purity was determined by SDS-PAGE.

### c) Reduction of Fab'₂ and generation of Fab'-fragments

The Fab'₂ was reduced at 37°C in a thermomixer (Eppendorf) in 15 mM cysteamine, 25 mM potassium phosphate, 5 mM EDTA, pH 6.5 for 60 min. The reaction was stopped by separation of the Fab' from the cysteamine in a size exlusion chromatography gel filtration on a Sephadex G25 P10 column (GE Healthcare) equilibrated in 100 mM potassium phosphate, 2 mM EDTA, pH 6.3. The Fab' was eluted with 25 mM KPO₄, 2 mM EDTA, pH 6.5.

### 3.3 DNA-labeling of Fab'-fragments

The Fab'-fragment obtained in Example 3.2 was conjugated with the ssDNA-maleimide derivative (SEQ ID NO:16) in a 1:1 molar ratio for 20 min. This ssDNA sequences is capable of hybridizing to the complementary sequence found on the linker and the adaptor oligonucleotide, respectively. The reaction was quenched by the addition of excess cysteine-HCl (for quenching of unconjugated DNA-maleimide) followed by the addition of excess N-methylmaleimide (quenching of free Cysteine-SH groups). Afterwards, the Fab' was dialyzed against 20 mM Tris pH 7.6. The mono-labeled Fab' was then separated from poly-labeled Fab' in an ion exchange chromatography with a Source Q column (GE Healthcare). The column was equilibrated in 20 mM Tris pH 7.6 and the Fab'-DNA conjugates were eluted with a NaCl gradient. The flow-rate was 2 ml/min and the Fab'-DNA conjugates eluted at a salt concentration between 400 and 700 mM NaCl. Mono-labelled Fab'-DNA conjugates were collected with a fraction collector and concentrated by ultrafiltration (Amicon Ultra 10 kDa MWCO) and dialyzed against 10 mM HEPES, 150 mM NaCl, 1 mM EDTA, 0.05 % Tween 20, pH 7.4.

### 3.4 Formation of the bivalent binding agent comprising two anti-IGF1-R Fab'-fragments

The Fab'fragment labeled with the ssDNA linker from Example 3.3, the linker DNA (SEQ ID NO:17) and the adaptor DNA (SEQ ID NO:18) were mixed in equimolar quantities at room temperature to allow for the formation of the bivalent homodimer binder (cf. Figure 13). After a 1 minute incubation step the reaction mix was analyzed on an analytical gel filtration column (SuperdexTM 200, 10/300 GL, GE Healthcare). The concentration of the Fab'-DNA conjugate was 2.5 µM, the concentration of the DNA-Linker and the DNA adaptor was 1.25 µM each.

### 3.5 Analytical size exclusion chromatography to investigate dual binder formation

Analytical size exclusion chromatography was performed using a GE Healthcare Superdex 200 10/300GL analytical size exclusion chromatography column with a flow rate of 0.5 ml/min. PBS was used as a running buffer and 100 µL of sample were injected in each run. The formation of the dual binder via DNA hybridization was established by differences in retention time between the Fab'-DNA conjugate and the ternary complex of Fab'-DNA, Linker-DNA and adaptor DNA (Table 4). The most efficient dual-binder formation was observed at a 2:1:1 ratio of Fab'-DNA, linker DNA and adaptor DNA.

The retention time was monitored by a change in absorbance at 280 nm (aromatic absorbance) and 495 nm (fluorescein absorbance).

**Table 4: Analytical size exclusion chromatography of the dual-binder against IGF1-receptor.**

| Sample | retention time (min) |
|---|---|
| Fab'-DNA conjugate | 30 |
| adaptor DNA | 32 |
| Linker-DNA | 32 |
| Fab'-DNA conjugate + Linker DNA | 26 |
| Fab'-DNA conjugate + Linker DNA + adaptor DNA | 22 |

The retention time was measured as the maximum absorbance (280nm) of the resulting peak in the elution profile of each run. A ternary complex of Fab'-DNA conjugate, linker DNA and adaptor DNA eluted with a shorter retention time than the individual components alone and the complex of Fab'-DNA and linker-DNA, indicating a higher molecular weight and, consequently, a ternary complex formation.

### 3.6 Immunhistochemical staining

NIH3T3 cells were stably transfected to overexpress human IGF1-receptor. The transfected cells were fixed and embedded in paraffin blocks according to standard procedures. 3µm cuts of the block were produced with a microtome (HM355S, Thermo Scientific) and transferred onto a microscope slide for automated IHC staining in the Ventana BenchMark® XT system. The staining procedure was performed using commercially available reagents from Ventana. The cells were deparaffinized and treated with standard CC1 buffer (Ventana) for antigen retrieval. 100 µL of 1µg/ml dual-binder, or dual-binder without adaptor DNA, in antibody diluent buffer (Ventana) were manually titrated onto the slides and incubated for 32 min. The cells were counter-stained with 100 µL hematoxylin II (Ventana) for 4 min and 100 µL bluing reagent (Ventana) for 8 min. The dual-binder detection was based on the XT iview DAB detection kit (Ventana) with the exception that the iview DAB biotinylated secondary antibody of the kit was substituted with reaction buffer (Ventana), and therefore the streptavidin-HRP conjugate of the kit only recognized the biotinylated linker-DNA. The staining was analyzed by light microscopy. Only the dual-binder stained the cells whereas the Fab'-DNA/ DNA linker complex without adaptor DNA (which cannot hybridize into a dual binder) was washed off and did not stain the IGF1-receptor positive cells (Figure 14).

Discussion: Mice were immunized with an IGF1 receptor antigen for antibody development. The most promising antibody, mAb 1.4.168, was characterized by surface plasmon resonance for kinetic properties and specificity. The antibody was then purified, cleaved and labeled with a 17-mer DNA sequence. The hybridization between Fab'-DNA, linker-DNA and adaptor DNA was confirmed by analytical size exclusion chromatography and the dual binder was then used in automated IHC staining (Ventana BenchMark® XT) on stably transfected cells. Only the dual binder positively stained the cells whereas the Fab'-DNA/linker DNA complex alone (without adaptor DNA) was washed off and therefore did not stain. Cooperative binding of both binding elements of the dual binder to the homodimeric receptor most probably resulted in a sufficiently high complex stability to resist the stringent washing on the IHC instrument, which was not the case for the single binding element alone (cf. Figure 14).

### Example 4:

### Detection of HER2/HER3 heterodimers with a dual-binder in immunohistochemistry

The receptor tyrosine kinase family of Her receptors consist of four members: HER1, HER2, HER3 and HER4. Upon ligand binding, the receptors dimerize as homo- or heterodimers in various ways to trigger different signal transduction pathways, depending on the ligand and the expression levels of each of the four family members. For example, HER3 undergoes a conformational shift when it is bound to its ligands Neuregulinl (NRG1) or Neuregulin2 (NRG2), respectively, and the HER3 dimerization domain is exposed and it can interact with other Her receptors. The dimerization domain is constitutively exposed in HER2HER2 and thus, it does not need to be activated by a specific ligand to induce its dimerization. Consequently, stimulation of HER3 by NRG1 or NRG2 can trigger its oligomerisation with HER2HER2. Upon dimerization, the constitutively active tyrosine kinase domain of HER2 phosphorylates HER3 which lacks a functional tyrosine kinase domain.

A dual binder to detect the heterodimer of HER2/HER3 can be provided. Monoclonal antibodies against HER3 and HER2 are developed with a dissociation rate constant within the range defined in the attached claims. Further, the anitbodies are validated in immunohistochemistry for target recognition in cancer cell lines, xenograft and tumor tissue. The selected antibodies are sequenced using standard molecular biology methods and a library of dual-binders is generated using recombinant protein expression. The dual-binders, with different length of linkers, are then screened on formalin-fixed and paraffin embedded cancer cells. Prior to embedding, the cells either have been starved, or they have been stimulated with human NRG1 to induce HER2/HER3 heterodimerization. The detection of heterodimerization is assessed in an automated immunohistochemistry staining system (Ventana BenchMark® XT). A positive hit in the screening is defined as a dual binder that stains stimulated cells (HER2/HER3 dimers present) but not the starved cells (no heterodimers).

### Development of monoclonal antibodies against HER3 and HER2 within a defined range of the dissociation rate constant

### a) Immunization of mice

SJL, Balb/c and NMRI mice are immunized with HER3(1242-1267)[KLH-MP-Cys-UZU-1243]amide and HER2(1223-1236)[KLH-MP-Cys-UZU]amide. The initial immunization dose is 100 µg. The mice are further immunized with 100 µg of the immunogen after 6 and 10 weeks.

### b) Fusion and cloning

Spleen cells of aforementioned mice are fused with myeloma cells according to Galfre, G. and Milstein, C. Methods in Enzymology 73 (1981) 3-46. In this process, 1x10⁸ spleen cells of the immunized mouse are mixed with 2x10⁷ myeloma cells (P3x63-Ag8-653, ATCC CRL1580) and centrifuged for 10 min at 250g and 37°C. The supernatant is discarded and the cell pellet is loosened by tapping. 1mL PEG (molecular weight 4000, Merck, Darmstadt) is added drop by drop within 1 minute at 37°C in a water bath. Afterwards, 5 mL RPMI 1640 without FCS is added drop by drop within 5 min, followed by further 10 mL RPMI 1640 without FCS, which is added drop by drop within 10 minutes. As a next step, 25 mL RPMI 1640 with 10% FCS, 100 mmol/l hypoxanthine, 1 µg/ml azaserine and 50u IL-6 are added and the cells are incubated at 37°C, 5% CO₂ for 60 min and then centrifuged at 250g for 10 min. The cell pellet is resuspended in 30 mL RPMI 1640 with 10% FCS, 100 mmol/l hypoxanthine, 1 µg/ml azaserine and 50u IL-6, and incubated at 37°C, 5% CO₂. The cell suspension is then transferred to 96-well plates and incubated at 37°C, 5% CO₂.

### c) Immunoglobulin isolation from the cell culture supernatants

After ca. 10 days the primary cultures are tested for antigen-specificity. The positive primary cultures are cloned in 96-well cell culture plates by means of a fluorescence activated cell sorter. After subsequent growth in a 24-well plate, a 6-well plate and a T75 flask (Corning) in Hyclone medium (Theromo Scientific) with Nutridoma supplements (Roche), the hybridoma cells expressing the desired low-affinity antibody are eventually cultured in a CELLine bioreactor (Integra biosciences) according to manufacturer's instructions.

### d) Biophysical characterization of monoclonal antibodies

The kinetic properties of the interaction between the monoclonal antibodies and HER2 or HER3 are investigated by surface plasmon resonance kinetic screening using Biacore™ technology.

A Biacore™ A100 instrument under control of the software version V1.1 is used. A Biacore™ CM5 chip is mounted into the instrument and is hydrodynamically addressed conditioned according to the manufacturer's instructions. As a running buffer an HBS-EP buffer is used (10 mM HEPES (pH 7.4), 150 mM NaCl, 1 mM EDTA, 0.05 % (w/v) P20). A polyclonal rabbit anti-mouse IgG Fc capture antibody is immobilized at 30 µg/ml in 10 mM sodium acetate buffer (pH 4.5) to spots 1, 2, 4 and 5 in flow cells 1, 2, 3 and 4 at 10,000 RU. The antibody is covalently immobilized via NHS/EDC chemistry. The sensor is deactivated thereafter with a 1 M ethanolamine solution. Spots 1 and 5 are used for the determination and spots 2 and 4 are used as reference. Prior to application to the sensor chip the hybridoma supernatants containing mAbs are diluted 1:2 in HBS-EP buffer. The diluted solution is applied at a flow rate of 30 µl/min for 1 min. Immediately thereafter the analyte, human_HER3(1242-1267)-Bi-PEG-amide (SEQ ID NO:26) or human_HER2(1223-1236)-Bi-PEG-amide (SEQ ID NO:25), singly grafted on streptavidin, is injected at a flow rate of 30 µl/min for 2 min. Thereafter, the signal is recorded for 5 min dissociation time. The sensor is regenerated by injecting a 10 mM glycine-HCl solution (pH 1.7) for 2 min, at a flow rate of 30 µl/min. The dissociation rate constant kd (1/s) is calculated according to a Langmuir model using the evaluation software according to the manufacturer's instructions. The selected monoclonal antibodies interact with HER2, respectively HER3, with a dissociation rate constant that lies within the boundaries of the patent claim. The antibodies have to bind their epitope specifically, i.e., the anti-HER2 antibody does not interact with HER3 and vice versa.

The selected antibody directed against HER3 was called 7.2.32 (variable region heavy chain shown in SEQ ID NO:19 and variable region light chain shown in SEQ ID NO:20, respectively) and its dissociation rate constant was determined as 2.3 x 10⁻³ 1/s and, consequently, is within the range required for the dualbinder approach. The selected antibody directed against HER2 was called 4.1.43 (variable region heavy chain shown in SEQ ID NO:21 and variable region light chain shown in SEQ ID NO:22, respectively) and the dissociation rate constant was 9.63 x 10⁻⁴ 1/s, and thus, also within the range required for the dual binder approach.

### Characterization of selected monoclonal antibodies in immunohistochemistry.

Not only do the selected monoclonal antibodies against HER2 and HER3 have to bind their target within a certain range of dissociation rate constant, but they also have to be well characterized in immunohistochemistry to be suitable as the foundation of the dual binder. First, it has to be ensured that both monoclonal antibodies still recognize their antigens in immunohistochemistry. This can be achieved by staining formal-fixed and paraffin embedded HEK293 that overexpress recombinant HER1, HER2, HER3 or HER4 after transient transfection. The stainings need to be performed with the Ventana BenchMark® automated immunohistochemistry platform using the same protocol for the anti-HER2 and the anti-HER3 antibody. For example, the stainings could be performed with the ultraview DAB kit with CC1Standard, antibody incubation time of 32 min at 37°C, using the ultraWash option and a counter staining with Hematoxylin II for 4 min and Blueing reagent for 8 min. Further, the antibodies are to be validated on xenograft and tumour tissues with known HER2 and HER3 expression levels using the same staining protocol. In addition, the amino acid sequences of heavy and light chains are obtained from the hybridoma clones using standard molecular biology procedures.

### Development of a dual-binder recognizing HER2 and HER3

### a) Development of dual-binder using peptide linkers

We develop dual-binders that consist of two Fab fragments which are conncected by a linker of glycine-glycine-glycine-glycine-serine (G4S) units and a hemagglutinin-Tag (YPYDVPDYA) (Figure 15). The length of the linker can easily be changed by incorporating different numbers of G4S units into the linker. The linker can contain between two and eight G4S units. The dual binder is expressed after transient expression in HEK293 cells using a plasmid (HC dual binder) encoding a fusion protein of Fab_7.2.32-linker-Fab_4.1.43 heavy chain (shown in SEQ ID NO:24), and two plasmids encoding the light chains of the Fabs. All plasmids also encode a signaling sequence for extracellular trafficking upsteam of the antibody coding sequences. The codon usage is optimized for recombinant eukaryotic expression and the corresponding DNA is then synthesized (Geneart) and cloned into a pUC vector using BamHI/XbaI restriction sites. The linker sequence is flanked with HindIII/KpnI restriction sites to allow easy exchange of the linker sequence of the plasmid using standard molecular biology methods.

HEK293-F cells in suspension are transfected with the plasmids for transient expression of the recombinant dual binder. In total, 50 mL 1x10⁶ cells/ml with a viability of >90 % are transfected with the plasmids HC-Dual binder (coding for the protein sequence shown in SEQ ID NO:24), LC_7.2.32 (coding for the protein sequence shown in SEQ ID NO:20) and LC_4.1.43 (coding for the protein sequence shown in SEQ ID NO:22) in a ratio of 1:1:1 using 293-Free™ Transfection Reagent (Novagen) according to the manufacturer's instructions. After transfection, the HEK293-F cells are incubated for 7 days at 130 rpm, 37°C and 8 % CO₂. The cells are then centrifuged at 4°C, 8000 rpm for 20 min. The supernatant containing the dual binder is further filtered using a 0.22 µm steriflip (Millipore) vacuum filtration system, aliquoted and flash frozen in liquid nitrogen and stored at 20°C.

### b) Development of dual-binders using DNA-based linkers

To generate dual binders based on hybridization via DNA-linkers, the recombinant Fab-fragments are expressed as fusion proteins encoding a sortase cleavage recognition sequence (SEQ ID NO:23). Next, the recombinant proteins are labeled site-specifically with 17mer (oligo for 4.1.43 labeling shown in SEQ ID NO:27) and 19mer (oligo for 7.2.32 labeling shown in SEQ ID NO:28) oligo molecules which can then hybridize with the available library of linker DNA.

### DNA-oligo conjugation using the enzyme Sortase in a transpeptidase reaction

The enzyme sortase is a prokaryotic and proteolytic enzyme that also has a transpeptidase activity (Ton-That et al, PNAS 1999). Here, the enzyme catalyzes a transpeptidase reaction between an LPXTG motive and a glycine residue that is attached to the DNA-oligo.

The recombinant Fab-fragments of 7.2.32 and 4.1.43 are expressed in HEK293 cells. 400 mL 1x10⁶ HEK 293 cells/ml with a viability of >90 % were transfected in a ratio of 1:1 with the plasmids encoding the heavy chain and light chain of 7.2.32 or 4.1.43 using 293-Free™ Transfection Reagent (Novagen) according to the manufacturer's instructions.

The proteins are expressed as fusion proteins and carry a C-terminal 6xHIS-Tag at the light chain and a C-terminal Sortase-cleavage sequence at the heavy chain of the Fab fragments. Further, 7.2.32 carries an HA-Tag upstream of the Sortase cleavage-tag.

After transfection, the HEK293-F cells are incubated for 7 days at 130 rpm, 37°C and 8 % CO₂. The cells are then centrifuged at 4°C, 8000 rpm for 20 min. The supernatant, containing the recombinant protein, is further filtered using a 0.22 µm steriflip (Millipore) vacuum filtration system.

The Fab fragments are purified by Nickel affinity-column chromatography and preparative gel filtration using the ÄKTA explorer FPLC system using standard purification methods. Purity was accessed by SDS-PAGE and analytical gel filtration.

The labeling is performed with 10 µM recombinant Sortase, 50 µM Fab fragment and 200 µM Oligo in a buffer of 50mM Tris pH 7.5, 150 mM NaCl, 5 mM CaCl₂, at 37°C overnight. Next, the labeling reaction is diluted 10 times in 20mM Tris pH 8.0 and applied to a Resource Q ion exchange column (GE Healthcare) which is equilibrated in 20 mM Tris pH 8.0. The strongly negatively charged Oligo and the Oligo-Fab fragments are eluted with a high salt gradient of 20 mM Tris pH 8.0 and 1M NaCl, and thus separated from the Sortase and the unlabeled Fab fragment that elute at a low salt concentration. The elution is monitored following the absorbance at 495nm, detecting the fluorescein-label of the Oligo. The eluted fractions containing Oligo and Fab-Oligo are pooled and the Fab-Oligo is separated from the unconjugated Oligo by preparative gel filtration on a HiLoad 16/60 column Superdex 200 column (GE Healthcare) using 20 mM Tris 8.0, 200 mM NaCl as equilibration and running buffer. The purity of the final product is assessed using analytical gel filtration and SDS-PAGE and only >90 % pure end product will be used in the assembly of dual binders.

### The assembly of the dual binder is investigated by analytical gel filtration.

The 7.2.32-Fab-19mer and 4.1.43-Fab-17mer conjugates in an equimolar ratio are mixed in individual experiments with each one of the linker molecules (linker 3-14 that are shown in Example 2.4. The assembly of the dual binder is then investigated using analytical gel filtration.

Analytical size exclusion chromatography is performed using a GE Healthcare Superdex 200 10/300GL analytical size exclusion chromatography column with a flow rate of 0.5 ml/min. 20 mM Tris pH 8.0, 200 mM NaCl is used as a running buffer and 100 µL of sample are injected in each run. The concentration of the Fab-oligo conjugates and the linker is 2.5 µM. The retention time is monitored by a change in absorbance at 280 nm (aromatic absorbance) and 495 nm (fluorescein absorbance). The formation of the dual binder via DNA hybridization is established by differences in retention time between the Fab-oligo conjugate, Fab-Oligo and linker complex and the ternary complex of the two Fab-fragments and the linker.

### Generation of formalin-fixed and paraffin embedded MCF-7 cells

The MCF-7 cancer cell line is known to express intermediate levels of HER2 and HER3 but no HER1 (DeFazio et al, Int. J. Cancer,87, 487-498 (2000)) and it is therefore a preferred study model to detect the induction of HER2/HER3 heterodimerization. Further, HER2/HER3 heterodimers have already been detected in stimulated MCF-7 cells using other methods (Mukherjee et al, PLOS One 6(1),2011).

The MCF-7 cells are cultured using RPMI 1640 (Gibco) with 2 mM L-Glutamine (Gibco) and 10 % FCS and they are grown to optical confluency in a T175cm2 flask. The confluent cells are starved in RPMI 1640, 2 mM L-Glutamine without FCS for approximately 18 hours. The cells are then stimulated with 20 nM NRG1-β1 (Peprotech) for 15 min at 37°C. As a negative control, cells are left without stimulation for 15 min at 37°C in fresh cell culture medium without FCS. The cells are fixed with formalin and embedded in paraffin as described (Defazio-Eli et al, Breast Cancer Res 13(2):R44 (2011).

Since phosphorylation of HER3 only occurs after ligand binding, a staining of phosphorylated HER3 should show a difference in activation status of HER3 between treated and non-treated MCF-7 cells that may also indicate different levels of HER2/HER3 heterodimerization. The stimulation of HER3 by NRG1-β1 is validated on the formalin-fixed and paraffin embedded MCF-7 cells with immunohistochemical stainings using the 21D3 (Cell Signaling) antibody that is specific for phosphorylated HER3. The manual immunohistochemical staining is performed using EDTA buffer (Thermo Scientific) for antigen retrieval and the Ultra Vision LP Large Volume Detection System (Thermo Scientific) for antigen detection with the DAB+ chromogen (Dako) according to standard protocols. Only MCF-7 cells that are stimulated with NRG1-β1 stain positively for phosphorylated HER3 and the non-stimulated cells stain negatively.

### Detection of HER2/HER3 heterodimers in MCF-7 cells

The immunohistochemistry stainings are performed on a Ventana BenchMark® XT system. The detection of dual binders with peptide linkers occurrs via the "bridging antibody" anti-HA-Tag antibody C29F4 (Cell Signaling). The Fc-Part of the anti-HA-Tag antibody is detected using the Optiview DAB detection kit (Ventana). The detection signal may be amplified using the Optiview Amplification kit (Ventana). Detection of dual binders with DNA-based linkers occurs either via the described "bridging antibody" anti-HA-Tag antibody C29F4 (targeting the HA-Tag at the C-terminus of the 7.2.32 heavy chain), or via the biotin label of the linker molecule that can serve as detection tag for the streptavidin-based iVIEW DAB detection kit (Ventana). The library of dual binders is prepared in individual experiments by mixing the 7.2.32-Fab-19mer and 4.1.43-Fab-17mer in equimolar ratio with each of the linkers (linker 3-14) that are shown in Example 2.4.

3.5µm cuts of the MCF-7 cell blocks are produced using a microtome (HM355S, Thermo Scientific) and transferred onto a microscope slide. In the automated BenchMark® XT staining system, the cells are deparaffinized and treated with CC1 buffer for 32 min for antigen retrieval. For the screening, each dual binder is diluted in Ventana antibody diluent and 100 µL manually applied to the slides in different dilutions between 1:1 and 1:1000, and incubated for 16 min at 37°C. The anti-HA-Tag antibody C29F4 (Cell Signaling) is diluted 1:400 in Ventana antibody diluent and transferred into a dispenser. The anti-HA antibody has the function of a "bridging antibody" as it binds the HA-Tag of the dual binder, and, at the same time, its Fc part can be detected using the anti-rabbit detection system of the Ventana Optiview DAB detection kit. As a post fixative, 100 µL of the bridging antibody are applied and incubated for 32 min. Next, the Optiview detection and amplification reagents are applied according to manufacturer's recommendations. The staining is analyzed in bright field microscopy.

A positive hit of the screening is a dual-binder that only stains the NRG1-β1-treated MCF-7 cells with induced HER2/HER3 heterodimerization and but does not stain the negative control of untreated MCF-7 cells (HER2 and HER3 monomers only). Further, the staining is inhibited by the addition of peptides of the epitope, 25 µg/ml HER2(1223-1236), shown in SEQ ID NO:25, or 25 µg/ml HER3(1242-1267), shown in SEQ ID NO:26, to inhibit one of the two binding site of the dual binder since the HER2/HER3 heterodimers should only be detectable if both arms of the dual binder are freely accessible.

## Claims

1. A bivalent binding agent of Formula I
A-a':a-S-b:b'-B (Formula I),
the bivalent binding agent being capable of binding a polypeptide dimer, the dimer consisting of two associated polypeptide chains,
a) wherein A is a first monovalent binder, binding to a single epitope of a first target polypeptide comprised in said dimer, wherein B is a second monovalent binder, binding to a single epitope of a second target polypeptide comprised in said dimer,
b) wherein each monovalent binder A and B is selected from the group consisting of a peptide, a peptide mimetic, an aptamer, a spiegelmer, a darpin, a lectin, an ankyrin repeat protein, a Kunitz type domain, a single domain antibody, and a monovalent fragment of an antibody, and has a Kdiss in the range of 5x10⁻³/sec to 10⁻⁴/sec,
c) wherein each of a':a and b:b' independently is a binding pair, wherein a':a and b:b' are different, wherein S is a spacer, wherein - represents a covalent bond,
d) wherein a - S - b is a linker having a length of 6 to 100 nm,
e) wherein the members of the binding pairs a':a and b:b' are hybridizing nucleic acid sequences, and
f) wherein the bivalent binding agent has a Kdiss of 3x10⁻⁵/sec or less.

2. The bivalent binding agent of claim 1,
wherein at least one of the monovalent binders is a single chain antibody, or an Fab-fragment or an Fab'-fragment of a monoclonal antibody.

3. The bivalent binding agent of claim 1,
wherein both the monovalent binders are derived from monoclonal antibodies and are Fab-fragments, or Fab'-fragments, or an Fab-fragment and an Fab'-fragment.

4. The bivalent binding agent according to any of claims 1 to 3,
wherein said bivalent binding agent has a Kdiss of 10⁻⁵/sec or less.

5. The bivalent binding agent according to any of claims 1 to 4,
wherein the linker is an L-DNA-linker.

6. A method for forming a bivalent binding agent that specifically binds a polypeptide dimer, the dimer consisting of two associated polypeptide chains, the method comprising the steps of
a) selecting a first monovalent binder selected from the group consisting of a peptide, a peptide mimetic, an aptamer, a spiegelmer, a darpin, a lectin, an ankyrin repeat protein, a Kunitz type domain, a single domain antibody, and a monovalent fragment of an antibody, that binds to a single epitope of a first target polypeptide comprised in said dimer with a Kdiss of between 5x10⁻³/sec to 10⁻⁴/sec,
b) selecting a second monovalent binder selected from the group consisting of a peptide, a peptide mimetic, an aptamer, a spiegelmer, a darpin, a lectin, an ankyrin repeat protein, a Kunitz type domain, a single domain antibody, and a monovalent fragment of an antibody, that binds to a single epitope of a second target polypeptide comprised in said dimer with a Kdiss of 5x10⁻³/sec to 10⁻⁴/sec,
c) coupling the first monovalent binder to a member of a first binding pair of hybridizing DNA sequences and the second monovalent binder to a member of a second binding pair of hybridizing DNA sequences, wherein the first and the second binding pair do not interfere with each other, and a linker having a length of 6 to 100 nm and comprising a spacer and the complementary binding pair members to the first and the second binding pair member, respectively are co-incubated, whereby a bivalent binding agent is formed,
d) selecting a bivalent binding agent having a Kdiss-value of 3x10⁻⁵/sec or less,
thereby forming a bivalent binding agent that specifically binds the polypeptide dimer.

7. The method of claim 6, wherein said linker is L-DNA.

8. The method of claim 6 or 7 further comprising the step e) of isolating the bivalent binding agent.

9. The bivalent binding agent according to any of claims 1 to 5 or the method according to any of claims 6 to 8, wherein the polypeptide dimer is a homodimer.

10. The bivalent binding agent according to claims 9, wherein both monovalent binders bind to an overlapping epitope.

11. The bivalent binding agent according to claim 9, wherein both monovalent binders bind to the same epitope.

12. The bivalent binding agent according to any of claims 1 to 5 or the method according to any of claims 6 to 8, wherein the polypeptide dimer is a heterodimer.

13. A histological staining method for a polypeptide dimer the method comprising the steps of
a) providing a cell or tissue sample,
b) incubating said sample with a bivalent binding agent according to any of the claims 1 to 5, and
c) detecting the bivalent binding agent, thereby staining said sample for the polypeptide dimer.

14. Use of a bivalent binding agent according to any of claims 1 to 5 in the staining of a cell or tissue sample.

## Patentansprüche

1. Bivalentes Bindemittel der Formel I
A - a':a - S - b:b' - B (Formel I),
wobei das bivalente Bindemittel in der Lage ist, ein Polypeptiddimer zu binden, wobei das Dimer aus zwei assoziierten Polypeptidketten besteht,
a) wobei A ein erster monovalenter Binder ist, der an ein einzelnes Epitop eines ersten in dem Dimer enthaltenen Zielpolypeptids bindet, wobei B ein zweiter monovalenter Binder ist, der an ein einzelnes Epitop eines zweiten in dem Dimer enthaltenen Zielpolypeptids bindet,
b) wobei jeder monovalente Binder A und B aus der Gruppe ausgewählt ist, bestehend aus einem Peptid, einem Peptidmimetikum, einem Aptamer, einem Spiegelmer, einem Darpin, einem Lektin, einem Ankyrin-Repeat-Protein, einer Domäne des Kunitz-Typs, einem Einzeldomänenantikörper und einem monovalenten Fragment eines Antikörpers, und eine Kdiss im Bereich von 5x10⁻³/s bis 10⁻⁴/s aufweist,
c) wobei jedes von a' :a und b:b' unabhängig ein Bindungspaar ist, wobei a' :a and b:b' unterschiedlich sind, wobei S ein Platzhalter ist, wobei - eine kovalente Bindung darstellt,
d) wobei a - S - b ein Linker mit einer Länge von 6 bis 100 nm ist,
e) wobei die Elemente der Bindungspaare a':a und b:b' hybridisierende Nukleinsäuresequenzen sind, und
f) wobei das bivalente Bindemittel eine Kdiss von 3x10⁻⁵/s oder weniger aufweist.

2. Bivalentes Bindemittel nach Anspruch 1,
wobei mindestens einer der monovalenten Binder ein Einzelkettenantikörper oder ein Fab-Fragment oder ein Fab'-Fragment eines monoklonalen Antikörpers ist.

3. Bivalentes Bindemittel nach Anspruch 1,
wobei beide monovalenten Binder von monoklonalen Antikörpern abgeleitet sind und Fab-Fragmente oder Fab'-Fragmente oder ein Fab-Fragment und ein Fab'-Fragment sind.

4. Bivalentes Bindemittel nach einem der Ansprüche 1 bis 3,
wobei das bivalente Bindemittel eine Kdiss von 10⁻⁵/s oder weniger aufweist.

5. Bivalentes Bindemittel nach einem der Ansprüche 1 bis 4,
wobei der Linker ein L-DNA-Linker ist.

6. Verfahren zum Bilden eines bivalenten Bindemittels, das spezifisch ein Polypeptiddimer bindet, wobei das Dimer aus zwei assoziierten Polypeptidketten besteht, wobei das Verfahren die Schritte umfasst
b) Auswählen eines ersten monovalenten Binders, ausgewählt aus der Gruppe, bestehend aus einem Peptid, einem Peptidmimetikum, einem Aptamer, einem Spiegelmer, einem Darpin, einem Lektin, einem Ankyrin-Repeat-Protein, einer Domäne des Kunitz-Typs, einem Einzeldomänenantikörper und einem monovalenten Fragment eines Antikörpers, der an ein einzelnes Epitop eines ersten in dem Dimer enthaltenen Zielpolypeptids bindet, mit einer Kdiss von zwischen 5x10⁻³/s bis 10⁻⁴/s,
b) Auswählen eines zweiten monovalenten Binders, ausgewählt aus der Gruppe, bestehend aus einem Peptid, einem Peptidmimetikum, einem Aptamer, einem Spiegelmer, einem Darpin, einem Lektin, einem Ankyrin-Repeat-Protein, einer Domäne des Kunitz-Typs, einem Einzeldomänenantikörper und einem monovalenten Fragment eines Antikörpers, der an ein einzelnes Epitop eines zweiten in dem Dimer enthaltenen Zielpolypeptids bindet, mit einer Kdiss von 5x10⁻³/s bis 10⁻⁴/s,
c) Koppeln des ersten monovalenten Binders an ein Element eines ersten Bindungspaares von hybridisierenden DNA-Sequenzen und des zweiten monovalenten Binders an ein Element eines zweiten Bindungspaares von hybridisierenden DNA-Sequenzen, wobei das erste und das zweite Bindungspaar sich nicht gegenseitig beeinflussen, und eines Linkers mit einer Länge von 6 bis 100 nm, der einen Platzhalter und die komplementären Bindungspaarelemente zu dem ersten und dem zweiten Bindungspaarelement umfasst, die jeweils co-inkubiert werden, wodurch ein bivalentes Bindemittel gebildet wird,
d) Auswählen eines bivalenten Bindemittels, das einen Kdiss-Wert von 3x10⁻⁵/s oder weniger aufweist,
wodurch ein bivalentes Bindemittel gebildet wird, das spezifisch das Polypeptiddimer bindet.

7. Verfahren nach Anspruch 6, wobei der Linker L-DNA ist.

8. Verfahren nach Anspruch 6 oder 7, ferner umfassend den Schritt e) des Isolierens des bivalenten Bindemittels.

9. Bivalentes Bindemittel nach einem der Ansprüche 1 bis 5 oder das Verfahren nach einem der Ansprüche 6 bis 8, wobei das Polypeptiddimer ein Homodimer ist.

10. Bivalentes Bindemittel nach Anspruch 9, wobei beide monovalenten Binder an ein überlappendes Epitop binden.

11. Bivalentes Bindemittel nach Anspruch 9, wobei beide monovalenten Binder an das gleiche Epitop binden.

12. Bivalentes Bindemittel nach einem der Ansprüche 1 bis 5 oder das Verfahren nach einem der Ansprüche 6 bis 8, wobei das Polypeptiddimer ein Heterodimer ist.

13. Histologisches Färbeverfahren für ein Polypeptiddimer, wobei das Verfahren die Schritte umfasst
a) Bereitstellen einer Zell- oder Gewebeprobe,
b) Inkubieren der Probe mit einem bivalenten Bindemittel nach einem der Ansprüche 1 bis 5, und
c) Erkennen des bivalenten Bindemittels, wodurch die Probe für das Polypeptiddimer gefärbt wird.

14. Verwendung eines bivalenten Bindemittels nach einem der Ansprüche 1 bis 5 bei der Färbung einer Zell- oder Gewebeprobe.

## Revendications

1. Agent de liaison bivalent de formule 1
A - a':a - S -b:b' - B (Formule 1),
l'agent de liaison bivalent étant capable de se lier à un dimère polypeptidique, le dimère se composant de deux chaînes polypeptidiques associées,
a) A étant un premier liant monovalent, se liant à un seul épitope d'un premier polypeptide cible compris dans ledit dimère, B étant un second liant monovalent, se liant à un seul épitope d'un second polypeptide cible compris dans ledit dimère,
b) chaque liant monovalent A et B étant choisi dans le groupe constitué par un peptide, un peptide mimétique, un aptamère, un Spiegelmer, une DARPin, une lectine, une protéine à répétitions ankyrine, un domaine de type Kunitz, un anticorps à domaine unique et un fragment monovalent d'un anticorps et ayant une Kdiss comprise dans la plage allant de 5x10⁻³/s à 10⁻⁴/s,
c) chaque a':a et b:b' étant indépendamment une paire de liaison, a':a et b:b' étant différents, S étant un espaceur, - représentant une liaison covalente,
d) a - S - b étant un lieur ayant une longueur de 6 à 100 nm,
e) les membres des paires de liaison a':a et b:b' étant des séquences d'acides nucléiques s'hybridant, et
f) l'agent de liaison bivalent ayant une Kdiss inférieure ou égale à 3x10⁻⁵/s.

2. Agent de liaison bivalent selon la revendication 1,
dans lequel au moins un des liants monovalents est un anticorps monocaténaire ou un fragment Fab ou un fragment Fab' d'un anticorps monoclonal.

3. Agent de liaison bivalent selon la revendication 1,
dans lequel les deux liants monovalents sont dérivés d'anticorps monoclonaux et sont des fragments Fab ou des fragments Fab', ou un fragment Fab et un fragment Fab'.

4. Agent de liaison bivalent selon l'une quelconque des revendications 1 à 3,
dans lequel ledit agent de liaison bivalent a une Kdiss inférieure ou égale à 10⁻⁵/s.

5. Agent de liaison bivalent selon l'une quelconque des revendications 1 à 4,
dans lequel le lieur est un lieur L-ADN.

6. Procédé de formation d'un agent de liaison bivalent qui se lie spécifiquement à un dimère polypeptidique, le dimère se composant de deux chaînes polypeptidiques associées, le procédé comprenant les étapes consistant à :
a) sélectionner un premier liant monovalent choisi dans le groupe constitué par un peptide, un peptide mimétique, un aptamère, un Spiegelmer, une DARPin, une lectine, une protéine à répétitions ankyrine, un domaine de type Kunitz, un anticorps à domaine unique et un fragment monovalent d'un anticorps, qui se lie à un seul épitope d'un premier polypeptide cible compris dans ledit dimère avec une Kdiss comprise dans la plage allant de 5x10⁻³/s à 10⁻⁴/s,
b) sélectionner un second liant monovalent choisi dans le groupe constitué par un peptide, un peptide mimétique, un aptamère, un Spiegelmer, une DARPin, une lectine, une protéine à répétitions ankyrine, un domaine de type Kunitz, un anticorps à domaine unique et un fragment monovalent d'un anticorps, qui se lie à un seul épitope d'un second polypeptide cible compris dans ledit dimère avec une Kdiss comprise dans la plage allant de 5x10⁻³/s à 10⁻⁴/s,
c) coupler le premier liant monovalent à un membre d'une première paire de liaison de séquences d'ADN s'hybridant et le second liant monovalent à un membre d'une seconde paire de liaison de séquences d'ADN s'hybridant, la première et la seconde paire de liaison n'interférant pas entre elles, et un lieur ayant une longueur de 6 à 100 nm et comprenant un espaceur et les membres de paires de liaison complémentaires respectivement des premier et second membres des paires de liaison étant co-incubés, permettant la formation d'un agent de liaison bivalent,
d) sélectionner un agent de liaison bivalent ayant une valeur Kdiss inférieure ou égale à 3x10⁻⁵/s,
permettant ainsi la formation d'un agent de liaison bivalent qui se lie spécifiquement au dimère polypeptidique.

7. Procédé selon la revendication 6, dans lequel ledit lieur est le L-ADN.

8. Procédé selon la revendication 6 ou 7, comprenant en outre l'étape e) consistant à isoler l'agent de liaison bivalent.

9. Agent de liaison bivalent selon l'une quelconque des revendications 1 à 5 ou procédé selon l'une quelconque des revendications 6 à 8, dans lequel le dimère polypeptidique est un homodimère.

10. Agent de liaison bivalent selon la revendication 9, dans lequel les deux liants monovalents se lient à un épitope chevauchant.

11. Agent de liaison bivalent selon la revendication 9, dans lequel les deux liants monovalents se lient au même épitope.

12. Agent de liaison bivalent selon l'une quelconque des revendications 1 à 5 ou procédé selon l'une quelconque des revendications 6 à 8, dans lequel le dimère polypeptidique est un hétérodimère.

13. Procédé de coloration histologique pour un dimère polypeptidique, le procédé comprenant les étapes consistant à :
a) utiliser un échantillon de cellule ou de tissu,
b) incuber ledit échantillon avec un agent de liaison bivalent selon l'une quelconque des revendications 1 à 5 et
c) détecter l'agent de liaison bivalent, colorant ainsi ledit échantillon pour le dimère polypeptidique.

14. Utilisation d'un agent de liaison bivalent selon l'une quelconque des revendications 1 à 5 pour la coloration d'un échantillon de cellule ou de tissu.
